(19) Europäisches Patentamt
European Patent Office
Office européen des brevets

(11) Numéro de publication: **0 180 492 B1**

(12) **FASCICULE DE BREVET EUROPEEN**

(45) Date de publication de fascicule du brevet: **02.06.93**

(21) Numéro de dépôt: **85401838.9**

(22) Date de dépôt: **23.09.85**

Le dossier contient des informations techniques présentées postérieurement au dépôt de la demande et ne figurant pas dans le présent fascicule.

(51) Int. Cl.5: **C07D 273/00**, G01N 33/533, C07D 498/22, C07F 5/00, //(C07D498/22,273:08,273:08, 221:00,221:10),(C07D498/22, 273:08,273:08),(C07D498/22, 259:00,259:00,221:10,221:10, 221:10,221:10,221:10,221:10)

(54) **Complexes macropolycycliques de terres rares-et application à titre de marqueurs fluorescents.**

(30) Priorité: **26.09.84 FR 8414799**

(43) Date de publication de la demande: **07.05.86 Bulletin 86/19**

(45) Mention de la délivrance du brevet: **02.06.93 Bulletin 93/22**

(84) Etats contractants désignés: **AT BE CH DE FR GB IT LI LU NL SE**

(56) Documents cités:
**EP-A- 0 068 875**
**EP-A- 0 085 320**
**FR-A- 2 088 519**

**CHEMICAL ABSTRACTS, vol. 99, no. 10, 5 septembre 1983, page 676, no. 81500q, Columbus, Ohio, US; O.A. GANSOW et al.: "Preparation and characterization of some bifunctional lanthanide cryptates"**

(73) Titulaire: **CIS BIO INTERNATIONAL**
**RN 306**
**F-91000 Saclay(FR)**

(72) Inventeur: **Mathis, Gerard**
**17, Impasse de la Capelle des Ladres**
**F-30200 - Bagnols S/Ceze(FR)**
Inventeur: **Lehn, Jean Marie**
**21, rue d'Oslo**
**F-67000 - Strasbourg(FR)**

(74) Mandataire: **Gillard, Marie-Louise et al**
**Cabinet Beau de Loménie 158, rue de l'Université**
**F-75340 Paris Cédex 07 (FR)**

EP 0 180 492 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

CHEM. BER., vol. 111, 1978, pages 200-204, Weinheim, DE; E. BUHLEIER et al.: "2,2'-Biparidin als Baustein für neue Aza-Kronenether und Cryptanden"

HELVETICA CHIMICA ACTA, vol. 67, 1984, pages 2265,2267-2269, Büle, DE; J.-C. RODRIGUEZ-UBIS et al.: "Photoactive cryptands. Synthesis of sodium cryptates of macrobicyclic ligands containing bipyridine and phananthroline groups"

JOURNAL OF THE CHEMICAL SOCIETY, CHEMICAL COMMUNICATIONS, no. 7, 1 avril 1985, pages 433-436, Heffers Printers Ltd., Cambridge, GB; J.P. KONOPELSKI et al.: "Synthesis, cation binding, and photophysical properties of macrobicyclic anthracenocrytands"

PIETRASZKIEWICZ et al., 16th International Symposium on Macrocyclic Chemistry, Sheffield, Sept. 1991

Finnish Chemical Letters, Vol. 15, No. 5, p. 95 (1988)

N. SABBATINI et al., 13th Int. Symposium of Macrocyclic Chem., Hamburg, 1988

## Description

La présente invention, qui résulte de travaux effectués en collaboration avec le Professeur J.M. LEHN et son équipe de recherche de l'Université Louis Pasteur de Strasbourg, concerne le domaine de la fluorescence et plus particulièrement des complexes macropolycycliques fluorescents, qui conviennent notamment comme marqueurs dans les dosages immunologiques.

Il est connu que les méthodes de détection utilisant la fluorescence sont intrinsèquement très sensibles et pourraient permettre des limites de détection inférieures à celles atteintes par des mesures de radioactivité, en particulier par l'utilisation de sources Laser (I. Wieder Immunofluorescence and related staining techniques, 1978, Elsevier).

Cependant, dans la pratique, ces limites de détection ne sont pas atteintes car la mesure s'effectue dans une matrice qui ne présente pas les qualités requises pour atteindre cet optimum. En général, le milieu de mesure est trouble et favorise la diffusion, et d'autres molécules fluorescentes à la même longueur d'ondes peuvent être présentes dans le milieu de mesure.

Dans certains cas, les améliorations apportées à l'appareillage de mesure ne suffisent pas à améliorer de façon sensible cette limite de détection ou sont très coûteuses (Laser, monochromateurs, etc.).

Cet état de fait est encore plus gênant dans le domaine de la biochimie et de l'immunologie où la mesure de très petites quantités de molécules actives doit s'effectuer dans des milieux biologiques qui peuvent présenter un trouble ou contenir des protéines ou autres molécules elles-mêmes fluorescentes (trouble et fluorescence intrinsèque du sérum).

Dans les dosages immunologiques utilisant un traceur fluorescent, et lorsque l'on marque l'antigène ou l'anticorps avec la molécule fluorescente, celle-ci doit posséder les propriétés suivantes :

- elle doit posséder une fonction chimique permettant un couplage avec la molécule biologique sans la dénaturer ni modifier ses propriétés immunologiques ;
- le coefficient d'absorption molaire de la molécule fluorescente doit être le plus élevé possible ;
- le rendement quantique de fluorescence doit être le plus élevé ;
- le déplacement de Stokes doit être le plus important possible ;
- la longueur d'onde d'émission doit être si possible supérieure à 500 nm;
- elle doit être soluble dans l'eau ou les solutions tampon. Ces conditions sont précisées par exemple dans l'article de E. SOINI dans Clin. Chem. 25, 353 (1979).

Or les molécules connues jusqu'à présent sont loin de posséder toutes ces qualités.

La fluorescence de certains chélates de terres rares est connue depuis de nombreuses années grâce aux travaux concernant leur application dans le domaine des lasers (A.P.B. SINHA Spectroscop Inorg Chem. 2, 255 (1971)).

Ces complexes sont formés :

- d'une part d'une molécule chélatante possédant un système électronique capable de peupler l'état triplet $T_1$ par conversion inter-système après excitation de l'état singulet $S_1$ suite à l'absorption d'énergie lumineuse ;
- d'autre part d'un ion de terre rare, qui dans le cas des applications de fluorescence possède une forte intensité de fluorescence d'ion (Eu, Tb) ou une intensité moyenne (Sm, Dy) et dont le niveau résonant est peuplé par transfert d'énergie non radiative à partir de l'état triplet du complexant.

Pour obtenir une forte fluorescence du chélate de terre rare il faut que l'énergie triplet de la molécule complexante soit supérieure à celle du niveau résonant de l'ion et que sa durée de vie soit suffisante (prépondérance de ce type de désactivation vis-à-vis d'autres processus, tels que phosphorescence et désactivation non radiative, par exemple thermique).

Dans ce cas, après excitation dans la bande d'absorption du chélate on observe une fluorescence caractéristique de l'ion de terre rare.

Ces composés possèdent de grands avantages dans le domaine de la détection par mesure de fluorescence ; par exemple ils sont caractérisés par :

- un déplacement de Stokes important ,
- un coefficient d'absorption molaire $\epsilon$ élevé par rapport à celui de la terre rare ,
- une possibilité de détection simultanée de plusieurs chélates en changeant la terre rare ,
- un spectre d'émission caractéristique de la terre rare (spectre de raie, $\lambda$ d'émission maximale > 500 nm),
- durée de vie longue (de la microseconde à la milliseconde).

Le brevet US 4 058 732 décrit une méthode de spectroscopie analytique par fluorescence mettant en oeuvre des molécules fluorescentes (chélates de terres rares) ayant une durée de vie relativement longue. Etant donné que la diffusion due aux particules de la matrice et que la fluorescence de la plupart des

molécules organiqués qui la composent sont des phénomènes de courte durée de vie (généralement inférieurs à 1 μ sec.) on préconise dans ce brevet une excitation pulsée et un chélate de terre rare fonctionnalisé pour marquer les molécules biologiques à identifier, la détection s'effectuant entre chaque pulsation et après un temps suffisamment long pour que les phénomènes non souhaités aient diminué de façon substantielle.

Selon l'art antérieur cité ci-après, les chélates de terres rares présentent théoriquement toutes les conditions pour constituer une classe de molécules fluorescentes idéales, en particulier à titre de marqueurs pour les molécules biologiques dans les dosages immunologiques, en cytologie et pour les trieurs de cellules :

- brevet US 4 058 732
- SOINI Clin. Chem. 25 353 (1979)
- LM VALLARINO et al. in Automation of Uterine Cancer Cytology 2 Proceeding of 2nd Int. Conf., GL WIED, G.F. GAHR, PH. BARTEL Editors Tutorials of Cytology Chicago LL 1977.

Or, dans la pratique on constate que les chélates utilisés jusqu'à présent ne présentent pas toutes les qualités suivantes exigées par ces applications, qui sont principalement :

- un rendement quantique de fluorescence et un coefficient d'absorption molaire élevés,
- une énergie Triplet du chélatant adaptée,
- une non-inhibition par le solvant (eau ou autre) ou par des molécules présentes dans le milieu où s'effectue la mesure,
- une facilité de la fonctionnalisation en vue d'un couplage sur des molécules d'interêt biologique ou autres,
- une sélectivité de la chélation en faveur de la terre rare et aux dépens d'autres cations qui peuvent se trouver en quantité importante dans le milieu de mesure,
- une solubilité dans l'eau dans les conditions d'application des dosages immunologiques,
- une grande stabilité en particulier à faible dilution.

Les chélates de β-dicétones décrits dans le brevet US 4 058 732 ne sont que peu ou pas solubles dans l'eau et les milieux biologiques et leur fluorescence est inhibée en grande partie par l'eau.

D'autres chélates ont été proposés comme marqueurs dans des dosages immunologiques ; on peut citer notamment les chélates dérivés d'EDTA, HEDTA et DTPA, d'imidodiacétate et similaires décrits par exemple dans : Proc. Nat. Acad. Sci. USA 72 4764 (1975), le brevet US 4 374 120 et dans les demandes de brevets EP-A-0068875 et DE-OS 3 033 691. Leur efficacité est supposée provenir de la valeur élevée de leur constante de stabilité en général supérieure à $10^{10}$.

Un tel critère, basé uniquement sur des considérations thermodynamiques, n'est certainement pas suffisant puisqu'il a été montré qu'il se produit une augmentation de la durée de vie d'un complexe Tb (III) EDTA par addition d'apotransférine (Biochem. 19, 5057, 1980), caractéristique d'une liaison protéine - chélate.

Récemment, il a été conseillé, lors de l'utilisation de marqueurs du type Eu (III) EDTA, tels que ceux décrits dans le brevet US 4 374 120, d'utiliser un tampon contenant du DTPA pour éliminer l'europium libre qui s'est dissocié du chélate, ce qui prouve bien l'instabilité de tels chélates (Clin. Chem. 29, 60, 1983).

Cet état de l'art antérieur montre que les chélates de terres rares mis en oeuvre jusqu'à présent ne sont pas utilisables à faible dilution dans des milieux aqueux contenant d'autres cations ou dans des milieux biologiques et en particulier dans les dosages immunologiques et qu'il convient de ténir compte des critères, tels que la stabilité cinétique (vitesse de dissociation du complexe) et la sélectivité de la complexation de la terre rare.

On notera d'ailleurs que la plupart des chélates de l'art antérieur ont trouvé, grâce à leur constante de formation élevée (en général > à $10^{10}$) une application en imagerie γ technique dans laquelle on utilise des ions lourds émetteurs γ de durée de vie relativement faible, obtenus en solutions aqueuses diluées. La fixation de l'ion doit alors s'effectuer rapidement sur la molécule d'intérêt biologique portant le chélatant ; en particulier la vitesse de formation du chélate doit être élevée :

$$L + n\ EDTA \underset{k-1}{\overset{k_1}{\rightleftharpoons}} L\ (EDTA)_n$$

$k_1$ et k-1 étant respectivement les constantes de vitesse de formation et de dissociation ; à l'équilibre on a :

$$K = \frac{k_1}{k\text{-}1} = \frac{L\ (EDTA)_n}{[L]\ [EDTA]^n}$$

Pour les complexes utilisés jusqu'à présent, et pour les raisons évoquées ci-dessus, on a toujours utilisé des chélates permettant d'obtenir une valeur de $k_1$ élevée [$\simeq$1,9 $10^{22}$ $M^{-1}$ $min^{-1}$ pour $Eu^{3+}$ EDTA - voir J. inorg. nucl. Chem. 1971, 33 p.[127]]. Il en résulte que pour une valeur donnée de K la constante de vitesse de dissociation est relativement importante, ce qui signifie que la vitesse d'échange de l'ion entre le chélate et la solution est relativement rapide.

D'autres composés macropolycycliques susceptibles de former des complexes contenant certains ions de métaux ont déjà été décrits. A cet effet, on peut citer notamment le brevet FR 2.088.519 qui a pour objet des hétéro-imines macropolycycliques s'apparentant aux composés "couronne", "lanterne" ou "haltère".

On a maintenant trouvé des complexes macropolycycliques de terres rares qui possèdent d'excellentes propriétés de sélectivité et de stabilité, notamment de stabilité cinétique dans les milieux aqueux et les milieux biologiques. Ces complexes macropolycycliques conviennent particulièrement bien comme marqueurs fluorescents de substances biologiques dans des techniques de détection ou de détermination immunologiques par fluorescence. Ils sont également appropriés comme réactifs dans des réactions de luminescence.

On a en effet trouvé qu'il était possible d'exalter la fluorescence d'un ion de terre rare en solution aqueuse en complexant celui-ci à l'aide d'un composé macropolycyclique possédant un motif donneur d'énergie triplet supérieure à celle du niveau émissif de l'ion de terre rare et en excitant le motif donneur.

Alors que l'excitation d'un ion de terre rare produit une très faible fluorescence étant donné que les terres rares possèdent en général des coefficients d'absorption molaires $\epsilon$ faibles, l'excitation du motif donneur du composé macropolycyclique défini ci-après permet d'obtenir une exaltation de la fluorescence caractéristique de l'ion de terre rare. Les complexes de terres rares ainsi formés sont d'excellents marqueurs fluorescents, qui de plus sont des composés stables en milieu aqueux, doués d'une très grande sélectivité vis-à-vis des ions terres rares.

Contrairement aux chélates de l'art antérieur, caractérisés par une constante de formation élevée, les complexes de terres rares selon l'invention possèdent, comme caractéristique essentielle, une stabilité cinétique élevée (faible vitesse de dissociation). Cette caractéristique est d'autant plus importante que les solutions biologiques utilisées dans les méthodes de détection et de détermination immunologiques contiennent en général des protéines capables elles aussi de fixer l'ion terre rare. Par contre la vitesse de formation des complexes de l'invention n'est pas critique. La complexation de l'ion terre rare d'une part et le couplage du complexe obtenu sur la molécule biologique d'autre part peuvent être réalisés séparément. Il en résulte que la complexation de l'ion terre rare selon l'invention peut être réalisée dans des conditions non biologiques (utilisation de solvants organiques, apports d'énergie possibles, temps, etc.)

Sous un premier aspect, la présente invention concerne donc l'utilisation, à titre de marqueurs fluorescents, notamment dans les méthodes de détection et de détermination immunologiques, de complexes macropolycycliques de terres rares qui sont constitués d'au moins un sel de terre rare complexé par un composé macropolycyclique de formule générale :

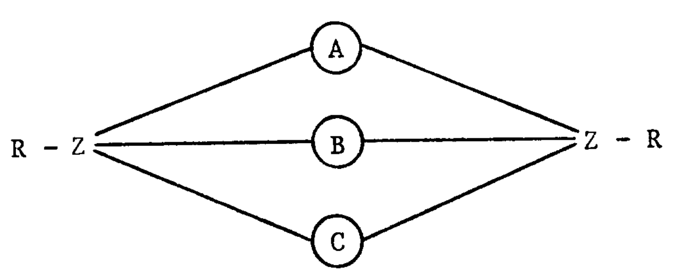

I

dans laquelle Z est un atome ayant trois ou quatre valences, tel que l'azote, le carbone ou le phosphore, R est rien ou représente l'hydrogène, le groupe hydroxy, un groupe amino ou un radical hydrocarboné, les radicaux bivalents Ⓐ, Ⓑ et Ⓒ sont indépendamment l'un de l'autre des chaînes hydrocarbonées qui

contiennent éventuellement un ou plusieurs hétéroatomes et sont éventuellement interrompues par un hétéromacrocycle, au moins l'un des radicaux Ⓐ , Ⓑ ou Ⓒ comportant de plus au moins un motif moléculaire ou étant essentiellement constitué par un motif moléculaire, ledit motif moléculaire possédant une énergie de triplet supérieure à celle du niveau émissif de l'ion de terre rare complexé.

Selon un autre aspect, l'invention concerne également un procédé d'exaltation de fluorescence d'un ion de terre rare, qui consiste à complexer au moins un ion de terre rare avec un composé macropolycyclique, tel que défini ci-dessus possédant un motif moléculaire donneur d'énergie triplet supérieure à celle du niveau émissif de l'ion de terre rare et à exciter le complexe ainsi formé à la longueur d'onde d'absorption dudit motif donneur.Les complexes de terres rares définis ci-dessus sont des composés nouveaux à l'exception des complexes d'europium et de terbium obtenus avec le composé macropolycyclique de formule ci-après :

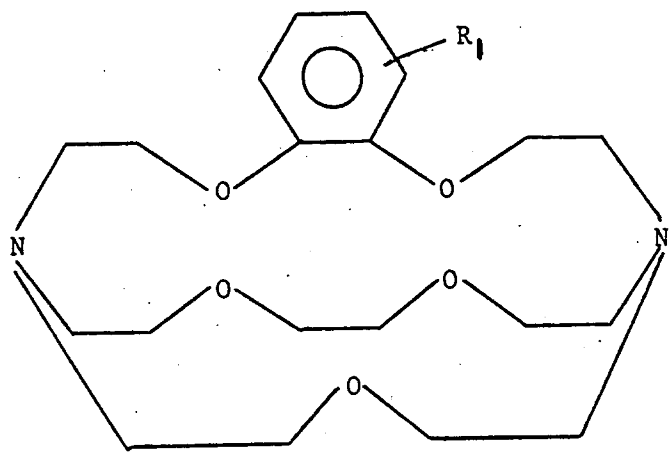

$R_1$= H ou $NH_2$

et les complexes de lanthane et de praseodyme avec le composé ci-dessus dans lequel $R_1$ est $NH_2$. Ces derniers sont décrits dans le document Chemical Abstracts, vol. 99, n° 10 (5 Septembre 1983), p.676, n° 81500 q.

Ainsi la présente invention concerne également à titre de composés nouveaux les complexes de terres rares constitués d'au moins un sel de terre rare complexé par composé macropolycyclique de formule I ci-dessus à la condition que, lorsque le sel de terre rare est un sel d'europium ou de terbium, Z est l'azote, Ⓐ est $-(CH_2)_2-O-C_6H_3R_1-O-(CH_2)_2-$ avec $R_1$ = H ou $NH_2$, Ⓑ et Ⓒ ne sont pas simultanément l'un le radical $-(CH_2)_2-O-(CH_2)_2-O-(CH_2)_2-$ et l'autre le radical $-(CH_2)_2-O-(CH_2)_2-$ et que lorsque la sel de terre rare est un sel de lanthane ou de praseodyme, Z est l'azote, Ⓐ est $-(CH_2)_2-O-C_6H_3NH_2-O-(CH_2)_2-$ Ⓑ et Ⓒ ne sont pas simultanément $-(CH_2)_2-O-(CH_2)_2-$ et $-(CH_2)_2-O-(CH_2)_2-O-(CH_2)_2-$.

Les chaînes hydrocarbonées qui constituent les radicaux Ⓐ , Ⓑ et Ⓒ peuvent contenir 2 atomes de carbone ou plus et être éventuellement interrompues par un ou plusieurs hétéroatomes choisis parmi les atomes d'oxygène, de soufre ou d'azote.

Les chaînes hydrocarbonées préférées aux fins de l'invention sont les chaînes de polyéthers, en particulier les chaînes oxyéthylénées ou polyoxyéthylénées.

Le motif moléculaire, qui constitue un élément essentiel du composé macropolycyclique selon l'invention, est motif donneur d'énergie triplet composé de molécules ou de groupes de molécules possédant une énergie de triplet supérieure à celle du niveau émissif de l'ion de terre rare complexé.

Le transfert d'énergie s'effectue depuis le niveau triplet du motif donneur vers l'un des niveaux émissifs de la terre rare complexée. Par exemple l'europium possède les niveaux émissifs $^5D_0$ à 17270 $cm^{-1}$ ; $^5D_1$ à 19030 $cm^{-1}$ et éventuellement $^5D_2$ et le terbium le niveau émissif $^5D_4$ à 20480 $cm^{-1}$.

Les motifs donneurs d'énergie triplet qui conviennent aux fins de l'invention doivent posséder une énergie triplet supérieure ou égale à celle des niveaux émissifs de l'ion de terre rare. Par exemple, dans le cas des complexes d'europium et de terbium selon l'invention, le niveau triplet du motif donneur doit être supérieur à 17270 $cm^{-1}$.

Le phénomène de phosphorescence étant dû à la désactivation radiative d'un état triplet, un critère de choix des motifs donneurs peut être la longueur d'onde d'émission de phosphorescence de ces motifs. On choisira par exemple les motifs qui émettent une phosphorescence à des longueurs d'ondes inférieures (énergies supérieures) aux longueurs d'ondes correspondant au peuplement des niveaux émissifs de la terre rare. Dans le cas présent la longueur d'onde de phosphorescence du motif donneur devra être

inférieure à 580 nm.

On notera que les complexes selon l'invention peuvent avoir un ou plusieurs motifs donneurs, lesdits motifs donneurs constituant soit une partie, soit la totalité des radicaux Ⓐ , Ⓑ et Ⓒ .

A titre de motifs moléculaires on peut utiliser de façon non limitative tous les sensibilisateurs de triplet ayant l'énergie triplet requise, par exemple ceux décrits dans la demande de brevet EP-A-0068875 ou dans Spectroscop. Inorg. Chem. 2, 255, 1971, ces documents étant cités dans la présente description à titre de référence.

Des motifs moléculaires particulièrement préférés aux fins de l'invention sont la phénanthroline, l'anthracène, le benzène, le naphtalène, les bi- et ter-phényle, les bipyridines, les biquinoléines, notamment les bi-isoquinoléines, par exemple la 2,2'-bipyridine, l'azobenzène, l'azopyridine, la pyridine ou la 2,2'-bi-isoquinoléine.

A titre d'exemples de radicaux Ⓐ, Ⓑ et Ⓒ comportant un motif donneur d'énergie, on peut citer notamment les chaînes ci-après :

- $C_2H_4$ - $X_1$ - $C_6H_4$ - $X_2$ - $C_2H_4$ -

- $C_2H_4$ - $X_1$ - $CH_2$ - $C_6H_4$ - $CH_2$ - $X_2$ - $C_2H_4$ - dans lesquelles

$X_1$ et $X_2$ pouvant être identiques ou différents désignent l'oxygène ou le soufre ;

- 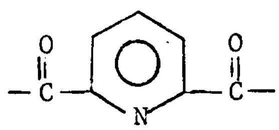 ; 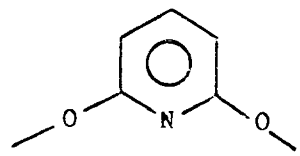

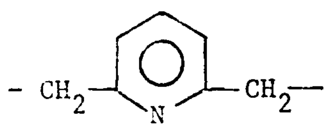

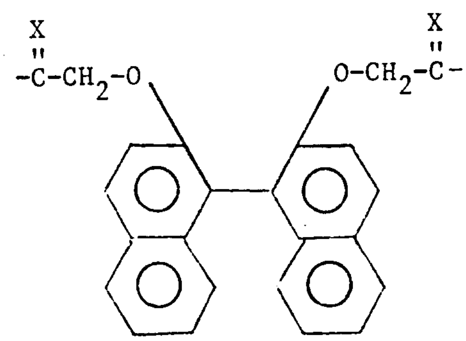

X étant l'oxygène ou l'hydrogène.

Les complexes macropolycycliques de terres rares selon l'invention peuvent être obtenus par les procédés classiques de préparation de complexes métalliques, qui consistent à faire réagir le composé complexant avec un composé donneur du cation à complexer. Des procédés de ce type sont décrits notamment dans les brevets US 3 888 377, 3 966 766 et 4 156 683, cités dans la présente description à titre de référence.

Par exemple, les complexes macropolycycliques peuvent être obtenus par réaction d'un composé donneur de cation de terre rare avec le composé macropolycyclique ayant les caractéristiques définies ci-dessus, chaque composé étant avantageusement en solution, de préférence dans le même solvant ou dans des solvants compatibles inertes vis-à-vis de la complexation. En général on utilise comme solvant l'acétonitrile, le DMSO, l'éthanol.

A titre de composé donneur de cation de terre rare on peut utiliser un sel quelconque de terre rare, avantageusement un chlorure, un acétylacétonate ou un nitrate.

On opère avantageusement à la température d'ébullition du solvant.

Lorsque le complexe macropolycyclique formé est soluble dans le solvant réactionnel, il est isolé de la solution par évaporation à siccité. Si le complexe macropolycyclique formé cristallise dans le solvant réactionnel, il est séparé par filtration ou tout autre moyen classique approprié. Les complexes ainsi obtenus peuvent être purifiés par cristallisation.

On peut également effectuer la réaction ci-dessus en utilisant une solution du composé macropolycyclique et le composé donneur de cation sous la forme cristalline. Un agent synergique protecteur de désactivation peut également être introduit dans la sphère de coordination du cation, comme cela est décrit dans J. Chem. Phys. 40, 2790 (1964) et 41, 157 (1964).

Dans la suite de la présente description on désigne également par "cryptates" les complexes macropolycycliques selon l'invention et par "cryptand" le composé macropolycyclique seul. On adoptera ci-après la nomenclature telle que définie par LEHN pour désigner les cryptates et les cryptands. A cet effet on peut se référer notamment aux articles de J.M. LEHN dans Struct. Bonding (Berlin) 16,1 1973 et Acc. Chem. Res. 11. 49 (1978).

Tous les ions de terres rares conviennent aux fins de la présente invention. Toutefois, on préférera ceux qui présentent la fluorescence d'ion la plus intense, soit Terbium et Europium, et, à un degré moindre, Samarium et Dysprosium.

A titre de composés macropolycycliques qui conviennent aux fins de l'invention on peut utiliser les agents cryptands connus, par exemple :

1) les benzo-cryptands de formule générale :

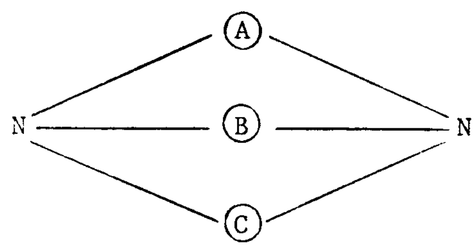

dans laquelle Ⓐ , Ⓑ et Ⓒ représentent indépendamment les uns des autres les groupes $2_B$, 2, 1 ayant les significations ci-après :

$2_B$ = $-C_2H_4-X_1-C_6H_4-X_2-C_2H_4-$ ou
$-C_2H_4-X_1-CH_2-C_6H_4-CH_2-X_2-C_2H_4-$
2 = $-C_2H_4-Y_1-C_2H_4-Y_2-C_2H_4-$
1 = $-C_2H_4-Z_1-C_2H_4-$

dans lesquels $X_{1,2}$, $Y_{1,2}$ et $Z_1$ représentent chacun un hétéroatome choisi parmi l'oxygène et le soufre, $X_1X_2$ et $Y_1Y_2$ pouvant être identiques ou différents.

A titre d'exemples de tels cryptands, on peut citer ceux dans lesquels les motifs Ⓐ , Ⓑ , Ⓒ , sont composés respectivement comme suit :

Ⓐ Ⓑ Ⓒ = ($2_B$11) ; ($2_B$21) ; ($2_B$22) ; ($2_B2_B$2) ; et le composé de formule :

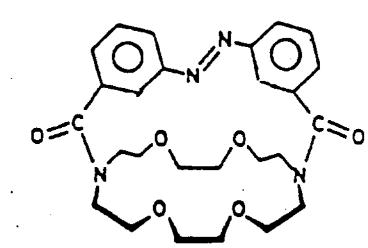

2) les agents cryptands comportant des hétérocycles azotés, tels que ceux décrits dans JACS 99 4683 (1977) de formule I dans laquelle :

- Ⓐ et Ⓑ, identiques, représentent la chaîne polyoxyéthylénée de formule $-(CH_2)_2-O-(CH_2)_2-O-(CH_2)_2-$
- Ⓒ est une chaîne hydrocarbonée comportant un hétérocycle azoté comme motif donneur d'énergie. A titre d'exemples de tels composés, on peut citer notamment les composés ci-après :
- (22) pyridine amide :
  2,6-[N,N',N,N'-bis(3,6-dioxaoctaméthylène) bis(aminocarbonyl)]-pyridine

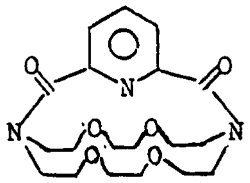

- (22) pyridine amine :
  2,6-[N,N',N,N'-bis(3,6-dioxaoctaméthylène) bis(aminométhyl)]-pyridine

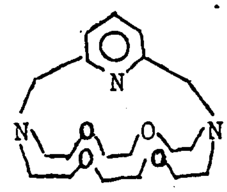

- (22) bipyridine:
  6,6'-[N, N', N, N'-bis(3,6-dioxaoctaméthylène)-bis(aminométhyl)]2,2'-bipyridine.

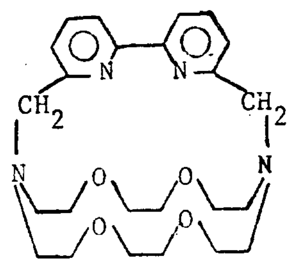

- (22) bipyridine amide :
  6,6'-[N, N', N, N'-bis(3,6-dioxaoctaméthylène)-bis(aminocarbonyl)]-2,2'-bipyridine.

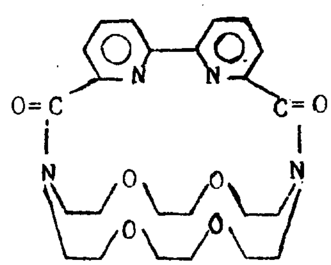

- (22) azopyridine :
  6,6'-[N, N', N, N'-bis(3,6-dioxaoctaméthylène)-bis(aminocarbonyl)]-2,2'-azopyridine.

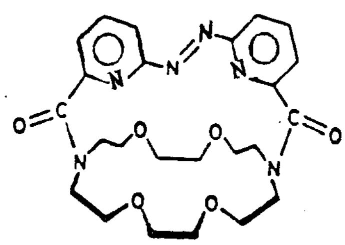

3) les agents cryptands, comportant plusieurs hétérocycles azotés comme motifs donneurs, tels que le composé de formule ci-après

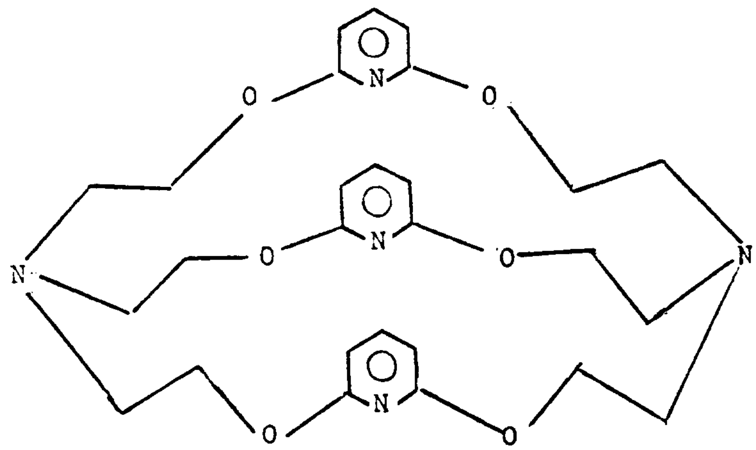

: décrit dans J. Am. Chem. Soc. 1979, 101 p. 1047.

4) Les agents cryptands polycycliques contenant des motifs aromatiques, par exemple les composés répondant à la formule ci-après :

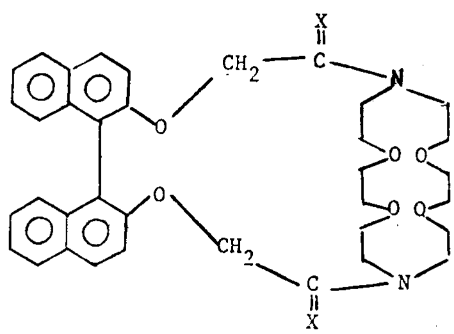

X = O, H

ou les composés de formule I dans lesquels la chaîne hydrocarbonée portant le ou les motifs donneurs est interrompue par un hétéromacrocycle tels que les composés de formules ci-après :

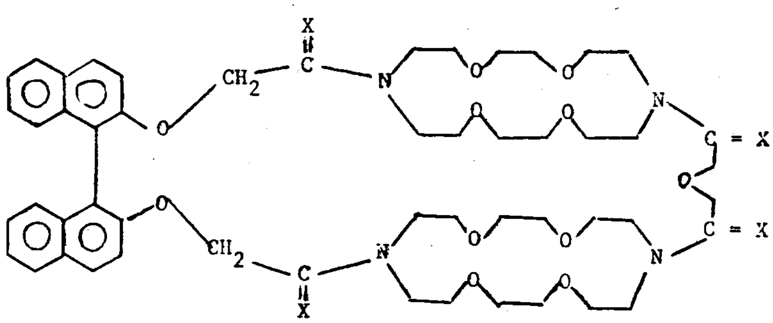

X = O, H

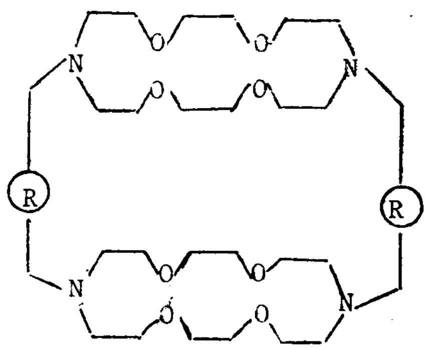

R =

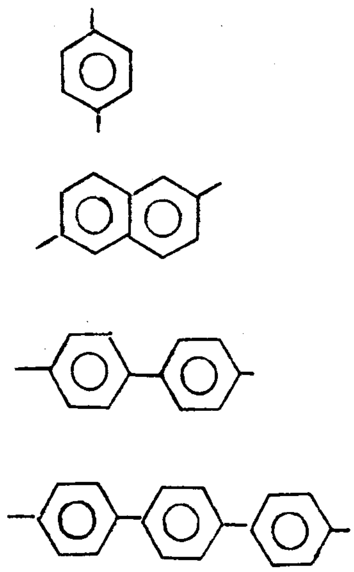

De tels composés sont décrits notamment dans :

- Angew. Chemie 86 443 (1974)

  Tet. letters 21 941 (1980) et Chem. Comm. 833, 1981.

On peut également utiliser comme agents cryptands les composés macropolycycliques de formule I ci-dessus dans laquelle le ou les motifs moléculaires possédant une énergie triplet supérieure à l'énergie de la terre rare à crypter sont choisis parmi la phénanthroline, l'anthracène, les bipyridines et les bi-quinoléines, éventuellement substitués en des positions appropriées et par des groupes susceptibles d'augmenter l'efficacité du transfert d'énergie ou de modifier le spectre d'excitation du cryptate de terre rare [J. Phys. Chem. 1964, vol. 68, p. 3324]. A titre d'exemple de tels groupes on citera notamment le groupe phényle.

Dans ces nouveaux composés macropolycycliques, au moins l'un des radicaux Ⓐ, Ⓑ ou Ⓒ répond de préférence à l'une des formules ci-après :

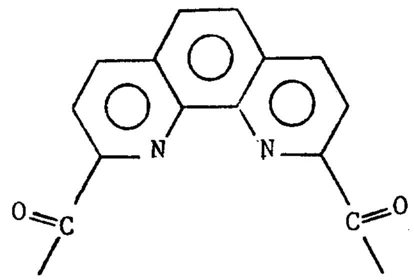 ; 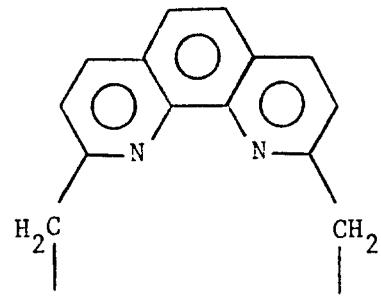

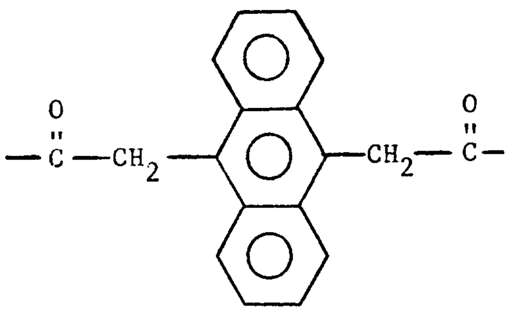 ; 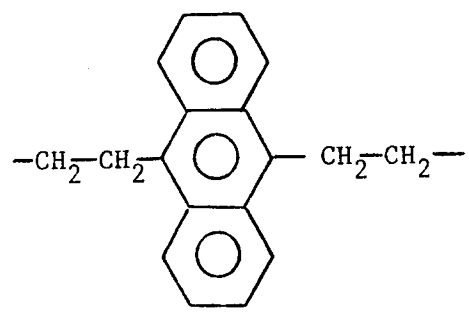

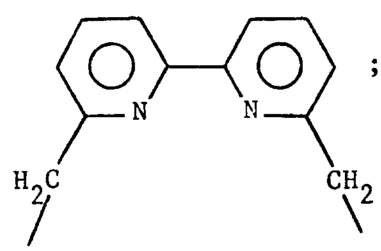 ; 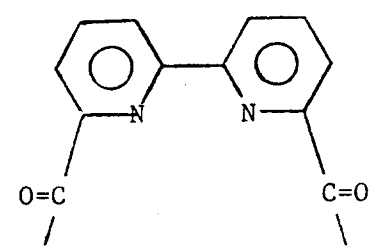 ;

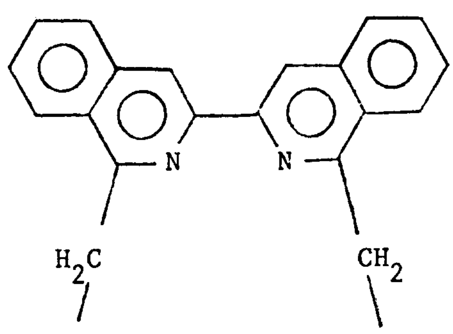 ou 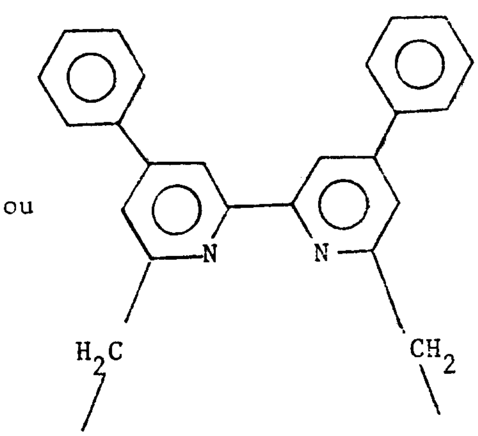

Une classe particulière de ces composés macropolycycliques est constituée par les composés de formule I, dans laquelle Z est l'azote, Ⓐ et Ⓑ sont deux chaînes mono- ou polyoxyéthylénées, de préférence dioxyéthylénées et Ⓒ répond à l'une des formules ci-dessus.

Parmi ces composés macropolycycliques nouveaux figurent également les composés de formule I dans laquelle Ⓐ et Ⓑ représentent chacun le groupe

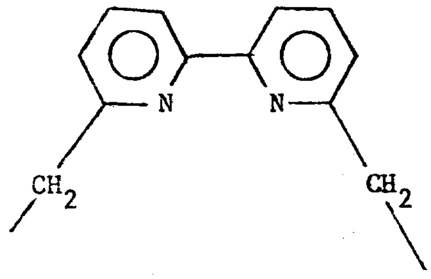

et Ⓒ est l'un des groupes suivants :

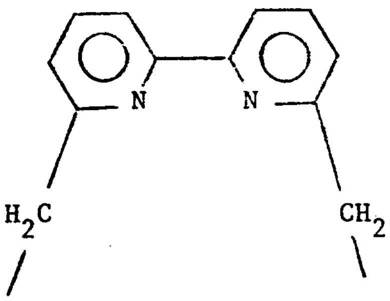

;

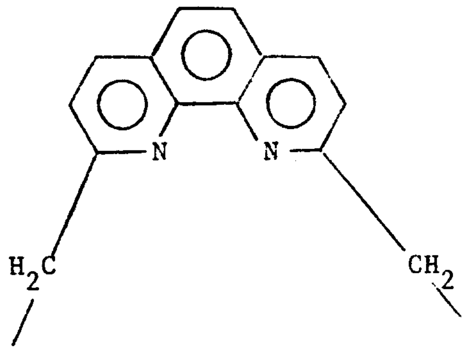

;

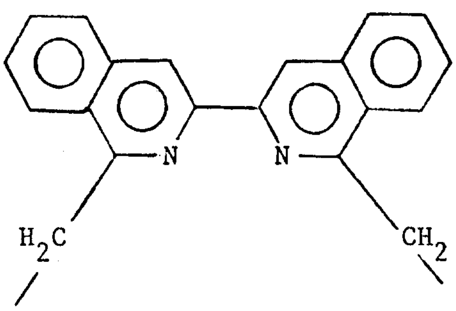

ou

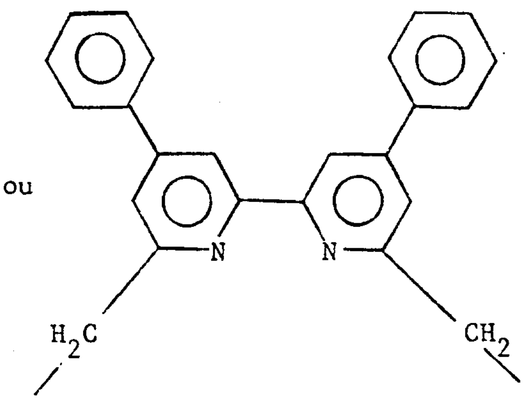

Une autre classe particulière de ces composés macropolycycliques nouveaux est constituée par les composés de formule I, dans laquelle Z est l'azote et Ⓐ, Ⓑ et Ⓒ sont identiques et représentent l'un des hétérocycles ci-dessus, notamment la 2,2'-bipyridine et la phénanthroline.

Les composés macropolycycliques de formule I, dans laquelle le ou les motifs moléculaires sont la phénanthroline, l'anthracène, les bipyridines ou les quinoléines sont des composés nouveaux à l'exception des composés de formule I dans laquelle Ⓐ et Ⓑ sont chacun une chaîne de formule $-(CH_2)_2-O-(CH_2)_2-O-(CH_2)_2-$et Ⓒ est le groupe de formule :

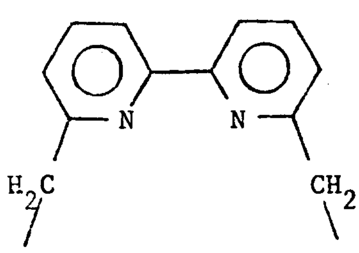

ou le groupe de formule

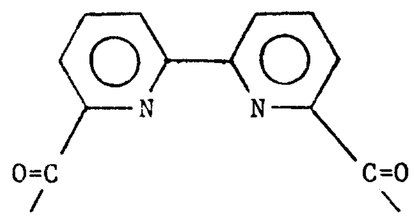

qui sont décrits dans Chem. Ber. 111, 200-204 (1978).

Les composés macropolycycliques de formule I peuvent être obtenus par des procédés chimiques connus, mettant principalement en oeuvre des réactions de condensation et/ou d'addition.

A titre d'exemples de tels procédés, on peut citer notamment les procédés décrits dans le brevet FR 70 21079 (2052947) et les brevets US 3 888 877, 3 966 766 et 4 156 683. Ces procédés conviennent notamment pour l'obtention de composés de formule I dans laquelle Z est l'azote ou le phosphore.

Pour l'obtention de composés de formule I dans laquelle Z est le carbone et R est tel que défini précédemment, on utilisera des procédés de condensation par analogie.

Dans tous les cas il suffit d'utiliser comme produits de départ des composés chimiques comportant chacun un des radicaux Ⓐ , Ⓑ ou Ⓒ et des groupes terminaux capables d'être substitués ou comportant des radicaux facilement éliminables.

A titre d'exemple on indiquera que le composé de formule I dans laquelle Z est le carbone, R est le groupe hydroxy et Ⓐ , Ⓑ et Ⓒ représentent chacun la 2,2'-bipyridine peut être obtenu par condensation de la 6,6'-dilithiobipyridine avec la 6,6'-dicyano-2,2'-bipyridine, hydrolyse du groupe cyano du macrocycle ainsi obtenu et condensation du produit résultant avec la 6,6'-dilithiobipyridine.

Selon un mode opératoire préféré les nouveaux composés macropolycycliques de formule I,dans laquelle Z est l'azote, Ⓐ et Ⓑ sont des chaînes polyoxyéthylénées, peuvent être obtenus par réaction :

- du macrocycle azoté constitué par les deux chaînes polyoxyéthylénées avec
- la chaîne hydrocarbonée comportant ledit motif moléculaire, ladite chaîne ayant des groupes terminaux clivables, tels que par exemple des groupes halogéno.

Cette réaction de couplage est avantageusement réalisée dans un solvant anhydre, par exemple le diméthylsulfoxyde (DMSO), l'acétonitrile, éventuellement en présence d'un agent réducteur, tel que l'hydrure de sodium ou le carbonate de sodium. On obtient alors le composé macropolycyclique sous la forme d'un sel de sodium.

On opère de préférence à une température inférieure au point d'ébullition du solvant, par exemple entre 60 et 100 °C.

Pour obtenir le composé macropolycyclique libre, on peut faire réagir le sel de sodium ainsi obtenu avec du nitrate d'argent pour former le complexe macropolycyclique d'argent correspondant, qui est ensuite traité avec un courant de $H_2S$. Le précipité forme, est ensuite neutralisé avec une solution de $N(CH_3)_4OH$ et extrait avec du chlorure de méthylène.

On notera que le complexe macropolycyclique de terre rare selon l'invention peut être obtenu à partir du complexe libre ou d'un sel de celui-ci, par exemple le sel de sodium.

Dans le procédé ci-dessus on peut utiliser avantageusement comme macrocycle l'un quelconque des composés macrocycles monocycliques azotés décrits dans le brevet US 3 966 766, les composés préférés étant : le 1,7,10,16-tétraoxa-4,13-diazacyclooctadécane de formule générale :

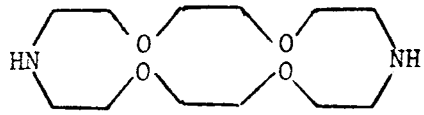

ou le 1,7,16-trioxa-4,13-diazacyclodécane de formule générale :

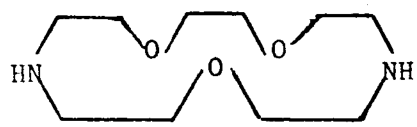

On notera que les complexes préférés selon l'invention, obtenus à partir des macrocycles ci-dessus, peuvent être considérés comme des dérivés de cryptates de type 222 ou 221, dans lesquels les motifs éther sur au moins l'une des chaînes hydrocarbonées ont été substitués par des motifs donneurs d'énergie (en général aromatiques ou polyaromatiques pouvant contenir des hétéroatomes).

Les cryptates d'europium de types 222 ou 221 possèdent des vitesses de dissociation très faibles en solutions aqueuses, bien que leur constante de formation soit relativement basse ($K = 10^{6,8}$ et $10^{5,9}$ respectivement pour les cryptates 221 et 222 - Inorganic Chem. 1981, 20 p. 616 et J.A.C.S. 1980 102 p. 22 78). De tels cryptates ne répondent pas aux critères de stabilité définis dans l'art antérieur. Par contre, la substitution des motifs éther par des motifs donneurs d'énergie, qui fournit les complexes préférés selon l'invention, ne modifie pas la caractéristique de dissociation de ces cryptates, mais permet par l'excitation du motif donneur d'énergie dans sa bande d'absorption d'obtenir une fluorescence fortement exaltée et caractéristique de la terre rare cryptée.

Lorsque les cryptates de terres rares, objet de la présente invention, sont utilisés pour marquer spécifiquement des molécules biologiquement actives à l'aide d'une liaison covalente, ils peuvent être substitués sur l'un ou plusieurs de leurs atomes constitutifs par un ou plusieurs substituants suffisamment accessibles et possédant un ou plusieurs motifs moléculaires permettant le couplage covalent avec la molécule biologique dans des conditions opératoires compatibles avec son intégrité biologique.

Parmi ces motifs moléculaires on peut citer, à titre d'exemples non limitatifs, les radicaux alkylamino, arylamino, isothiocyano, cyano, isocyano, thiocyano, carboxyle, hydroxyle, mercapto, phénol, imidazole, aldéhyde, époxyde, halogénure : de thionyle, de sulfonyle, de nitrobenzoyle, de carbonyle ; triazo, succinimido, anhydride, halogénoacétate, hydrazino, dihalogénotriazinyle etc. (Biol. Chem. 245, 3059 (1970)). La longueur du bras liant le complexe macrocyclique à la molécule d'intérêt biologique peut varier par exemple de 1 à 20 atomes et contenir des atomes de carbone mais aussi des hétéroatomes tels que N, O, S, P. L'invention concerne donc également les complexes biologiques constitués d'une molécule biologique associée par couplage ou adsorption à un complexe macropolycyclique selon l'invention.

Pour réaliser le couplage on peut utiliser tous les réactifs décrits à cet effet dans la littérature et séparer la molécule marquée du complexe macropolycyclique n'ayant pas réagi par tout moyen de séparation adapté (filtration sur gel par exemple).

Parmi les molécules d'intérêt biologique présentant un intérêt pour le marquage avec l'objet de la présente invention on pourra citer de façon non limitative les antigènes, les anticorps, les anticorps monoclonaux, les fragments et combinaisons Anticorps-fragments, les drogues, les récepteurs, les hormones, les récepteurs hormonaux, les bactéries, les stéroïdes, les aminoacides, les peptides, les vitamines, les virus, les nucléotides ou poly-nucléotides dans les méthodes d'hybridation, les enzymes et leurs substrats, les lectines, les acides nucléiques, l'ADN et l'ARN.

Les complexes macropolycycliques de terres rares selon la présente invention trouvent une application importante à titre de marqueurs fluorescents dans les dosages immunologiques, aussi bien dans les méthodes de dosage dites par compétition ou par excès, en phase homogène ou hétérogène, décrits dans l'art antérieur (LANDON, Ann. Clin. Biochem. 1981, 18, p. 253 et SOINI Clin. Chem. 25, 353, 1979).

Dans les méthodes hétérogènes on peut utiliser avantageusement :

- des tubes revêtus d'anticorps spécifiques de la substance à doser et réaliser la lecture de la fluorescence par les méthodes décrites plus haut, directement au travers du tube (Clin. Chem. 29, 60, 1983),
- ou une phase solide autre, en particulier une bandelette ou un film gélatineux sur lequel on a déposé préalablement un milieu contenant un anticorps spécifique, la lecture de la fluorescence étant réalisée à un angle différent de l'excitation et de la réflexion de l'onde excitatrice ou directement au travers du support, si celui-ci est transparent.

Il est également possible en raison du spectre de raie de ces marqueurs de détecter simultanément plusieurs antigènes soit en utilisant des cryptates de différentes terres rares dont les raies de fluorescence ne se recouvrent pas (par exemple Tb et Eu), soit en utilisant des marqueurs fluorescents classiques (fluorescéine ou rhodamine) et des marqueurs selon la présente invention.

Une autre application concerne l'immunochimie où la fluorescence de la cellule marquée est détectée par microscopie comme décrit par R.C. NAIRN dans "Fluorescent Protein Tracing Longman Group Ltd" 1976, avec également la possibilité de faire de la multidétection.

Une autre application des complexes de terres rares selon l'invention concerne la cytologie et les trieurs de cellules ou' l'utilisation d'un marqueur présentant un spectre de raie à longueur d'onde élevée couplée à celle de marqueurs classiques permet de réaliser des analyses multiparamétriques.

De plus, avec un agent cryptand donné et des ions de terres rares différentes il est possible avec une seule longueur d'onde d'excitation qui est celle du motif moléculaire qui transfère l'énergie et qui peut être générée avec une seule source (Laser par exemple) d'obtenir deux spectres de fluorescence de raies caractéristiques des deux ions cryptés (par exemple Tb et Eu) et révélant ainsi les molécules biologiques respectives sur lesquelles les cryptates sont fixés.

Une autre application concerne l'utilisation des cryptates de terres rares selon la présente invention dans le domaine du génie génétique par exemple à titre d'indicateur dans les réactions d'hybridation, telles que celles décrites dans les demandes de brevets EP-A-0 070 685 et 0 070 687.

L'invention va être maintenant décrite plus en détail par les exemples illustratifs non limitatifs ci-après.

Exemple 1 Préparation du (22) phénanthroline : 2,9-[N,N',N,N' -bis(3,6-dioxaoctaméthylène)bis-(aminométhyl)]-1,10-phénanthroline (dénommée ci-après : (22) phèn)

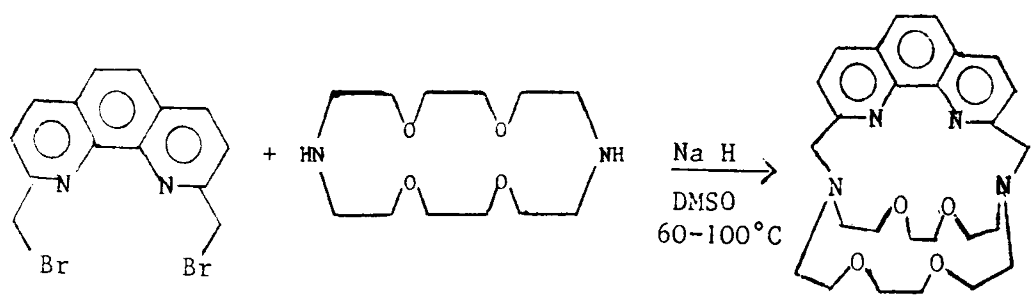


di $CH_2$ Br phèn    macrocycle $N_2O_4$    (22)phèn

Le solvant utilisé dans cette synthèse est le DMSO qui a été séché plusieurs jours sur tamis moléculaire 4 Å, et éventuellement distillé sous vide.

1 mmol (366 mg) de composé dibromométhylphénanthroline (di $CH_2$ Br Phèn) sec et fraîchement chromatographié a été dissous dans 10 à 20 ml de DMSO. Il a été transféré dans une ampoule à addition sèche ; le volume a été complété à 100 ml avec du DMSO sec.

On a placé 100 à 200 mg de NaH dans de l'huile (FLUKA) dans un ballon sec ; on a ajouté 5 ml de toluène sec (ou hexane) au mélange et on a laissé décanter. La solution a été évaporée sous vide (1 Torr) pendant 1 h. On a redissous NaH dans 20 ml de DMSO sec (dégagement $H_2$). On peut éventuellement chauffer à 50 °C.

On a séché 1 mmol du macrocycle $N_2O_4$ (262 mg) et on a ajouté 10 à 20 ml de DMSO sec.

Les solutions de NaH et $N_2O_4$ macrocycle ont été chacune introduites dans une ampoule à addition et le volume a été complété à 100 ml par du DMSO sec.

Dans un ballon tricol on a placé 100 ml de DMSO sec. On a équipé ce ballon avec les deux ampoules à addition, un réfrigérant à reflux et un système désséchant (Silicagel). Le ballon a été chauffé à 60-100 °C avec une agitation magnétique.

Pendant 1 à 2 h on a ajouté goutte à goutte et simultanément le contenu des deux ampoules.

Le mélange réactionnel a été chauffé 1 h après l'addition des réactifs. Le mélange a ensuite été distillé sous vide pour éliminer le solvant.

Il est possible d'ajouter une petite quantité d'eau pour éliminer les hydrures et les bases.

Le mélange réactionnel a été amené à sec ; on a obtenu un résidu rouge sec ou pâteux.

On a ajouté 100 à 200 ml de $CH_2Cl_2$ ; la pâte a été triturée à température ambiante pendant au moins 30 min. Le résidu à été filtré et lavé. La phase liquide organique a été conservée et évaporée sous vide. Le résidu obtenu a été séché sous vide (1 Torr, au moins 30 min).

On a ajouté 100 ml d'éther éthylique au résidu, on a mélangé, on a laissé décanter ; cette opération a été répétée 3 à 4 fois.

Pour éliminer de façon plus complète le reste du monocycle $N_2O_4$ on a ajouté au résidu 50 ml d'hexane et 1 ml de $CH_3OH$ ; la solution a été évaporée sous vide (chauffage léger) jusqu'à l'apparition d'un trouble ; la solution a été décantée et on a répété l'opération plusieurs fois.

Le résidu rouge a été testé par chromatographie sur plaque (Polygram Alox N/$UV_{254}$ de Macherey-Nagel), la migration en milieu $CH_2Cl_2/CH_3OH$ 9/1 a donné les valeurs suivantes :

(22) Phèn Rf = 0,4 - 0,8

Monocycle $N_2O_4$ Rf = 0,2 - 0,5

Le résidu a été chromatographié sur colonne (20 cm ⌀ 15 cm) garnie d'oxyde d'aluminium 90 activé Neutre, 70-230 mesh (Merck), l'éluant étant constitué des mélanges :

$CH_2Cl_2$:$CH_3OH$ (5 %), $CHCl_3/C_2H_5OH$ (5 %), $CH_3OH$/hexane 9 : 1

Rendements de 12 à 35 % suivant les conditions opératoires.

On a ainsi obtenu le sel de sodium du (22)phèn de formule [$Na^+$C(22 phèn)Br ; $H_2O$] dont l'analyse élémentaire était la suivante :

| | | | |
|---|---|---|---|
| trouvé : | C 53,00 | H 6,21 | N 9,44 |
| calculé: | C 53,16 | H 6,18 | N 9,55 |

B - Formation du complexe [$Eu^{3+}$C(22)phèn]

0,07 mol(19,4 mg) de $EuCl_3$ anhydre ont été mis en solution dans 4 ml de $CH_3CN$ sec et on a chauffé à reflux pendant 1 h 30. On a ajouté 35 mg du sel de sodium du (22)phèn obtenu précédemment dans 2,5 ml $CH_3CN$. On a chauffé à reflux pendant 2 h 30. Après avoir laissé le mélange reposer une nuit à 4 °C on a filtré le précipité jaune.

Le précipité recueilli (11 mg) présente une forte fluorescence de même quand il est en solution dans $H_2O$ et $CH_3OH$ (UV 254 nm).

On n'a pas noté de modification des spectres d'excitation et d'émission après trois mois en solution aqueuse à une concentration inférieure à $10^{-4}$ mol/l.

Exemple 2 Préparation du (22) phénanthroline amide : 2,9-[N,N', N,N'-bis(3,6-dioxaoctaméthylène)bis-(aminocarbonyl)]-1,10-phénanthroline (dénommée ci-après (22) phèn amide)

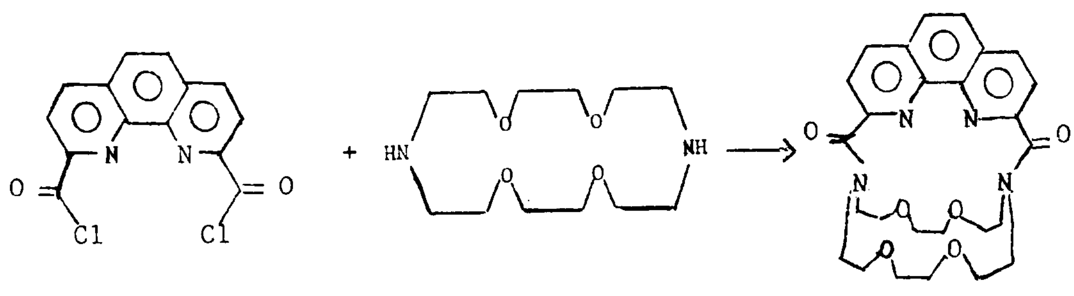

diCOCl phèn (22) (22)phèn amide

Le solvant utilisé dans cette synthèse est l'acétonitrile distillé sur $P_2O_5$. On a opéré dans un appareillage en verre anhydre.

305 mg de di COCl phèn (1 mmol) recristallisé et sec ont été dissous dans 50 ml de $CH_3CN$ ; après une nuit, la solution a été filtrée et transférée dans une ampoule à addition ;le volume a été complété à 90 ml par $CH_3CN$.

524 mg de (22) (2 mmol) ont été séchés sous vide (< 1 Torr ; pendant une nuit). Le produit a été dissous dans 90 ml de $CH_3CN$ et transféré dans une ampoule à addition.

Les deux ampoules ont été montées sur un ballon de 2 à 3 l contenant 1 litre de $CH_3CN$. L'addition simultanée des deux réactifs s'est effectuée en 2 h à 2 h 30 sous agitation rapide (magnétique). L'agitation a été poursuivie 30 min après l'addition. Le solvant a été éliminé sous vide à 50 °C. Le résidu a été séché 1 h au moins à P < 1 Torr. Le résidu a été brassé 30 min dans 300 ml de $CH_2Cl_2$ et filtré. Le filtrat a été

évaporé à sec. Il a été chromatographié sur Alox activé 90 neutre (colonne 30 cm ∅ 1,5 cm), l'éluant étant $CH_2Cl_2$ contenant 1 % $CH_3OH$. On a récupéré le premier produit élué.

On a récupéré 310 mg de (22)phèn amide avec un rendement de 63 %. Le produit a été recristallisé dans le mélange toluène/hexane 1 : 1.

T.fusion : 300 - 302 °C
I R bande amide à 1630 $cm^{-1}$
Spectre de masse $M^+$ à 494 pour PM = 494,55

| Analyse élémentaire : $C_{26}$ $H_{30}$ $N_4$ $O_6$ | C | H | N |
|---|---|---|---|
| Calculé | 63,15 | 6,11 | 11,33 |
| Trouvé | ( 59,8 | 5,7 | 10,5 |
| | ( 59,9 | 5,9 | 10,6 |

B - Formation du complexe [$Eu^{3+}$C(22)phèn amide]

45 mg (0,17 mmol) de $EuCl_3$ anhydre ont été dissous dans 10 ml $CH_3CN$ sec. On a chauffé à reflux pendant 3 h 80 mg (0,16 mmol) de (22)phèn amide dissous dans 2 ml de $CH_2Cl_2$ ; on a ajouté 50 ml $CH_3CN$ ; on a chauffé à ébullition du $CH_3CN$ ($CH_2Cl_2$ est éliminé par évaporation). On a alors ajouté la solution de $Eu^{3+}$.

On a chauffé à reflux pendant 2 h puis on a laissé reposer à température ambiante. Le précipité blanc a été récupéré par filtration (50 mg). Il présentait une forte fluorescence rouge (λ excitation : 254 nm) ainsi qu'en solution dans $H_2O$, $CH_3OH$ et DMSO.
Analyse élémentaire pour Eu C (22)phèn amide (OH) $Cl_2$. $2H_2O$

| $C_{26}H_{37}N_4O_{10}Cl_2Eu$ PM = 788,47 | C | H | N |
|---|---|---|---|
| Calculé | 39,61 | 4,73 | 7,11 |
| Trouvé | ( 40,1 | 4,9 | 7,2 |
| | ( 40,3 | 4,8 | 7,1 |

On n'a pas noté de modifications des spectres d'excitation et d'émission de ce complexe après trois mois en solution aqueuse à une concentration inférieure à $10^{-4}$ mol/l.

Exemple 3 Préparation du (22) anthracène amide : 9,10-[N,N',N,N' -bis (3,6-dioxaoctaméthylène)bis-(carbamoylméthyl)]anthracène

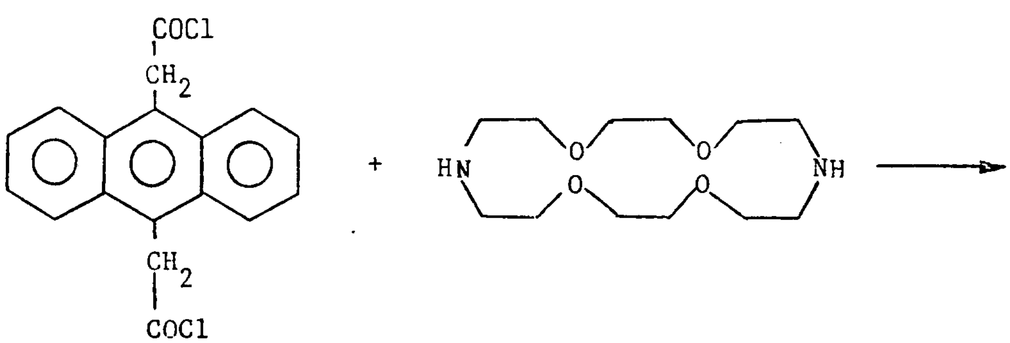

1 2

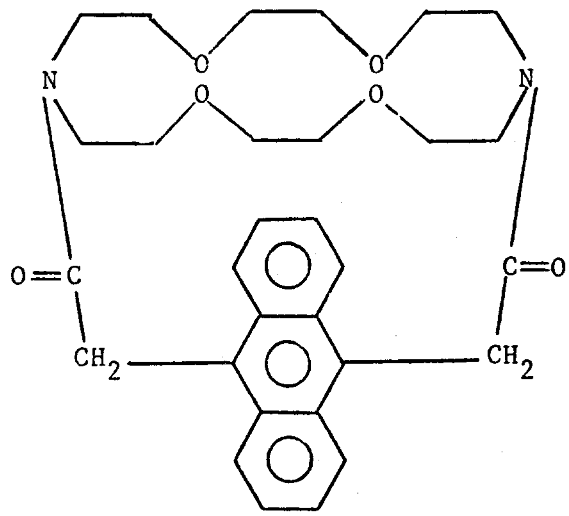

3

Dans cet exemple, on fait appel à la méthode de haute dilution.

- 0,564 g du dichlorure d'acide 1 (1,7 mmol) ont été dissous dans 70 ml de $CH_2Cl_2$ anhydre et introduits dans une ampoule à addition de 100 ml,
- 0,446 g du macrocycle (22), soit 1,7 mmol, et 0,47 ml de triéthylamine (2 x 1,7 mmol) ont été également dissous dans 70 ml de $CH_2Cl_2$ anhydre et introduits dans une ampoule à addition de 100 ml.

Dans un tricol de 3 l, on a introduit 0,4 ml de toluène et 0,15 l de chlorure de méthylène anhydre. L'introduction simultanée dans le tricol des deux réactifs préparés ci-dessus dans les ampoules à addition s'est effectué en 5 h (14 ml/h).

On a recueilli la phase organique, que l'on a concentrée jusqu'à quelques millilitres, puis passée sur une colonne de $SiO_2$ sous pression (diamètre de la colonne 3,5 cm ; hauteur = 17 cm ; éluant $CH_2Cl_2$ - 1 % méthanol).

On a obtenu un produit fluorescent jaune pâle cristallisé.

Rendement : 45 %

Chromatographie en couche mince (CCM)

solvant $CH_2Cl_2$ / 2 % méthanol ; $SiO_2$ ; Rf = 0,6

RMN $^1H$ : solvant $CDCl_3/CD_2Cl_2$ 5/5 à 200 MHz

| | | | |
|---|---|---|---|
| ppm | 2,53 (m) | } 24 H, $HCH_2$ + $OCH_2$ couronne | |
| | 2,93 (m) | | |
| | 3,18 (m) | | |
| | 3,42 (m) | | |
| | 4,02 (d) | | |
| | $\delta_A$ = 4,62 | $J_{AB}$ = 16 Hz 4H $OCCH_2N$ | |
| | $\delta_B$ = 5,03 | | |
| | 7,6 (m) | 4H | } aromatique |
| | 8,43 (d) | 2H | |
| | 8,82 (d) | 2H | |

| microanalyse : $C_{30}H_{36}O_6N_2$ | | | |
|---|---|---|---|
| Calculé<br>Trouvé | C 69,20<br>C 69,07 | H 6,97<br>H 7,00 | N 5,38<br>N 5,22 |

Poids moléculaire 520,55

Le dichlorure d'acide 1 utilisé comme produit de départ dans cet exemple peut être obtenu par les procédés décrits par M.W. Miller et al. dans R.W. Amidon, P.O. TAWNEY J.A.C.S. 77 2845 (1955) ou par B.M. MIKHAILOV Izvest. Akad. Nank. SS. Osdel Khim. Nank. 1948, 420-6 ; CA 42, 6350.

Exemple 4 Préparation du (22) anthracène : 9,10-[N,N',N,N'-bis (3,6-dioxaoctaméthylène)bis(aminoéthyl)]-anthracène

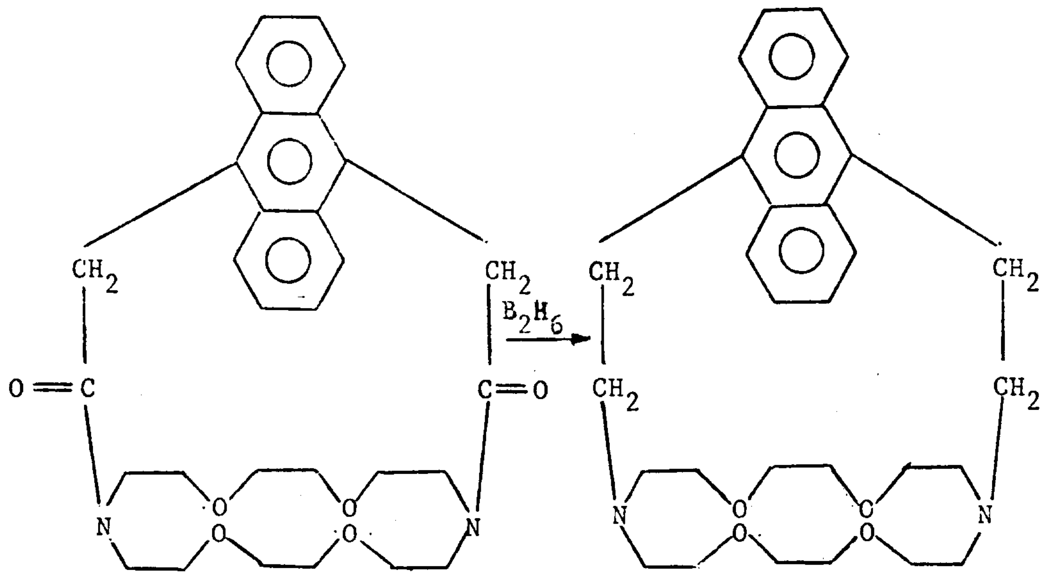


3 4

380 mg du diamide 3 (0,73 mmol) ont été partiellement dissous dans 20 ml de THF anhydre contenus dans un ballon.

On a mis le ballon avec un reflux sous atmosphère d'argon et on a ajouté à température ambiante 8 ml de $B_2H_6$ 1,1 M dans du THF anhydre.

On a maintenu le tout sous reflux pendant une nuit puis on a détruit à 0 °C l'excès de diborane par quelques gouttes d'eau distillée.

On a évaporé les solvants et on a ajouté au solide blanc résiduel 40 ml d'une solution 6 N d'HCl.

On a chauffé à reflux pendant 30 h sous atmosphère d'argon, la solution est devenue vert foncé.

On a laissé refroidir, puis on a évaporé l'eau et on a essoré le solide résultant à la pompe à palette pendant 1 h, puis on l'a dissous dans 50 ml d'eau distillée ; on a ajouté 50 ml de $CH_2Cl_2$ et on a agité les deux phases, puis on a séparé la phase aqueuse que l'on a basifiée à 0 °C par une solution aqueuse de LiOH jusqu'à pH 13 ; un solide a précipité. On a ajouté encore 50 ml de $CH_2Cl_2$, on a agité les deux phases et on a laissé décanter. On a récupéré la phase organique que l'on a séchée sur $MgSO_4$.

On a passé le brut sur une colonne d'alumine et on a élué avec $CH_2Cl_2$ 1 % méthanol. On a récupéré un produit cristallin pur sur plaque de chromatographie couche mince.

Rendement : 62 - 70 %

CCM : $Al_2O_3$ ; éluant $CH_2Cl_2$ 6 % MeOH

Rf : 0,33

Point de fusion : > 260 °C

RMN $^{13}C$ : solvant $CDCl_3$

ppm 25,2 ($CH_2$ aromatique)

54,3 )
56,3 ) $NCH_2$ macrocycle + branche

69,3 )
70,0 ) $OCH_2$ macrocycle

123,9 (1)
125,5 (2)
129,9 (3)
131,9 (4)

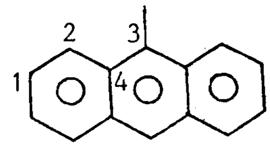

RMN $^1H$ : solvant $CDCl_3$

ppm 2,39 (t) 8H $NCH_2$ macrocycle

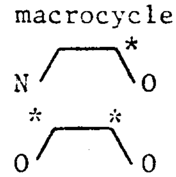

2,55 et 2,76 (m) - 2 x 4H $OCH_2$

2,89 et 3,19 (m) - 2 x 4H $OCH_2$

3,03 et 3,82 (t) - 2 x 4H - $CH_2$ - $CH_2$

7,47 et 8,31 (AB) - 4H )
7,50 et 8,36 (AB) - 4H ) H aromatiques

| microanalyse : $C_{30}H_{40}O_4N_2$ (poids moléculaire 492,6) | | | |
|---|---|---|---|
| Calculé :<br>Trouvé : | C 73,13<br>C 73,06 | H 8,18<br>H 8,04 | N 5,6<br>N 5,2 |

En opérant selon le mode opératoire décrit à l'exemple 1 on a préparé le complexe [$Eu^{3+}$ C(22)-anthracène].

Exemple 5 Préparation du composé macropolycyclique de formule (7) : 6,6',6'',6''',6'''',6'''''-bis[nitrilotri-(méthylène)]tris(2,2'-bipyridine) et du complexe d'europium correspondant

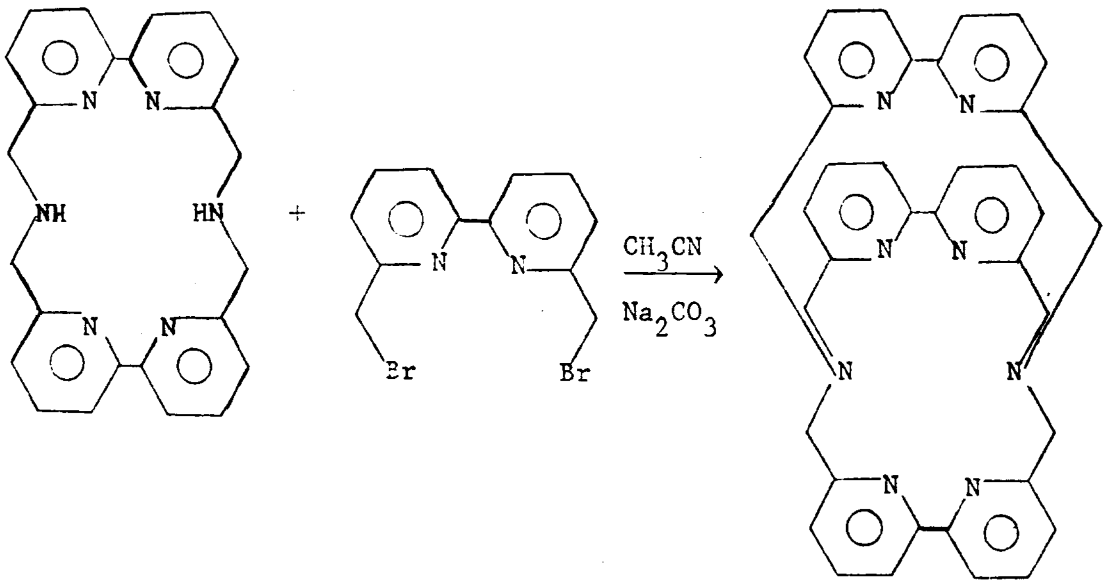


5 6 7

A - Préparation du 6,6'-bis-bromométhyl-2,2'-bipyridine

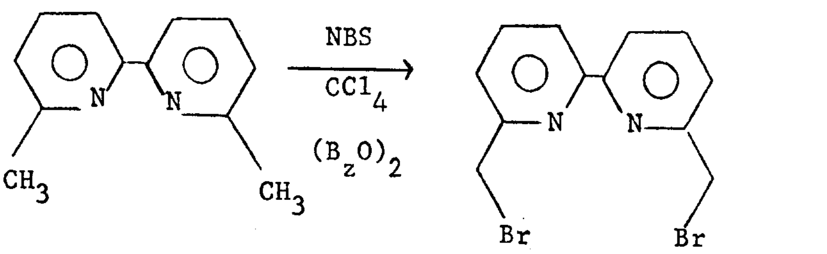


poids moléculaire 184 $C_{12}H_{12}N_2$

poids moléculaire 342 $C_{12}H_{10}N_2Br_2$

Un mélange de 6,6'-diméthyl-2,2'-bipyridine (2,76 g, 15 mmol) et de N-bromosuccinimide (5,10 g, 28,6 mmol) dans $CCl_4$ (150 ml) est porté au reflux pendant 30 min. Puis on a alors ajouté au mélange du péroxyde de benzoyle (30 mg) et le mélange a été à nouveau porté à reflux pendant 2 h, puis le succinimide a été filtré. La solution a été refroidie jusqu'à 0 °C et le solide filtré et lavé avec du méthanol pour donner du bis-dibromure sous la forme d'un solide blanc cristallin (1,65 g).

Rendement 32 % ; point de fusion 180-181 °C.

La solution de $CCl_4$ a été concentrée et chromatographiée sur colonne (gel de silice) par élution avec un mélange dichlorométhane/méthanol 98 : 1 pour donner :

- 6,6'-bis-dibromométhyl-2,2'-bipyridine 0,9 g 12 %
- 6,6'-bis-bromométhyl-2,2'-bipyridine 1,38 g 27 %
- 6-méthyl-6'-bromométhyl-2,2'-bipyridine 0,55 g 14 %

B - Préparation du composé macropolycyclique

Un mélange du macrocycle bis-bipyridine de formule (5) (0,15 g, 0,38 mmol) (décrit dans J. Org. Chem. 1983, 48, 4848), et de $Na_2CO_3$ (0,4 g) dans l'acétonitrile (200 ml) a été chauffé au reflux et ensuite une solution de 6,6'-bis-bromométhyl-2,2'-bipyridine (0,12 g, 0,38 mmol) a été ajoutée pendant une période de 3 h. Ensuite, le mélange a été porté au reflux pendant 20 h. Le $Na_2CO_3$ a été filtré et le filtrat évaporé. Le résidu a été filtré sur une courte colonne d'alumine en éluant avec $CH_2Cl_2$/MeOH 98 : 2 pour obtenir le sel de sodium du macrobicycle tris-bipyridine sous la forme d'un solide blanc (0,16 g ; 73 % ; point de fusion supérieur à 270 ° C).

| microanalyse : $C_{36}$ $H_{30}$ $N_8$, Na Br (677,6) | | | |
|---|---|---|---|
| Calculé : | C 63,81 | H 4,46 | N 16,53 |
| Trouvé : | C 63,79 | H 4,48 | N 16,49 |

RMN $^1H$ : solvant $CDCl_3$
3,85 (s, $6CH_2$) ;
7,33 (dd, J = 7,2 ; 1,2 ; 6H ; H-C(5) ; H-C(5'))
7,82 (t, J = 7,2 ; 6H ; H-C(4) ; H-C(4'))
7,90 (dd, J = 7,2 ; 1,2 ; 6H ; H-C(3) ; H-C(3'))

Un mélange de nitrate d'argent $AgNO_3$ (15 mg, 0,08 mmol) et du sel de sodium obtenu ci-dessus (20 mg, 0,03 mmol) a été chauffé avec 5 ml de $CH_3OH$ pendant 30 min. Le méthanol a été évaporé et le complexe résultant a été purifié par passage sur une colonne de gel de silice avec $CH_2Cl_2$/$CH_3OH$ comme éluant (96:4).

Le complexe d'argent ainsi obtenu (20 mg) a été dissous dans un mélange eau/méthanol (1:1 - 5 ml) et traité avec un courant de $H_2S$ pendant 15 min. Le précipité formé a été centrifugé et la solution a été neutralisée avec $N(CH_3)_4OH$ 0,1N et extraite avec du chlorure de méthylène (3,5 ml). La solution a été séchée sur $MgSO_4$ et évaporée ; le solide résultant a été filtré sur gel de silice $CH_2Cl_2$ : $CH_3OH$ (96:4) pour donner le complexe libre (13 mg ; 86 %) ayant les caractéristiques suivantes :
RMN $^1H$ : solvant $CDCl_3$

3,82 (s, 12H, $CH_2$)
7,44 }
7,78 } système ABX J=8 , 7,5 et 0,9Hz)
7,83 }

| microanalyse : $C_{36}$ $H_{30}$ $N_8$ (574,7) | | | |
|---|---|---|---|
| Calculé : | C 75,24 | H 5,26 | N 19,50 |
| Trouvé : | C 75,37 | H 4,98 | N 19,41 |

A partir du composé macropolycyclique obtenu, on a préparé le complexe [$Eu^{3+}$C (macropolycycle tris-bipyridine)] en opérant selon le mode opératoire décrit à l'exemple 1.

Les longueurs d'ondes d'excitation et d'émission caractéristiques de ce complexe figurent dans le tableau ci-après.

Exemple 6 Préparation-du composé macropolycyclique bis-bipyridine phénanthroline de formule (9) : 2,9-[N,N',N,N'-[bis(2,2'-bipyridine -6,6'-diméthyl)]bis(aminométhyl)]-1,10-phénanthroline et du complexe d'europium correspondant

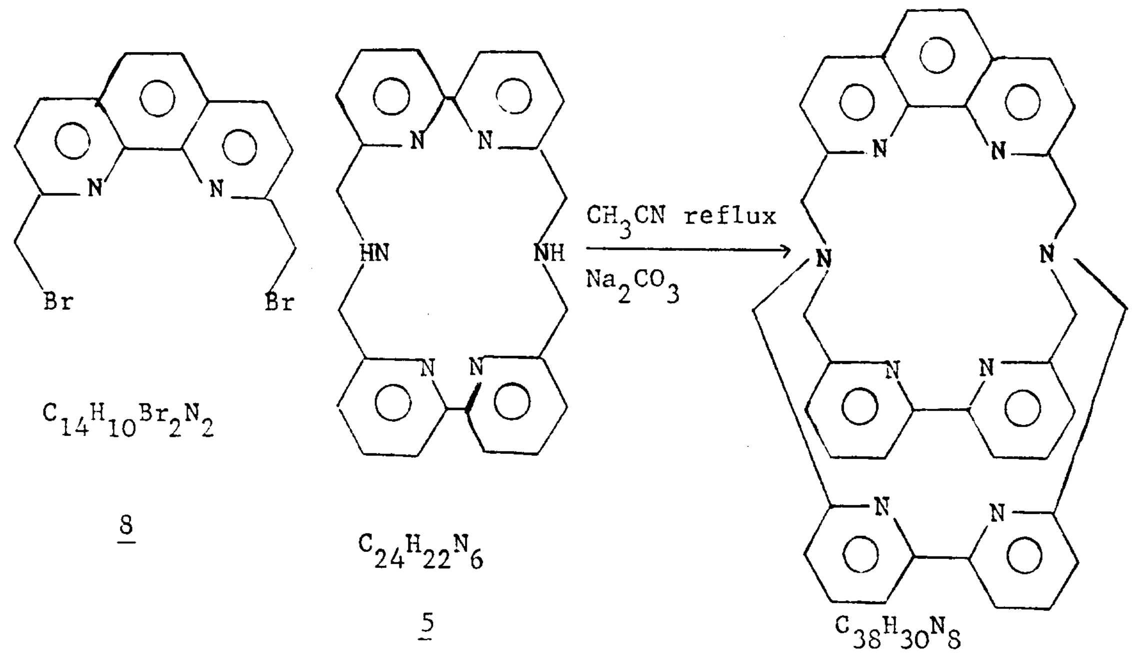


$C_{14}H_{10}Br_2N_2$ 8

$C_{24}H_{22}N_6$ 5

$C_{38}H_{30}N_8$ 9

Dans un ballon bicol de 500 ml, on a pesé 0,158 mmol du macrocycle bis-pyridine (5). On a ajouté 0,75 mmol de $Na_2CO_3$ (excès 5 fois environ) et 105 ml de $CH_3CN$ fraîchement distillé. On a chauffé à reflux et sous agitation magnétique pendant 30 min.

On a ajouté alors goutte à goutte la solution de dibromophénanthroline équimoléculaire dans 100 ml d'acétonitrile.

L'addition assez lente (2 h 10 min) a eu lieu tout en maintenant le reflux et l'agitation magnétique.

On a chauffé alors encore 18 h sous les mêmes conditions. La solution obtenue a été évaporée à sec. Le produit brut en solution dans $CH_2Cl_2$ a été lavé à l'eau. (chromatographie couche mince sur alumine : éluant : $CH_2Cl_2$/MeOH : 90/10).

Ce produit brut a été purifié sur colonne d'alumine standardisée (Activité II-III). L'éluant est $CH_2Cl_2$/MeOH : 98/2.

| microanalyse : $C_{38}$ $H_{30}$ $N_8$, NaBr, $H_2O$ (719,6) | | | |
|---|---|---|---|
| Calculé : | C 63,42 | H 4,45 | N 15,57 |
| Trouvé : | C 62,77 | H 4,46 | N 15,31 |

RMN $^1H$ : solvant $CDCl_3$
3,91 (s, 8H, $CH_2$-bpy)
4,07 (s, 4H, $CH_2$-phèn)
7,36 (dd, J = 7,2 ; 1,3 ; 4H ; H-C(5) ; H-C(5') de la bipyridine)
7,64 (d, J = 8,2 ; 2H ; H-C(3) ; H-C(8) de la phénantroline)
7,78 (s, 2H, H-C(5) ; H-C(6) de la phénantroline
7,83 (t, J = 7,2 ; 4H ; H-C(4) ; H-C(4') de la bipyridine)
7,91 (dd, J = 7,2 ; 1,3 ; 4H ; H-C(3) ; H-C(3') de la bipyridine)
8,27 (d, J = 8,2 ; 2H ; H-C(4) ; H-C(7) de la phénantroline)

A partir du composé macropolycyclique ainsi obtenu on a préparé le complexe [$Eu^{3+}$C(macropolycycle bis-bipyridinephénanthroline)] en opérant selon le mode opératoire décrit à l'exemple 1.

Les longueurs d'ondes d'excitation et d'émission caractéristiques de ce complexe figurent dans le tableau ci-après.

Exemple 7

En opérant selon le mode opératoire de l'exemple 1 avec les composés macropolycycliques (22) pyridine amide et ($222_B$) on a obtenu respectivement les complexes macropolycycliques ci-après :

[ $Eu^{3+}$ C(22) pyridine amide ]

[ $Tb^{3+}$ C($222_B$) ]

Exemple 8 Préparation du-composé macropolycyclique (22) bis-isoquinoléine de formule (12) : 1,1'-[N,N',N,N'-bis(3,6-dioxaocta méthylène)bis(aminométhyl]-3,3'-biisoquinoléine et du complexe d'europium correspondant

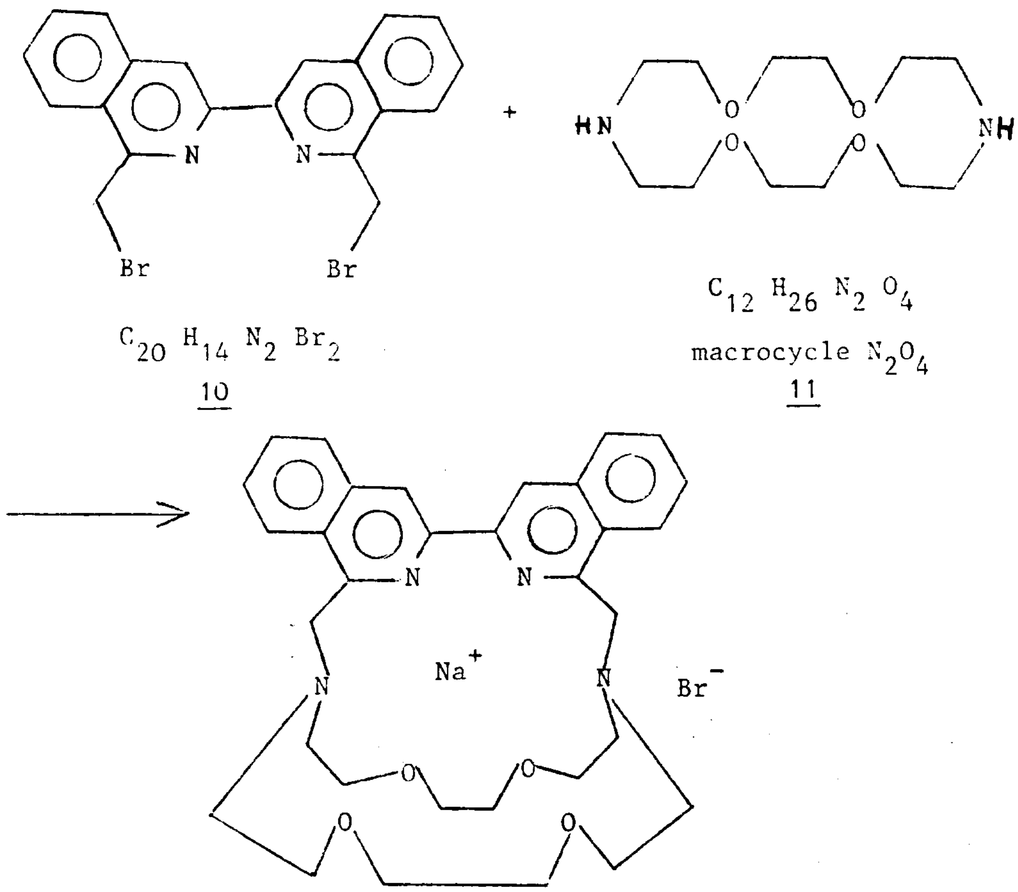

$C_{20}H_{14}N_2Br_2$

10

$C_{12}H_{26}N_2O_4$

macrocycle $N_2O_4$

11

$C_{32}H_{38}N_4O_4NaBr$

12

a) Préparation du 1,1'-bis(bromométhyl)-3,3'-biisoquinoléine de formule 10

Ce composé a été fabriqué à partir du 1-méthyl-3-hydroxyisoquinoléine de formule

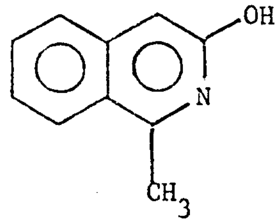

.

Le 1-méthyl-3-hydroxyisoquinoléine a été préparé par cyclisation du N-(±)-phényléthyl-diéthoxyacétamide. 72 g de ce composé de départ, préparé selon le mode opératoire décrit par H. FUKUMI et al. dans "HETEROCYCLES" 9(9) 1197 (1978) et purifié par distillation sous pression réduite (140 °C - 0,1 mm Hg), ont été versés, goutte à goutte et sous agitation, pendant une heure dans 400 ml de $H_2SO_4$ concentré refroidi à 10 °C. Le mélange réactionnel a été refroidi au cours de l'addition pour que la température de celui-ci n'excède pas 10 °C. Le mélange réactionnel a été ensuite agité pendant 10 h à la température ambiante et versé ensuite dans 600 g de glace. Après filtration, la solution claire a été neutralisée avec de l'ammoniaque à 20% sous refroidissement efficace. Le précipité jaune a été filtré, lavé avec de l'eau et séché sous vide. On a obtenu 41,5 g de 1-méthyl-3-hydroxyisoquinoléine (rendement 90%).

La 1-méthyl-3-hydroxyisoquinoléine ainsi obtenue a ensuite été tosylée.

Une suspension de 41,5 g de 1-méthyl-3-hydroxyisoquinoléine a été agitée avec de la pyridine (250 ml) sous refroidissement dans un bain de glace. On a ensuite progressivement ajouté en 30 min du chlorure de p-toluènesulfonyle (70 g, 1,5 équivalent). Le composé de départ jaune a alors disparu. La réaction a été suivie par chromatographie couche mine. Dès que la réaction a été terminée, on a ajouté de l'eau (50 ml) et agité pendant encore une heure. Le mélange a été dilué dans 700 ml d'eau et neutralisé avec du $Na_2CO_3$ solide. Le précipité formé a été filtré, lavé soigneusement avec de l'eau et séché. On a obtenu 70,5 g d'un produit non hygroscopique stable à l'air (rendement 86%).

La 1-méthyl-3-p-toluènesulfonyloxyisoquinoléine ainsi obtenue a été ensuite couplée selon le mode opératoire décrit par M. Tiecco et al. dans SYNTHESIS 736, 1984.

Dans un ballon a trois tubulures, équipé d'un agitateur magnétique, purgé à l'argon, on a introduit 1 l de diméthylformamide (DMF), 236,3 g de triphénylphosphine et 53,5 g de $NiCl_2,6H_2O$ qui ont formé une solution bleu-vert foncé. Lorsque la température du bain d'huile a atteint 50 °C, on a ajouté 14,64 g de poudre de zinc et la couleur de la solution est passée au rouge-brun. Après une heure, la solution du produit de départ (70,5 g dans 200 ml de DMF), à savoir la 1-méthyl-3-p-toluènesulfonyloxyisoquinoléine, a été ajoutée rapidement, goutte à goutte et sous agitation, sous chauffage à 50 °C, lequel a été maintenu pendant 6 h. Après refroidissement jusqu'à la température ambiante, le mélange réactionnel a été versé dans 4 l d'ammoniac dilué (1,5 l de $H_2O$ et 500 ml de $NH_3$ à 20%). Un courant d'air vigoureux a été envoyé dans la suspension pour oxyder le complexe $Ni^\circ(Ph_3P)_4$ (Ph = phényle). La fin de l'oxydation a été indiquée par la disparition de la couleur brune. La suspension a été filtrée, lavée avec de l'eau et placée dans 400 ml de HCl à 20% pour transformer la biisoquinoléine en son chlorhydrate insoluble dans l'eau. La suspension a été agitée avec deux portions de 400 ml d'éther éthylique pour éliminer la triphénylphosphine et la couche acide a été filtrée, lavée avec 100 ml d'eau et de l'acétone pour éliminer les traces de $Ph_3P$ et de $Ph_3PO$. Le chlorhydrate a été placé dans un ballon à fond rond avec 200 ml d'ammoniaque à 20% et agité toute la nuit pour libérer la base. Le produit blanc obtenu (26 g), à savoir la 1,1'-diméthyl-3,3-biisoquinoléine, a été filtré, lavé soigneusement avec de l'eau et séché sous vide (rendement 81%). Ce produit était peu soluble dans la plupart des solvants et légèrement soluble dans $CHCl_3$ et THF.

La 1,1'-diméthyl-3,3'-biisoquinoléine ainsi obtenue a été ensuite bromée pour former le 1,1'-bis-(bromométhyl)-3,3'-biisoquiloléine.

1,20 g de 1,1'-diméthyl-3,3'-biisoquinoléine ont été dissous dans 500 ml de $CCl_4$ au reflux et du N-bromosuccinimide (2,26 g, 3 équivalents) a été ajouté. Après 10 min, 10 g de 2,2'-azobis(2-méthylpropionitrile) ont été ajoutés. La réaction a été suivie par chromatographie couche mince. Deux autres portions d'intitiateur (10 mg chacune) ont été ajoutées en 1 h. Après 3 h, la solution a été évaporée jusqu'à siccité et le résidu a été traité avec 150 ml de méthanol, agité pendant 30 min et filtré. Le solide obtenu a été lavé avec 100 ml de méthanol. Le gâteau de filtration a été lavé sous vide, dissous dans du toluène bouillant (100 ml) et filtré rapidement. Le produit a précipité pendant la nuit au réfrigérateur (1,28 g - rendement 68%).

b) Préparation du composé macropolycyclique de formule 12

A un mélange agité de 1,416 g du macrocycle $N_2O_4$ et 3,39 g de $Na_2CO_3$ dans 250 ml de $CH_3CN$, on a ajouté en 3 h une suspension de 1,1'-bis(bromométhyl)-3,3'-biisoquinoléine dans $CH_3CN$ (100 ml). L'agitation a été maintenue pendant 20 h. Après filtration et lavage avec du $CH_3CN$ et évaporation, on a obtenu un produit brut qui a été chromatographié doux fois, tout d'abord sur alumine (éluants : 3 % $CH_3OH$ dans $CHCl_3$, puis 10% $CH_3OH$ dans $CHCl_2$ v/v) et ensuite sur du gel de silice (éluant : 10 % de $CH_3OH$ dans $CHCl_3$). Le rendement pour les deux purifications a été de 0,289 g, soit 14 %. Une autre purification a été effectuée par cristallisation dans un mélange éthanol-éther par diffusion de vapeur.

c) Préparation du complexe [$Eu^{3+}$ C(22)biisoaquinoléine]

24,5 mg (1 équivalent) de nitrate d'europium anhydre ont été dissous dans 0,5 ml de $CH_3CN$ anhydre. Le complexe de sodium de la (22)biisoquinoléine (37,0 mg) dans 0,3 ml de $CH_3CN$ a été ajouté et le mélange résultant a été traité selon le mode opératoire décrit à l'exemple 2 B. Le précipité jaune pâle obtenu a été dilué dans 3 ml d'acétonitrile et chauffé pendant 2 h. La solution a été laissée à la température ambiante pendant plusieurs jours. On a obtenu un produit cristallisé jaune de formule 12.

Exemple 9 Préparation du composé macropolycyclique (22) diphénylbipyridine de formule 18 : 6,6'-[N,N',N,N'-bis(3,6-dioxaocta méthylène)bis(aminométhyl)]-4,4'-diphényl-2,2'-bipyridine

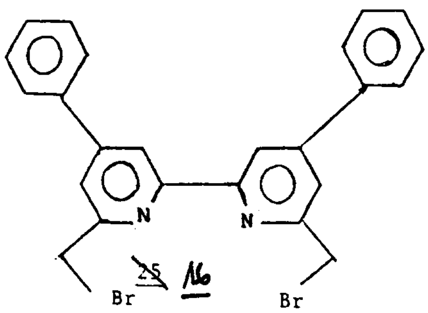

16

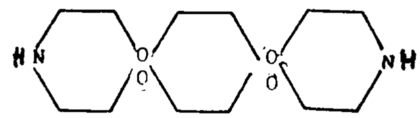

17

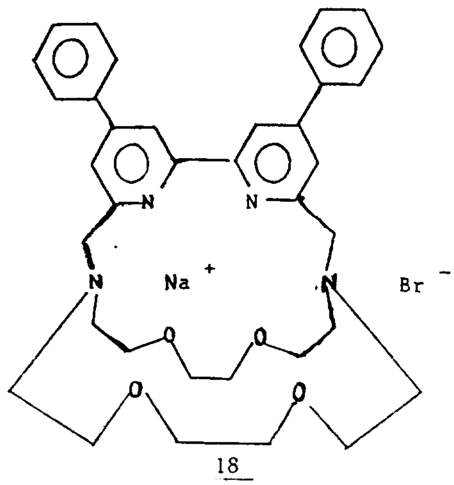

18

a) Préparation de la 6,6'-bis(bromométhyl)-4,4'-diphényl-2,2'-bipyridine (16)

Ce composé a été préparé à partir de la 4,4'-diphényl-2,2'-bipyridine disponible dans le commerce, selon le mode opératoire ci-après :

- 6,6'-diméthyl-4,4'-diphényl-2,2'-bipyridine

A une suspension agitée de 4,4'-diphényl-2,2'-bipyridine (4 g, 13 mmol) dans du tétrahydrofuranne anhydre (THF), refroidie avec un bain de glace, on a ajouté goutte à goutte du méthyllithium 1,5 M (3 équivalents) sous atmosphère d'azote. Après cette addition, la solution a été agitée pendant encore 30 min dans les mêmes conditions. Le mélange rouge foncé obtenu a été ensuite chauffé et agité à 40 ° C pendant 3 h sous azote.

Un excès d'eau a été lentement ajouté à 0 ° C sous azote. La phase organique a été séparée et la phase aqueuse a été extraite trois fois avec du dichlorométhane. On a ensuite ajouté du dioxyde de manganèse (20 à 40 fois le poids du composé de départ) à la solution organique orange. Le mélange a été agité à la température ambiante pendant 30 à 50 min. La réaction a été suivie par chromatographie couche mince ($Al_2O_3$ - éluant : toluène).

On a ensuite ajouté du sulfate de magnésium au mélange réactionnel, qui a été agité pendant 30 min supplémentaires. On a ensuite filtré et évaporé le filtrat. Le résidu a été chromatographié sur alumine

(éluant : toluène/hexane 1:1) et on a obtenu deux produits :
la 6,6'-diméthyl-4,4'-diphényl-2,2'-bipyridine (0,85 g ; 19 %)
la 6-monométhyl-4,4'-diphényl-2,2'-bipyridine (0,45 g ; 10,8 %)

- 6,6'-diméthyl-4,4'-diphényl-2,2'-bipyridine-N,N'-dioxyde

Dans un ballon de 250 ml contenant une solution du composé ci-dessus (0,28 g, 0,83 mmol) dans le chloroforme (60 ml) agitée et refroidie au bain de glace on a ajouté graduellement une solution d'acide m-chloroperbenzoïque (0,57 g - 3,3 mmol) dans le chloroforme (60 ml). L'agitation a été poursuivie pendant 3 h, pendant laquelle on a laissé le mélange revenir à la température ambiante.

Le mélange réactionnel a été traité avec une solution aqueuse de bicarbonate de sodium (3,3 mmol) agitée pendant 30 min.

La phase organique a été séparée et évaporée sous vide. Le résidu a été redissous dans le dichlorométhane ($CH_2Cl_2$) et passé sur une colonne d'alumine basique. Une seconde chromatographie a été effectuée sur de l'alumine standard pour purifier complètement le produit (0,21 g ; 68 %).

- 6,6'-bis(acétoxyméthyl)-4,4'-diphényl-2,2'-bipyridine

On a chauffé pendant une heure au reflux 0,21 g (0,57 mmol) du composé ci-dessus dans de l'anhydride acétique (1,3ml). La solution a été concentrée sous vide et du toluène a été ajouté jusqu'à la formation de l'azéotrope du mélange.

Le solide résultant a été redissous dans du dichlorométhane, lavé avec une solution aqueuse à 10 % de bicarbonate de sodium, séché et le solvant a été évaporé. Le produit brut a été passé sur une colonne de gel de silice et élué avec du dichlorométhane pour donner 0,126 g (49 % rendement) du composé attendu.

- 6,6'-bis(bromométhyl)-4,4'-diphényl-2,2'-bipyridine 16

Une solution agitée du composé ci-dessus (0,053g) ; 0,117 mmol) et d'acide bromhydrique à 47 % (0,9 ml) a été chauffée pendant 4 h à 130 ° C.

La solution a été ensuite refroidie dans un bain de méthanol/glace. On y a ajouté 15 ml d'eau, 40 ml de chloroforme, puis, graduellement, une solution saturée de carbonate de sodium jusqu'à ce que le pH de la solution devienne alcalin.

La phase organique a été séparée et la phase aqueuse a été extraite avec du chloroforme (2 fois 10 ml). Toutes les fractions organiques ont été combinées et évaporées. Le résidu a été chromatographié sur une colonne d'alumine avec le chloroforme comme éluant. On a obtenu 40 mg (rendement 69 %) du dibromure de formule 16.

b) Préparation du sel [$Na^+$ C(22)diphénylbipyridine Br] de formule 18

On a opéré selon le mode opératoire décrit à l'exemple 5 B en utilisant :
21,2 mg (0,081 mmol) du macrocycle $N_2O_4$ de formule 17
43 mg (0,40 mmol) de carbonate de sodium
40 mg (0,081 mmol) du dibromure de formule 16

On a obtenu 29,8 mg (rendement 53 %) du complexe de formule 18.

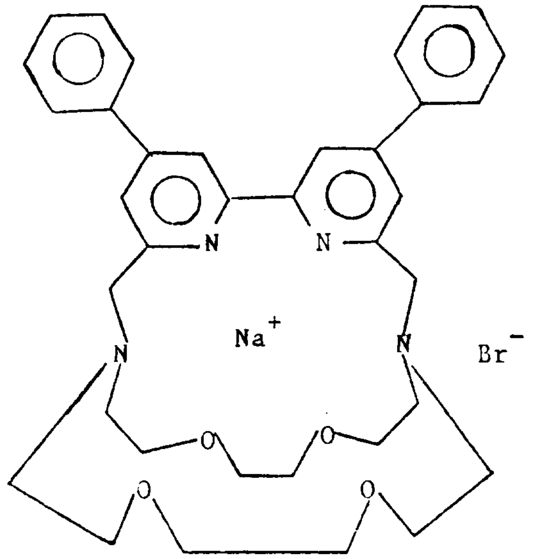

18

Exemple 10 Préparation du complexe macropolycycique diphénylbipyridine- bis-bipyridine de formule 19 : 6,6'-[N,N',N,N'-[bis (2,2'-bipyridine-6,6'-diméthyl)]bis(aminométhyl)]-4,4'-diphényl-2,2'-bipyridine

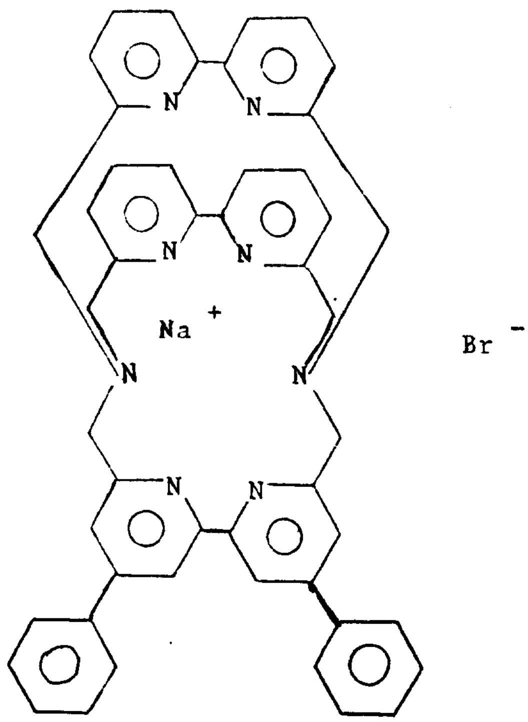

19

On a répété le mode opératoire de l'exemple ci-dessus en utilisant le macrocycle bis-pyridine de formule (5) (exemple 5) à la place du macrocycle $N_2O_4$ de formule 17.

On a utilisé :
24,7 mg (0,063 mmol) du macrocycle bis-pyridine 5
31,1 mg (0,063 mmol) du bibromure de formule 16
0,1 mg (0,94 mmol) de carbonate de sodium.

La réaction a duré 23 h et on a obtenu le composé de formule 19 avec un rendement de 20 %.

Exemple 11 Préparation du composé macropolycyclique isoquinoléine bis- bipyridine de formule 20 : 1,1'-[N,N',N,N'-[bis(2,2'-bipyridine 6,6'-diméthyl)]bis(aminométhyl)]-3,3'-biisoquinoléine

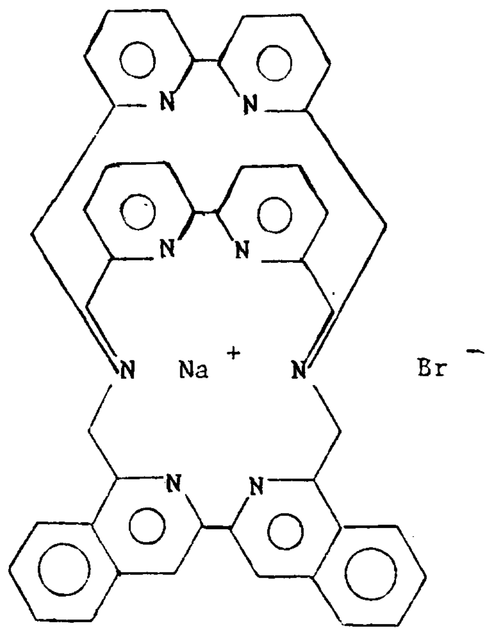

20

On a répété le mode opératoire de l'exemple 8 en utilisant le macrocycle bis-bipyridine de formule 5 à la place du macrocycle $N_2O_4$ de formule 11. On a utilisé des quantité équimoléculaires du composé de formule 5 et du dibromure de formule 10, et on a obtenu le composé de formule 20.

Exemple 12 - Préparation du composé macropolycyclique (22)bipyridine

On a opéré selon le mode opératoire de l'exemple 5 en utilisant le macrocycle $N_2O_4$ de formule 11 à la place du macrocycle bis-bipyridine de formule 5 et on a obtenu le composé macropolycyclique de formule 21 :

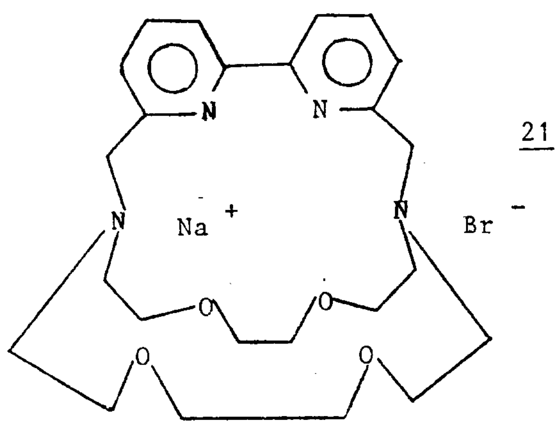

21

Exemple 13

En opérant selon le mode opératoire de l'exemple 1 avec les composés macropolycycliques obtenus selon les exemples 9 à 12, on a obtenu respectivement les complexes macropolycycliques ci-après :

[$Eu^{3+}$ C(22)bipyridine] : complexe $Eu^{3+}$ de 6,6'-[N,N',N,N'-bis (3,6-dioxaoctaméthylène)-bis(aminométhyl)]-2,2'-bipyridine

[$Eu^{3+}$ C(bpy.bpy.biisoq.)] : complexe $Eu^{3+}$ de 1,1'-[N,N',N,N'-(bis 2,2'-bipyridine-6,6'-diméthyl)bis-

(aminométhyl)]3,3'-biisoquinoléine

[$Eu^{3+}$ C(22)diph.bpy] : complexe $Eu^{3+}$ de 6,6'-[N,N',N,N'-bis (3,6-dioxaoctaméthylène)bis(aminométhyl)]-4,4'-diphényl-2,2'-bipyridine

[$Tb^{3+}$ C(22)bipyridine] : complexe $Tb^{3+}$ de 6,6-[N,N',N,N'-bis (3,6-dioxaoctaméthyléne)-bis(aminométhyl)]-2,2'-bipyridine

De même, en opérant selon le mode opératoire de l'exemple 5 on a préparé le complexe [$Eu^{3+}$C (macropolycycle trisphénanthroline)] (complexe de $Eu^{3+}$ de 2,2',2'',9,9',9''-bis [nitrilotri(méthylène)]tris(1,10-phénanthroline)

en utilisant la 6,6'-bis-bromométhyl-phénanthroline et le macrocycle bis(phénanthrolinediyl)diamine. Le composé macropolycyclique correspondant avait les caractéristiques ci-après :

| microanalyse : $C_{42}H_{38}N_8$, NaBr, $H_2O$ (767,6) | | | |
|---|---|---|---|
| Calculé : | C 65,71 | H 4,17 | N 14,6 |
| Trouvé : | C 65,23 | H 4,26 | N 13,10 |

RMN $^1H$ : solvant $CDCl_3$

4,03 4,45 (très large ; AB, 12H, $CH_2$)

7,66 (d, J = 8,1 ; 6H ; H-C(3) ; H-C(8))

7,78 (s, 6H, H-C(5); H-C(6))

8,27 (d, J = 8,1 ; 6H ; H-C(4) ; H-C(7))

Caractéristiques fluorescentes des cryptates de l'invention.

a) pics d'excitation pour une longueur d'onde d'émission donnée

Pour chaque complexe, on a déterminé, pour une longueur d'onde d'émission donnée, caractéristique de l'ion de terre rare du complexe, les longueurs d'ondes d'excitation ; les pics d'excitation enregistrés ne correspondent pas à ceux de l'ion de terre rare seul. Par ailleurs, on a constaté qu'en excitant le complexe à la longueur d'onde correspondant au pic d'excitation déterminé ci-dessus, le maximum de fluorescence était caractéristique de l'ion de terre rare.

Les déterminations ci-dessus ont été effectuées avec un spectromètre PERKIN-ELMER LS 5 dans les conditions opératoires indiquées dans le tableau I ci-après.

b) mesure de la durée de vie $\tau$ des cryptates d'europium et de terbium

Sur un spectromètre LS5, on a enregistré le spectre d'émission du pic situé entre 610 et 620 nm pour l'europium et entre 540 et 550 pour le terbium, la solution étant excitée dans l'un des pics d'absorption. On a opéré en mode "phosphorescence", la valeur de la fenêtre a été fixée à 1 ms = tg. Les enregistrements ont été réalisés pour plusieurs valeurs de $t_d$ (délai), soit 0,1 ; 0,2 ; 0,3 ; 0,4 et 0,5 ms. On a mesuré l'intensité du pic $I_t$ et déterminé $\tau$ par la formule :

$$I_t = I_o\, e^{\frac{-t}{\tau}}$$

$$\tau = \frac{0,434\, t}{\log I_o - \log I_t}$$

En traçant la courbe log $I_t$ en fonction de td, on peut déterminer $I_o$ et calculer $\tau$. Les résultats obtenus figurent dans le tableau II ci-après.

TABLEAU I

CARACTERISTIQUES D'EXCITATION ET D'EMISSION DES COMPLEXES DE L'INVENTION

(Perkin Elmer LS 5)

| Complexe | Concentration (solvant) | largeur des fentes | facteur d'expansion | EXCITATION | | EMISSION | |
|---|---|---|---|---|---|---|---|
| | | | | λ d'émission donnée | λ d'excitation enregistrée | λ d'excitation donnée | maximum de fluorescence observé à : |
| [$Eu^{3+}$C(22)phèn] | 3,7.$10^{-6}$ mol/l (eau) | 2,5/20 nm | 10 | 615 nm | 290 nm (pic)<br>312 nm (épaulement)<br>330 nm ( " ) | 290 nm | 615 nm (pic)<br>593 (second pic) |
| [$Eu^{3+}$C(22)phèn amide] | 2,5.$10^{-4}$ mol/l (eau) | 2,5/10 nm | 3 | 613 nm | 290 nm (pic)<br>323 nm (pic)<br>333 nm (épaulement)<br>345 nm (épaulement) | 290 nm | 613 nm<br>590 nm |
| [$Eu^{3+}$C(macropolycycle tris-bipyridine)] | 7.$10^{-7}$ mol/l (tampon phosphate 0,1 M pH 7,5) | 2,5/10 nm | 15 | 617 nm | maximum à 300 nm | 300 nm | maximum à 617 nm |
| [$Eu^{3+}$C(phénanthroline bis-bipyridine] | 2,8.$10^{-5}$ mol/l (eau) | 2,5/10 nm | 2 | | | 280 nm | 590 nm<br>613 nm |

TABLEAU I (suite)

CARACTERISTIQUES D'EXCITATION ET D'EMISSION DES COMPLEXES DE L'INVENTION

(Perkin Elmer LS 5)

| Complexe | Concentration (solvant) | largeur des fentes | facteur d'expansion | EXCITATION | | EMISSION | |
|---|---|---|---|---|---|---|---|
| | | | | λ d'émission donnée | λ d'excitation enregistrée | λ d'excitation donnée | maximum de fluorescence observé à : |
| [$Eu^{3+}$C(22)bipyridine] | $10^{-5}$ M (eau) | 2,5/10 nm | 10 | 617 nm | { 237 nm (pic)<br>303 nm (pic) | 310 nm | { 617 nm<br>592 nm |
| [$Eu^{3+}$C(22)bi-isoquinoléine] | $10^{-5}$ M tampon tris 0,01 M pH 7 | 2,5/10 nm | 0,5 | 617 | 355 nm (pic)<br>325 nm (pic) | 325 nm | 617 nm<br>592 nm |
| [$Eu^{3+}$C(22)biphénylbipyridine] | $10^{-5}$ M $H_2O$ | 2,5/10 nm | 5 | 617 nm | 254 (pic)<br>323 (pic) | 320 nm | 617 nm<br>592 nm |
| [$Eu^{3+}$C(bis-bipyridine bi-isoquinoléine)] | 0,06 mg/ 2,4 ml d'eau | 2,5/10 nm | 20 | 617 nm | 310 (pic) | 310 nm | 617 nm<br>592 nm |

TABLEAU I (suite)

CARACTERISTIQUES D'EXCITATION ET D'EMISSION DES COMPLEXES DE L'INVENTION

(Perkin Elmer LS 5)

| Complexe | Concentration | largeur des fentes | facteur d'expansion | EXCITATION | | EMISSION | |
|---|---|---|---|---|---|---|---|
| | | | | λ d'émission donnée | λ d'excitation enregistrée | λ d'excitation donnée | maximum de fluorescence observé à : |
| [$Eu^{3+}$C(22) anthracène] | 0,2 mg dans 3 ml d'eau | 2,5/10 nm | 0,2 | 613 nm | 287 nm (pic)<br>317 nm (épaulement) | 287 nm | 590 nm (pic)<br>613 nm (pic) |
| [$Eu^{3+}$C(22) pyridine amide]<br>* | $2.10^{-4}$ mol/1 (DMSO) | 2,5/5 nm | 40 | - | - | 290 nm | 613 nm<br>590 nm |
| [$Tb^{3+}$ C 222 $_B$]<br>** | $10^{-3}$ mol/1 (DMSO) | 2,5/20 nm | 15 | 541 nm | 290 nm (épaulement)<br>333 nm (pic) | 290 nm | 541 nm<br>485 nm |
| [$Eu^{3+}$C(tris-phénanthroline)] | 0,3 mg/3 ml de tampon tris | 2,5/10 nm | 5 | 617 nm | 272 (pic) | 270 nm | 617 nm<br>593 nm |
| [$Tb^{3+}$C(22)bipyridine] | 0,1 mg/2,4 ml $H_2O$ | 2,5/10 nm | 1 | 617 nm | 240 (pic)<br>305 (pic)<br>314 (épaulement) | 320 nm | 545 nm<br>492 nm<br>586 nm |

* mesure faite en mode phosphorescence ; délai td = 0,1 milliseconde
tg = 2 millisecondes

** mesure faite en mode phosphorescence ; délai td = 0,2 milliseconde
tg = 5 millisecondes

TABLEAU II

| Complexe macropolycyclique | λ excitation nm | milieu | concentration | τ ms |
|---|---|---|---|---|
| [$Eu^{3+}$C(22)phèn] | 280 | $H_2O$ | $3,7.10^{-4}$ M | 0,27 |
| [$Eu^{3+}$C(22) phèn amide] | 280 | $H_2O$ | $2,5.10^{-6}$ M | 0,21 |
| [$Eu^{3+}$C(22)bipyridine] | 310 | $H_2O$ | $1,2.10^{-5}$ M | 0,41 |
| [$Eu^{3+}$C(22)bi-isoquinoléine] | 325 | tris 0,01 M pH 7 | $10^{-5}$ M | 0,135 |
| [$Eu^{3+}$C(22)biphénylbipyridine] | 320 | $H_2O$ | $10^{-5}$ M | 0,32 |
| [$Eu^{3+}$C(tris-bipyridine)] | 300 | tampon phosphate 0,01 M pH 7,4 | $2.10^{-7}$ M | 0,43 |
| [$Eu^{3+}$C(bis-bipyridine-bi-isoquinoléine)] | 311 | $H_2O$ | $10^{-5}$ M | 0,25 |
| [$Eu^{3+}$C(tris-phénanthroline)] | 272 | tris 0,01 M pH 7 | $10^{-4}$ M | 0,32 |
| [$Tb^{3+}$C(22)bipyridine] | 318 | " | $4.10^{-5}$ M | 0,72 |

**Revendications**

**Revendications pour les Etats contractants suivants : BE, CH, DE, FR, GB, IT, LI, LU, NL, SE**

**1.** Application, à titre de marqueurs de produits biologiques dans les méthodes de détection et de détermination immunologiques par fluorescence, des complexes macropolycycliques de terres rares

constitués d'au moins un sel de terre rare complexé par un composé macropolycyclique de formule générale :

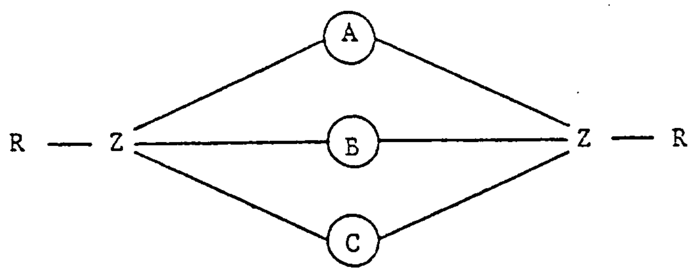

I

dans laquelle Z est un atome choisi parmi l'azote, le carbone ou le phosphore, R est rien ou représente l'hydrogène, le groupe hydroxy, un groupe amino ou un radical hydrocarboné, les radicaux bivalents Ⓐ , Ⓑ et Ⓒ sont indépendamment l'un de l'autre des chaînes hydrocarbonées contenant au moins deux atomes de carbone et éventuellement un ou plusieurs hétéroatomes choisis parmi l'oxygène ou le soufre et étant éventuellement interrompues par un hétéromacrocycle, au moins l'un des radicaux Ⓐ , Ⓑ ou Ⓒ comportant de plus au moins un motif moléculaire ou étant essentiellement constitué par un motif moléculaire, ledit motif moléculaire possédant une énergie de triplet supérieure à celle du niveau émissif de l'ion de terre rare complexé.

**2.** Application selon la revendication 1, caractérisée en ce que lesdits complexes de terres rares sont substitués par un radical choisi parmi les radicaux alkylamino, arylamino, isothiocyano, cyano, isocyano, thiocyano, carboxyle, hydroxyle, mercapto, phénol, imidazole, aldéhyde, époxyde, halogénure : de thionyle, de sulfonyle, de nitrobenzoyle, de carbonyle ; triazo, succinimido, anhydride, halogénoacétate, hydrazino, dihalogénotriazinyle.

**3.** A titre de produits nouveaux, les complexes macropolycycliques de terres rares constitués d'au moins un sel de terre rare complexé par un composé macropolycyclique de formule :

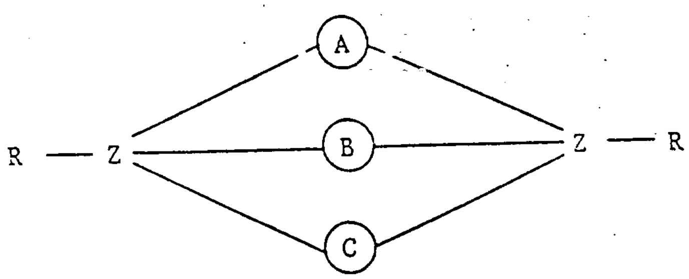

I

dans laquelle Z est un atome choisi parmi l'azote, le carbone ou le phosphore, R est rien ou représente l'hydrogène, le groupe hydroxy, un groupe amino ou un radical hydrocarboné, les radicaux bivalents Ⓐ , Ⓑ et Ⓒ , sont indépendamment l'un de l'autre des chaînes hydrocarbonées contenant au moins deux atomes de carbone et éventuellement un ou plusieurs hétéroatomes choisis parmi l'oxygène et le soufre et éventuellement interrompues par un hétéromacrocycle, au moins l'un des radicaux Ⓐ , Ⓑ ou Ⓒ comportant de plus au moins un motif moléculaire ou étant essentiellement constitué par un motif moléculaire, ledit motif moléculaire possédant une énergie de triplet supérieure à celle du niveau émissif de l'ion de terre rare complexé, à la condition que, lorsque le sel de terre rare est un sel d'europium ou de terbium, Z est l'azote, Ⓐ est $-(CH_2)_2-O-C_6H_3R_1-O-(CH_2)_2-$avec $R_1 =$ H ou $NH_2$, Ⓑ et Ⓒ ne sont pas simultanément l'un le radical $-(CH_2)_2-O-(CH_2)_2-O-(CH_2)_2-$ et l'autre le radical $-(CH_2)_2-O-(CH_2)_2-$, et que lorsque le sel de terre rare est un sel de lanthane ou de praseodyme, Z est l'azote, Ⓐ est $-(CH_2)_2-O-C_6H_3NH_2-O-(CH_2)_2-$ Ⓑ et Ⓒ ne sont pas simultanément $-(CH_2)_2-O-(CH_2)_2-$ et $-(CH_2)_2-O-(CH_2)_2-O-(CH_2)_2$.

**4.** Complexes selon la revendication 3, caractérisés en ce que les chaînes hydrocarbonées sont des chaînes oxyéthylénées ou polyoxyéthylénées.

**5.** Complexes macropolycycliques selon l'une des revendications 3 ou 4, caractérisés en ce que l'ion de terre rare est choisi parmi l'europium, le terbium, le samarium et le dysprosium.

**6.** Complexes macropolycycliques selon l'une quelconque des revendications 3 à 5, caractérisés en ce que le motif donneur d'énergie a une longueur d'onde de phosphorescence inférieure à 580 nm.

**7.** Complexes macropolycycliques selon l'une quelconque des revendications 3 à 6, caractérisés en ce que le motif moléculaire est choisi parmi la phénanthroline, l'anthracène, le benzène, le naphtalène, les bi- et ter-phényle, l'azobenzène, l'azopyridine, la pyridine, les bipyridine, les bi-quinoléines notamment les bi-isoquinoléines et les composés de formules ci-après :
$- C_2H_4 - X_1 - C_6H_4 - X_2 - C_2H_4 -$
$- C_2H_4 - X_1 CH_2 - C_6H_4 - CH_2 - X_2 - C_2H_4 -$ dans lesquelles,
$X_1$ et $X_2$ pouvant être identiques ou différents désignent l'oxygène ou le soufre ;

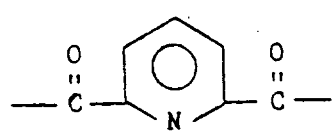

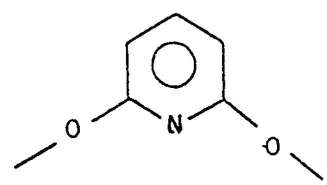

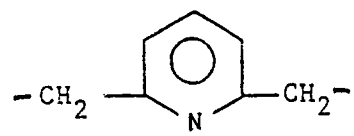

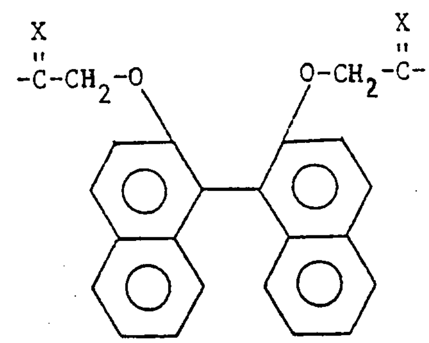

X étant l'oxygène ou l'hydrogène.

**8.** Complexe macropolycyclique selon l'une quelconque des revendications 3 à 7, caractérisé en ce qu'il est constitué de l'ion terbium ou europium complexé par l'un des composés macrocycliques ci-après : 2,9-[N,N',N,N'-bis(3,6-dioxaoctamèthylène) bis(aminométhyl)]-1,10-phénanthroline ; 2,9-[N,N',N,N'-bis-(3,6-dioxaoctaméthylène) bis(aminocarbonyl)]-1,10-phénanthroline ; 9,10-[N,N',N,N'-bis(3,6-dioxaoctaméthylène) bis (aminoéthyl)] anthracène ; 9,10-[N,N',N,N'-bis(3,6-dioxaoctaméthylène) bis-(carbamoylméthyl)]anthracène ; 1,1'-[N,N',N,N'-bis(3,6-dioxaoctaméthylène)bis(aminométhyl)]-3,3'-biisoquinoléine ; 6,6'-[N,N',N,N'-bis(3,6-dioxaoctaméthylène)bis(aminomethyl)]-4,4'-diphényl-2,2'-bipyridine ; 6,6'-[N,N',N,N'-bis(3,6-dioxaoctaméthylène)- bis(aminométhyl)]-2,2'-bipyridine ; 6,6'-[N,N',N,N'-bis(3,6-dioxaoctaméthyléne)- bis(aminocarbonyl)]-2,2'-bipyridine ; 6,6',6'',6''',6'''',6'''''-bis[nitrilotri(méthylène)] tris-2,2'-bipyridine ; 2,2',2'',9,9',9''-bis[nitrilotri(méthylène)] tris(1,10-phénanthroline) ; 2,9-[N,N',N,N'-bis-(2,2'-bipyridine-6,6'-diméthyl)bis(aminométhyl)] -1,10-phénanthroline ; 1,1'-[N,N',N,N'-bis(2,2'-bipyridine-6,6'diméthyl) bis(aminométhyl)]-3,3'-biisoquinoléine ; 6,6'-[N,N',N,N'-bis(2,2'-bipyridine-6,6'-diméthyl) bis(aminométhyl)]-4,4'-diphényl-2,2'-bipyridine.

**9.** Complexes macropolycycliques selon la revendication 3, caractérisés en ce qu'ils répondent à la formule (I) dans laquelle Z = N.

10. Complexes macropolycycliques de terres rares constitués d'au moins un sel de terre rare complexé par un composé macropolycyclique, caractérisé en ce que ledit composé macropolycyclique est choisi parmi les composés ci-après :

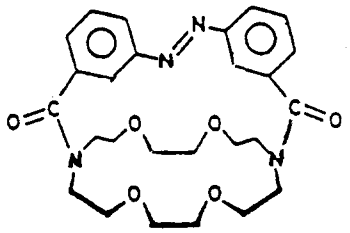

- (22) pyridine amide

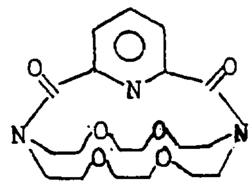

- (22) pyridine amine

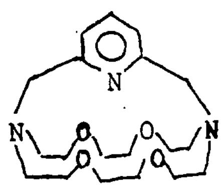

- (22) bipyridine

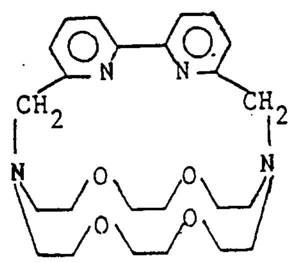

- (22) bipyridine amide

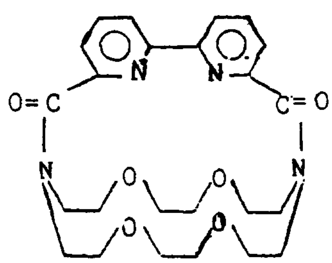

- (22) azopyridine

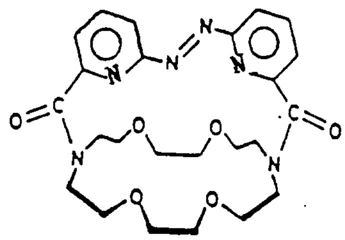

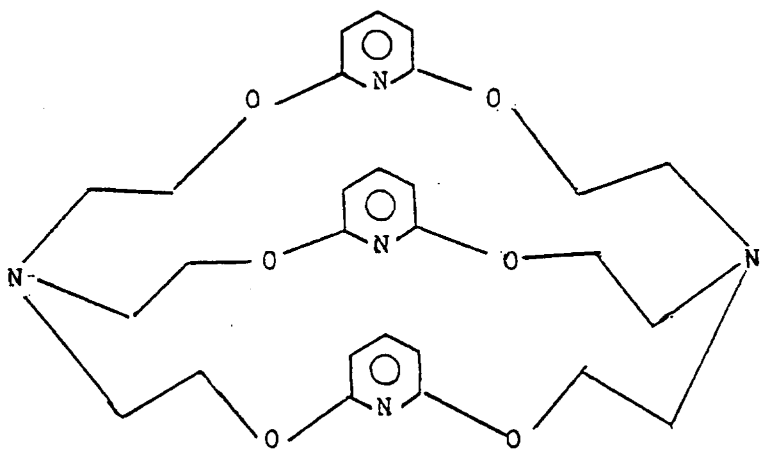

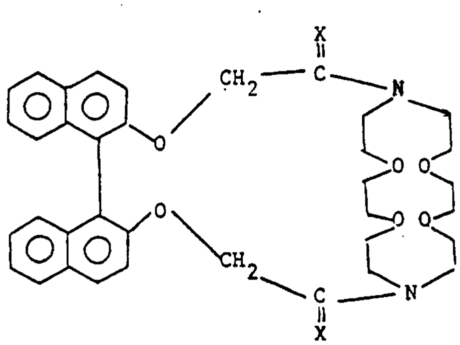

X = O, H

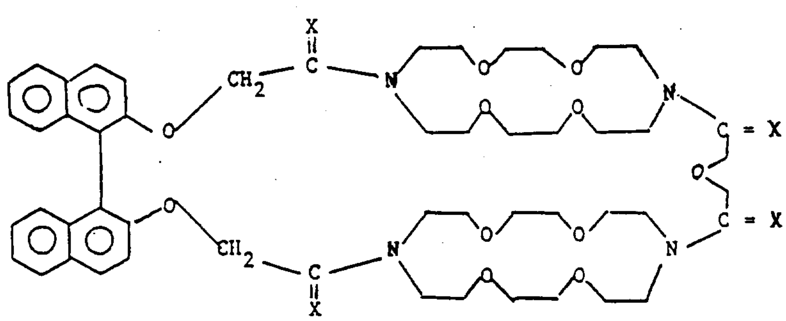

X = O, H

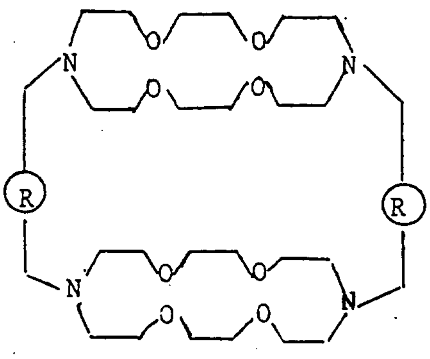

R =

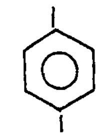

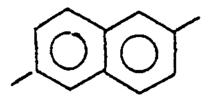

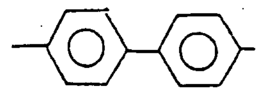

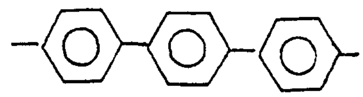

**11.** Complexes macropolycycliques de terres rares constitués d'au moins un sel de terre rare complexé par un composé macropolycyclique de formule I selon la revendication 3, caractérisés en ce que Z = N, Ⓐ et Ⓑ sont deux chaînes mono ou polyoxygénées et en ce que Ⓒ répond à l'une des formules ci-après :

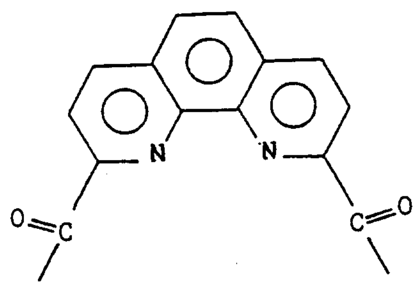

;

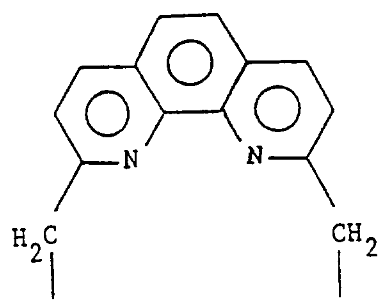

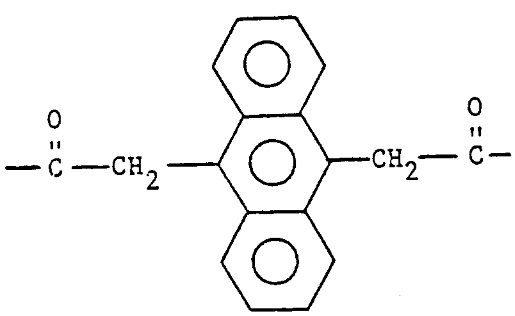

;

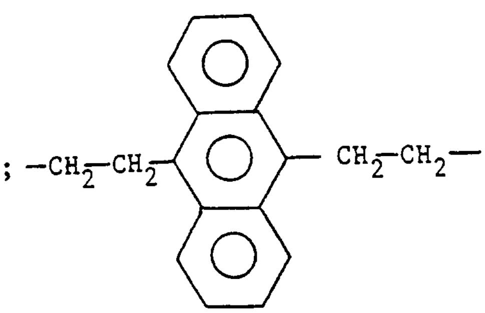

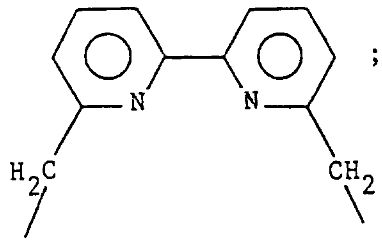 ; 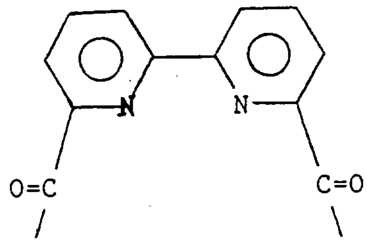 ;

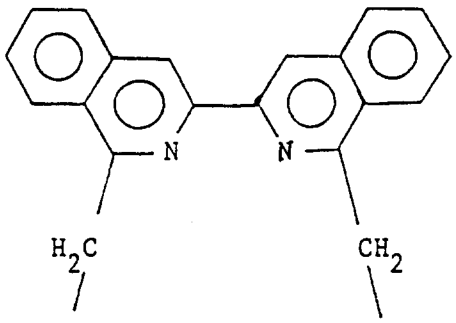 ou 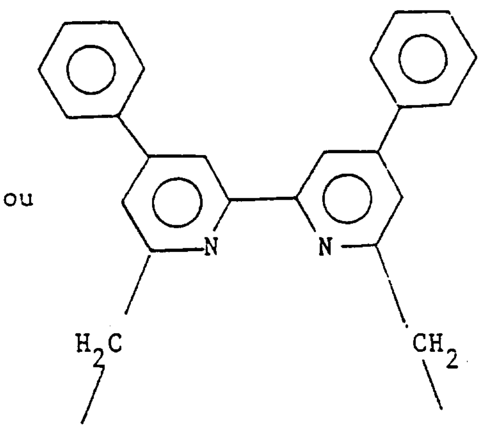

**12.** Complexes macropolycycliques de terres rares constitués d'au moins un sel de terre rare complexé par un composé macropolycyclique de formule I selon la revendication 3, caractérisés en ce que Ⓐ et Ⓑ représentent chacun le groupe

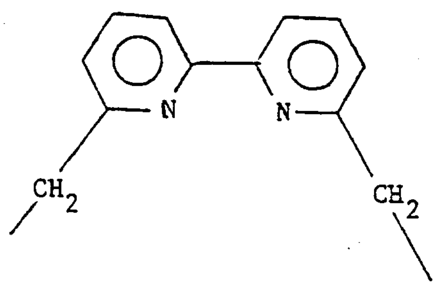

et Ⓒ est l'un des groupes suivants :

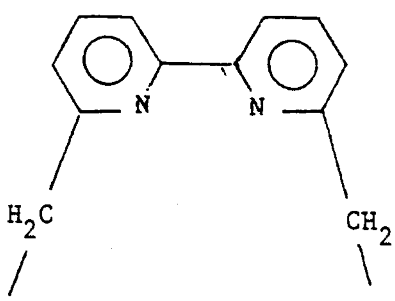 ; 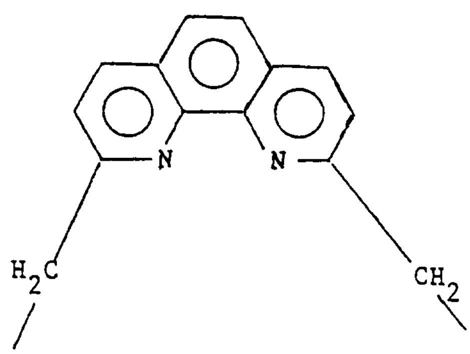 ;

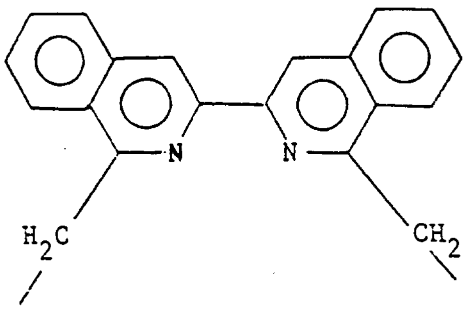

ou

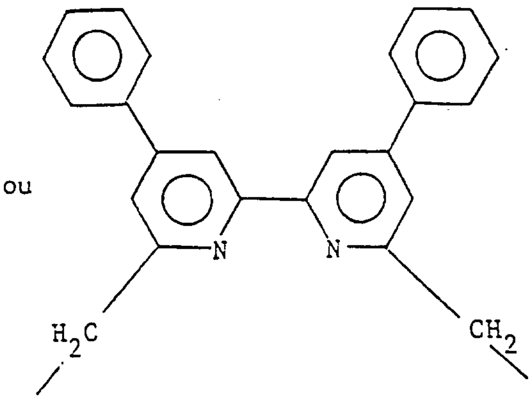

**13.** Complexes macropolycycliques de terres rares constitués d'au moins un sel de terre rare complexé par un composé macropolycyclique de formule I selon la revendication 3, caractérisé en ce que Z = N ; Ⓐ , Ⓑ et Ⓒ sont identiques et représentent l'un des hétérocycles ci-après :

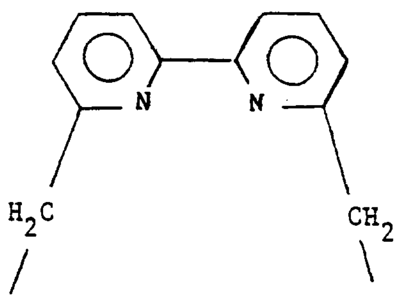

;

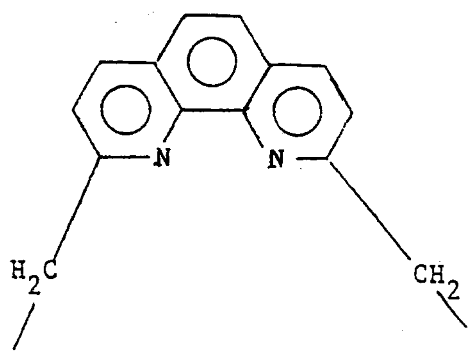

;

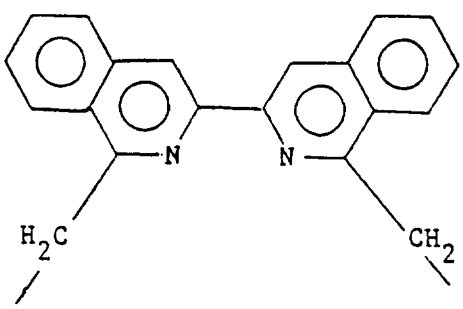

ou

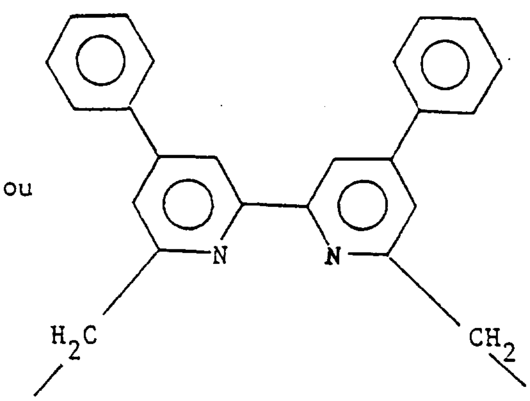

**14.** Composé macropolycyclique répondant à la formule :

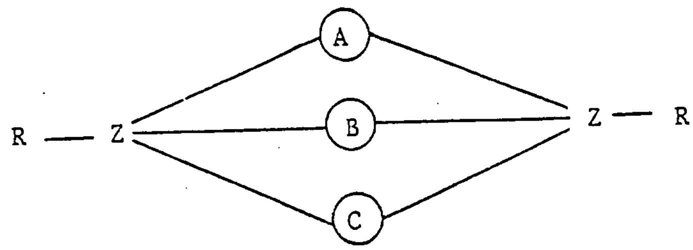

I

dans laquelle Z est un atome choisi parmi l'azote, le carbone ou le phosphore, R est rien ou représente l'hydrogène, le groupe hydroxy, un groupe amino ou un radical hydrocarboné, les radicaux bivalents Ⓐ , Ⓑ et Ⓒ sont indépendamment l'un de l'autre des chaînes hydrocarbonées contenant au moins deux atomes de carbone et éventuellement un ou plusieurs hétéroatomes choisis parmi l'oxygène ou le soufre et étant éventuellement interrompues par un hétéromacrocycle, au moins l'un des radicaux Ⓐ , Ⓑ ou Ⓒ comportant de plus au moins un motif moléculaire ou étant essentiellement constitué par un motif moléculaire, ledit motif moléculaire possédant une énergie de triplet supérieure à celle du niveau émissif de l'ion de terre rare complexé et étant choisi parmi la phénanthroline, l'anthracène, les bipyridines et les bi-quinoléines notamment les bi-isoquinoléines, à la condition que, lorsque Z est l'azote et Ⓐ répond à l'une des formules ci-après :

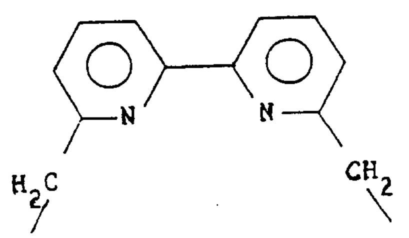

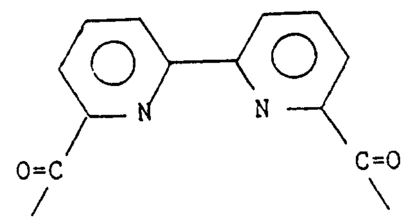

Ⓑ et Ⓒ ne sont pas simultanément $-(CH_2)_2-O-(CH_2)_2-O-(CH_2)_2-$.

**15.** Composé selon la revendication 14, caractérisé en ce que Z est l'azote et Ⓐ et Ⓑ sont des chaînes polyoxyéthylénés.

**16.** Composé selon l'une des revendications 14 ou 15, caractérisé en ce qu'il est l'un des composés ci-après :
2,9-[N,N',N,N'-bis(3,6-dioxaoctaméthylène) bis(aminométhyl)]-1,10-phénanthroline
2,9-[N,N',N,N'-bis(3,6-dioxaoctaméthylène) bis(aminocarbonyl)]-1,10-phénanthroline
9,10-[N,N',N,N'-bis(3,6-dioxaoctaméthylène) bis (aminoéthyl)] anthracène
9,10-[N,N',N,N'-bis(3,6-dioxaoctaméthylène)bis(carbamoylméthyl)] anthracène
1,1'-[N,N',N,N'-bis-(3,6-dioxaoctaméthylène)bis(aminométhyl)]-3,3'-bi-isoquinoléine
6,6'[N,N',N,N'-bis(3,6-dioxaoctaméthylène)bis(aminométhyl)] -4,4'-diphényl-2,2'-bipyridine

**17.** Composé selon la revendication 14, caractérisé en ce que ledit composé est tel que défini dans l'une quelconque des revendications 9 à 13.

**18.** Composé selon la revendication 14, caractérisé en ce qu'il est 6,6',6'',6''',6'''',6'''''-bis[nitrilotri-(méthyléne)]tris-2,2'-bipyridine ; 2,9-[N,N',N,N'-bis(2,2'-pyridine-6,6'-diméthyl)bis(aminométhyl)]-1,10-phénanthroline ; 1,1'-[N,N',N,N'-bis(2,2'-bipyridine-6,6'-diméthyl) bis(aminométhyl)]-3,3'-biisoquinoléine ; 2,2',2'',9,9',9''-bis[nitrilotri(méthylène)]tris(1,10-phénanthroline); 6,6'-[N,N',N,N'- bis(2,2'-bipyridine-6,6'-diméthyl) bis(aminométhyl)]-4,4'-diphényl-2,2'-bipyridine.

**19.** Complexe biologique, caractérisé en ce qu'il est constitué d'une molécule biologiquement active associée par couplage avec, ou adsorption sur, un complexe macropolycyclique de terre rare tel que

défini dans les revendications 1, 3 à 13.

**20.** Complexe biologique selon la revendication 19, caractérisé en ce que la molécule biologiquement active est choisie parmi les antigènes, les anticorps, les anticorps monoclonaux, les fragments et combinaisons Ac-fragments, les drogues, les récepteurs, les hormones, les récepteurs hormonaux, les bactéries, les stéroïdes, les aminoacides, les peptides, les virus, les vitamines, les nucléotides ou polynucléotides, les enzymes, leurs substrats, les lectines, les acides nucléiques, l'ADN et l'ARN.

**21.** Procédé d'exaltation de la fluorescence d'ions de terres rares, caractérisé en ce qu'il consiste à complexer au moins un ion de terre rare avec un composé macropolycyclique tel que défini dans les revendications 1, 3 à 13, et à exciter le motif moléculaire dudit composé.

**Revendications pour l'Etat contractant suivant : AT**

**1.** Procédé d'exaltation de la fluorescence d'un ion de terre rare, caractérisé en ce qu'il consiste à complexer un ion de terre rare avec un composé macropolycyclique de formule générale I ci-après ou un de ses sels :

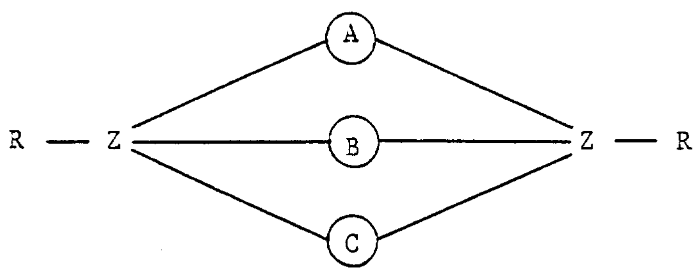

I

dans laquelle Z est un atome choisi parmi l'azote, le carbone ou le phosphore, R est rien ou représente l'hydrogène, le groupe hydroxy, un groupe amino ou un radical hydrocarboné, les radicaux bivalents Ⓐ , Ⓑ et Ⓒ sont indépendamment l'un de l'autre des chaînes hydrocarbonées contenant au moins deux atomes de carbone et éventuellement un ou plusieurs hétéroatomes choisis parmi l'oxygène ou le soufre et étant éventuellement interrompues par un hétéromacrocycle, au moins l'un des radicaux Ⓐ , Ⓑ ou Ⓒ comportant de plus au moins un motif moléculaire ou étant essentiellement constitué par un motif moléculaire, ledit motif moléculaire possédant une énergie de triplet supérieure à celle du niveau émissif de l'ion de terre rare complexé, et à exciter le motif moléculaire dudit composé macropolycyclique.

**2.** Procédé d'obtention de complexes macropolycycliques de terres rares, caractérisé en ce qu'il consiste à faire réagir au moins un sel de terre rare avec un composé macropolycyclique de formule :

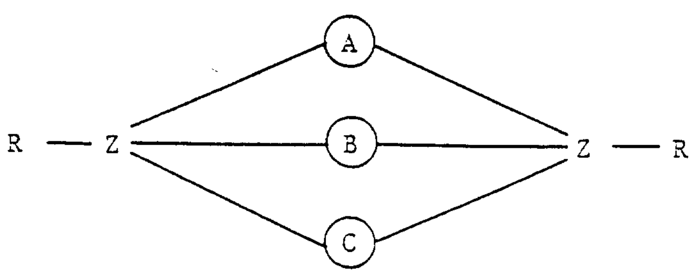

I

dans laquelle Z est un atome choisi parmi l'azote, le carbone ou le phosphore, R est rien ou représente l'hydrogène, le groupe hydroxy, un groupe amino ou un radical hydrocarboné, les radicaux bivalents Ⓐ , Ⓑ et Ⓒ sont indépendamment l'un de l'autre des chaînes hydrocarbonées contenant au moins deux atomes de carbone et éventuellement un ou plusieurs hétéroatomes choisis parmi l'oxygène et le soufre et éventuellement interrompues par un hétéromacrocycle, au moins l'un des radicaux Ⓐ , Ⓑ ou Ⓒ comportant de plus au moins un motif moléculaire ou étant essentiellement constitué par un motif moléculaire, ledit motif moléculaire possédant une énergie de triplet supérieure à celle du niveau

émissif de l'ion de terre rare complexé, à la condition que, lorsque le sel de terre rare est un sel d'europium ou de terbium, Z est l'azote, Ⓐ est $-(CH_2)_2-O-C_6H_3R_1-O-(CH_2)_2-$ avec $R_1$ =H ou $NH_2$, Ⓑ et Ⓒ ne sont pas simultanément l'un le radical $-(CH_2)_2-O-(CH_2)_2-O-(CH_2)_2-$ et l'autre le radical $-(CH_2)_2-O-(CH_2)_2-$, et que, lorsque le sel de terre rare est un sel de lanthane ou de praséodyme, Z est l'azote, Ⓐ est $-(CH_2)_2-O-C_6H_3NH_2-O-(CH_2)_2-$ Ⓑ et Ⓒ ne sont pas simultanément $-(CH_2)_2-O-(CH_2)_2-$ et $-(CH_2)_2-O-(CH_2)_2-O-(CH_2)_2$.

**3.** Procédé selon la revendication 2, caractérisé en ce que les chaînes hydrocarbonées sont des chaînes oxyéthylénées ou polyoxyéthylénées.

**4.** Procédé selon l'une des revendications 2 ou 3, caractérisé en ce que l'ion de terre rare est choisi parmi l'europium, le terbium, le samarium et le dysprosium.

**5.** Procédé selon l'une quelconque des revendications 2 à 4, caractérisé en ce que le motif donneur d'énergie a une longueur d'onde de phosphorescence inférieure à 580 nm.

**6.** Procédé selon l'une quelconque des revendications 2 à 5, caractérisé en ce que le motif moléculaire est choisi parmi la phénanthroline, l'anthracène, le benzène, le naphtalène, les bi- et ter-phényle, l'azobenzène, l'azopyridine, la pyridine, les bipyridines, les bi-quinoléines notamment les bi-isoquinoléines et les composés de formules ci-après :

$- C_2H_4 - X_1 - C_6H_4 - X_2 - C_2H_4 -$

$- C_2H_4 - X_1 - CH_2 - C_6H_4 - CH_2 - X_2 - C_2H_4 -$

dans lesquelles $X_1$ et $X_2$ pouvant être identiques ou différents désignent l'oxygène ou le soufre ;

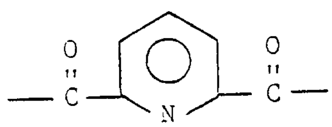

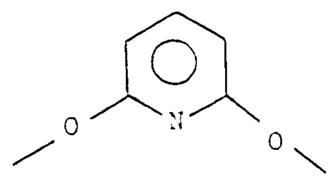

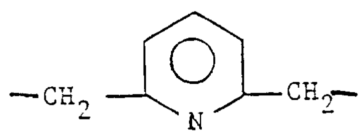

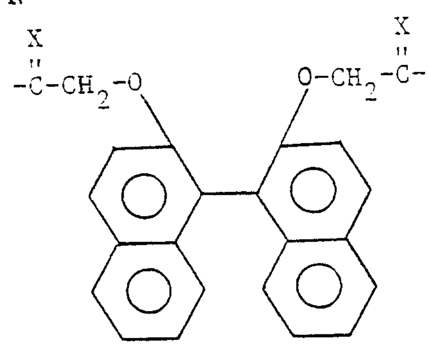

X étant l'oxygène ou l'hydrogène.

**7.** Procédé selon l'une quelconque des revendications 2 à 6, caractérisé en ce que le complexe macropolycyclique est constitué de l'ion terbium ou europium complexé par l'un des composés macrocycliques ci-après :

2,9-[N,N',N,N'-bis(3,6-dioxaoctaméthylène)bis(aminométhyl)]-1,10-phénanthroline ; 2,9-[N,N',N,N'-bis-(3,6-dioxaoctaméthylène)bis(aminocarbonyl)]-1,10-phénanthroline ; 9,10-[N,N',N,N'-bis(3,6-dioxaoctaméthylène)bis(aminoéthyl)]anthracène ; 9,10-[N,N',N,N'-bis(3,6-dioxaoctaméthylène)bis(carbamoylméthyl)]-anthracène ; 1,1'-[N,N',N,N'-bis(3,6-dioxaoctaméthylène)bis(aminométhyl)]-3,3'-biisoquinoléine ; 6,6'-[N,N',N,N'-bis(3,6-dioxaoctaméthylène)bis(aminométhyl)]-4,4'-diphényl-2,2'-bipyridine ; 6,6'-[N,N',N,N'-bis(3,6-dioxaoctaméthylène)bis(aminométhyl)-2,2'-bipyridine ; 6,6'-[N,N',N,-N'-bis(3,6-dioxaoctaméthylène)bis(aminocarbonyl)]-2,2'-bipyridine ; 6,6',6'',6''',6'''',6'''''-bis[nitrilotri(méthylène)]tris-2,2'-bipyridine ;

2,2',2'',9,9',9''-bis[nitrilotri(méthylène)]tris(1,10-phénanthroline) ; 2,9-[N,N',N,N'-bis(2,2'-bipyridine-6,6'-diméthyl) bis(aminométhyl)]-1,10-phénanthroline ; 1,1'-[N,N',N,N'-bis(2,2'-bipyridine-6,6'-diméthyl)bis-(aminométhyl)]-3,3'-biisoquinoléine ; 6,6'-[N,N',N,N'-bis(2,2'-bipyridine-6,6'-diméthyl) - bis-(aminométhyl)]-4,4'-diphényl-2,2'-bipyridine.

**8.** Procédé selon la revendication 2, caractérisé en ce que Z est l'azote.

**9.** Procédé selon la revendication 2, caractérisé en ce que le composé macropolycyclique est choisi parmi les composés ci-après :

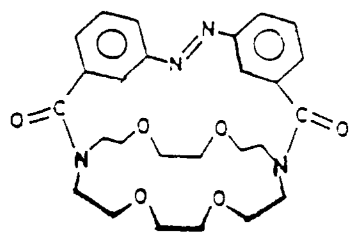

- (22) pyridine amide

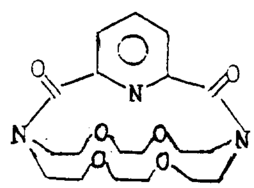

- (22) pyridine amine

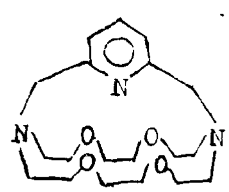

- (22) bipyridine

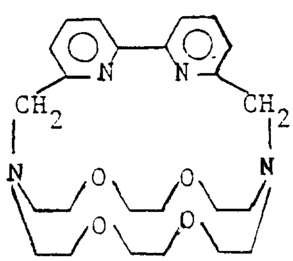

- (22) bipyridine amide

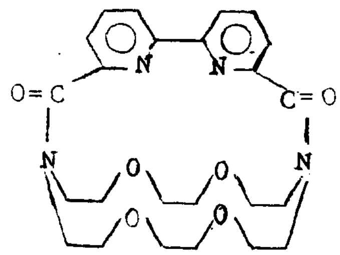

- (22) azopyridine

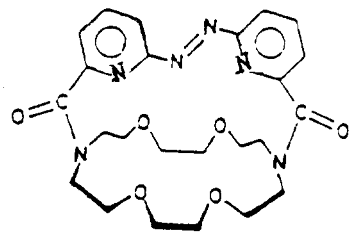

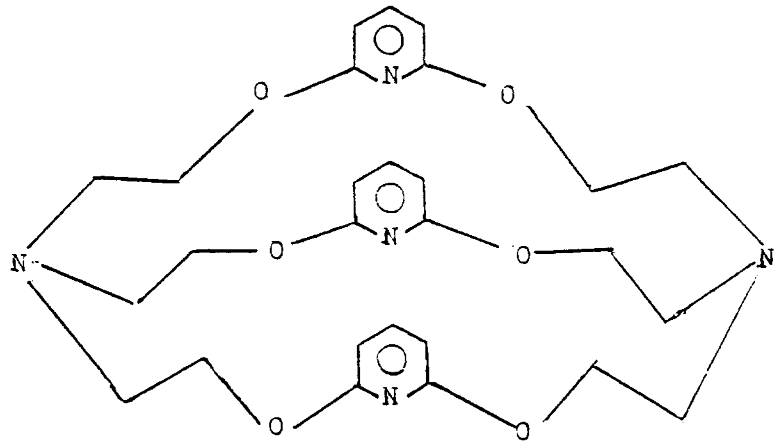

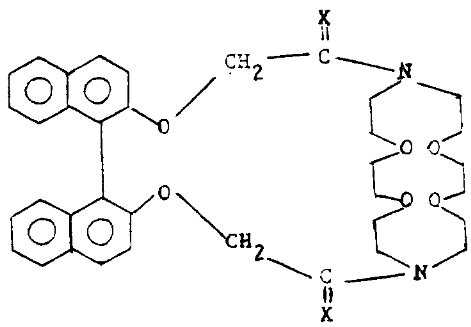

X = O, H

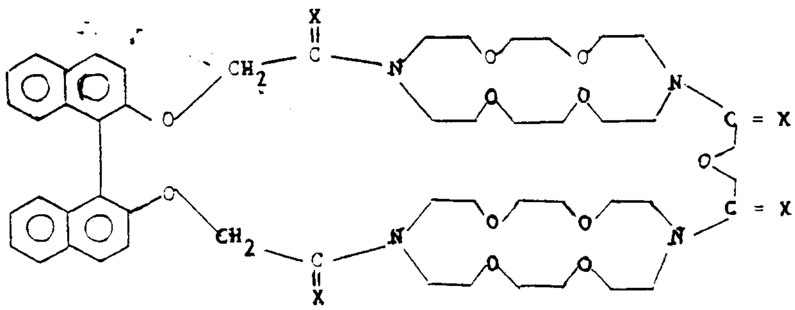

X = O, H

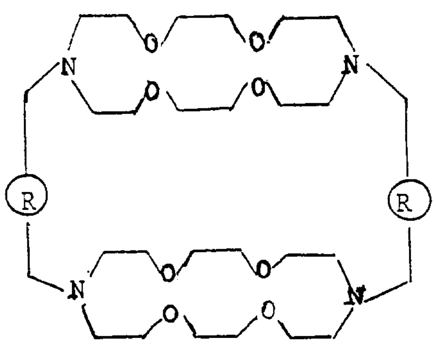

R =

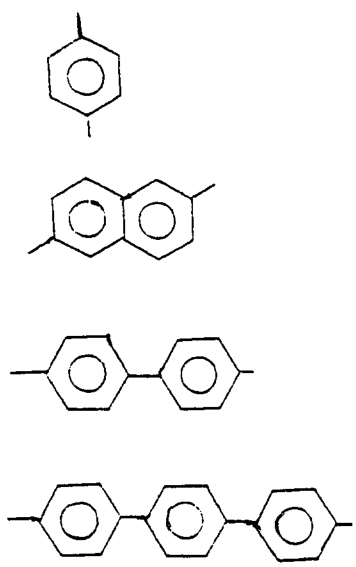

**10.** Procédé selon la revendication 2, caractérisé en ce que Z = N, Ⓐ et Ⓑ sont deux chaînes mono ou polyoxygénées et en ce que Ⓒ répond à l'une des formules ci-après :

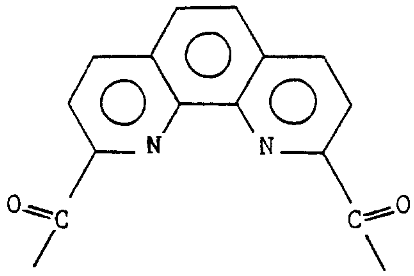
;
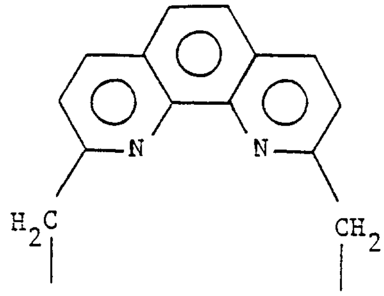

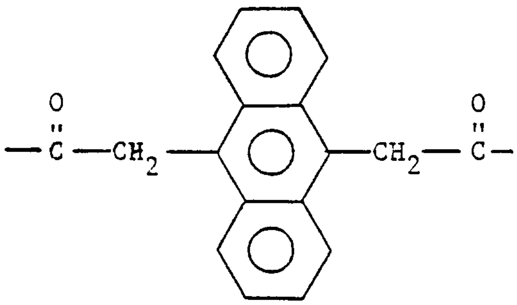
;
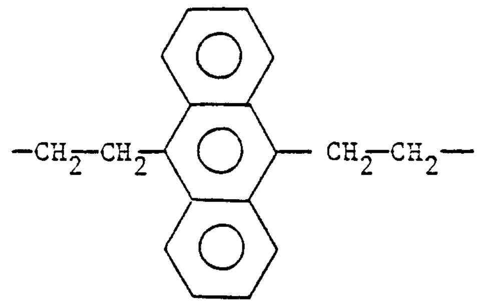

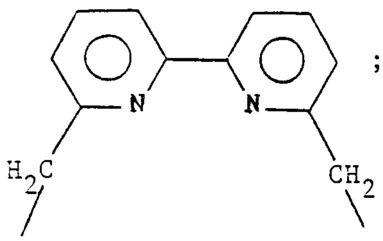
;
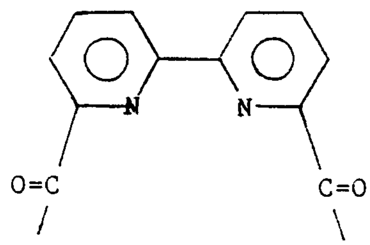
;

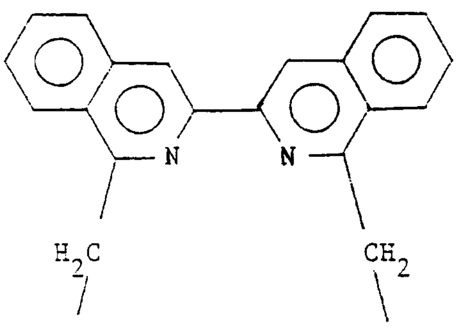
ou
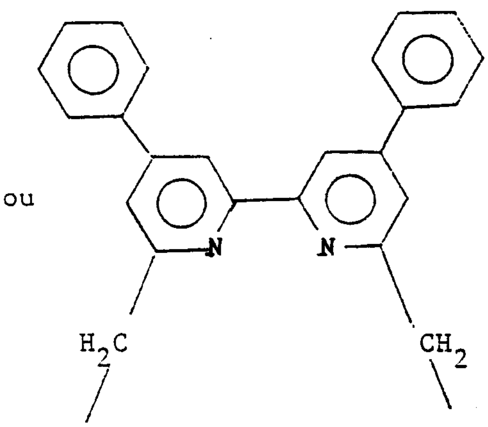

**11.** Procédé selon la revendication 2, caractérisé en ce que Ⓐ et Ⓑ représentent chacun le groupe

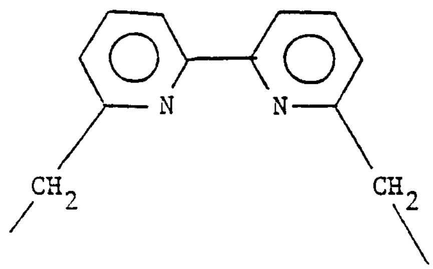

et Ⓒ est l'un des groupes suivants :

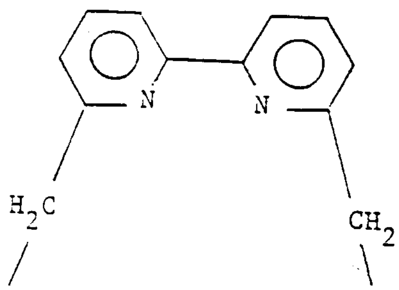

;

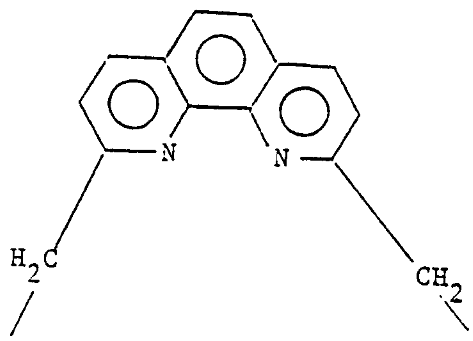

;

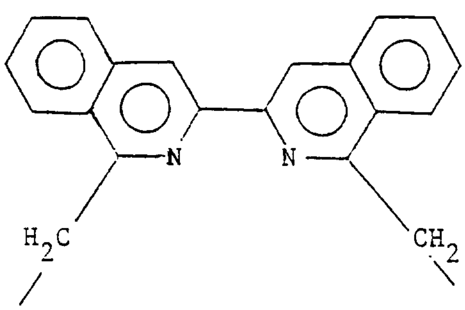

ou

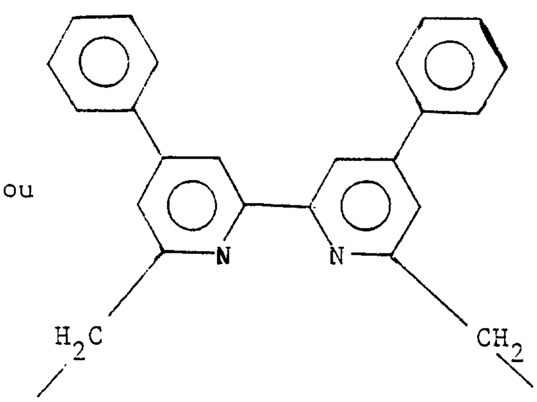

**12.** Procédé selon la revendication 2, caractérisé en ce que Z = N; Ⓐ, Ⓑ et Ⓒ sont identiques et représentent l'un des hétérocycles ci-après :

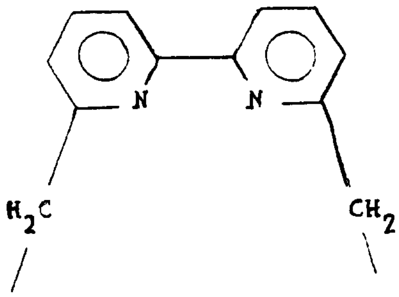 ; 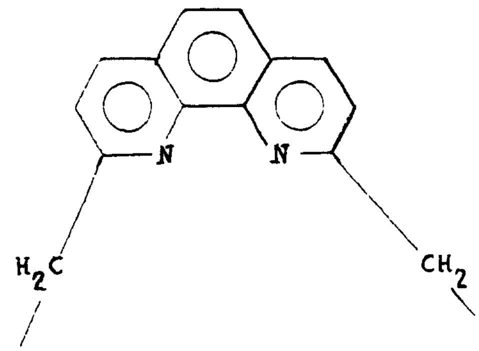 ;

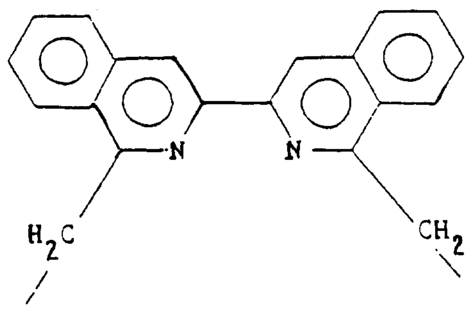 ou 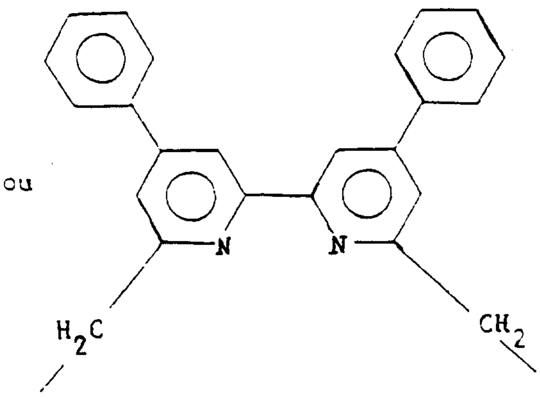

**13.** Procédé pour l'obtention de composés macropolycycliques de formule :

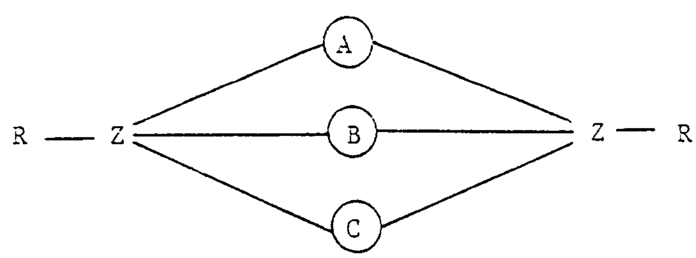 I

dans laquelle Z est un atome choisi parmi l'azote, le carbone ou le phosphore, R est rien ou représente l'hydrogène, le groupe hydroxy, un groupe amino ou un radical hydrocarboné, les radicaux bivalents Ⓐ , Ⓑ et Ⓒ sont indépendamment l'un de l'autre des chaînes hydrocarbonées contenant au moins deux atomes de carbone et éventuellement un ou plusieurs hétéroatomes choisis parmi l'oxygène ou le soufre et étant éventuellement interrompues par un hétéromacrocycle, au moins l'un des radicaux Ⓐ , Ⓑ ou Ⓒ comportant de plus au moins un motif moléculaire ou étant essentiellement constitué par un motif moléculaire, ledit motif moléculaire possédant une énergie de triplet supérieure à celle du niveau émissif de l'ion de terre rare complexé et étant choisi parmi la phénanthroline, l'anthracène, les bipyridines et les bi-quinoléines, notamment les bi-isoquinoléines, à la condition que, lorsque Z est l'azote et Ⓐ répond à l'une des formules ci-après :

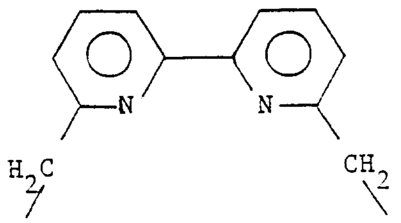

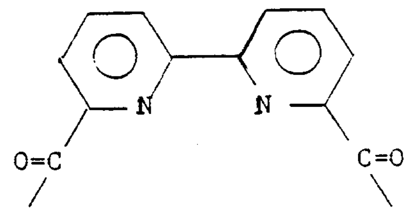

Ⓑ et Ⓒ ne sont pas simultanément $-(CH_2)_2-O-(CH_2)_2-O-(CH_2)_2-$,caractérisé en ce qu'il consiste à condenser des composés chimiques comportant chacun un des radicaux Ⓐ , Ⓑ ou Ⓒ et des groupes terminaux capables d'être substitués ou comportant des radicaux facilement éliminables.

**14.** Procédé selon la revendication 13, caractérisé en ce que Z est l'azote et Ⓐ et Ⓑ sont des chaînes polyoxyéthylénées, caractérisé en ce qu'il consiste :

1) à faire réagir :

- le macrocycle azoté constitué par les deux chaînes polyoxyéthylénées avec
- une chaîne hydrocarbonée comportant ledit motif moléculaire, ladite chaîne ayant des groupes terminaux clivables, tels que par exemple des groupes halogéno,

dans un solvant anhydre et éventuellement en présence d'un agent réducteur tel que l'hydrure de sodium ou le carbonate de sodium et

2) éventuellement à transformer le sel obtenu en composé macropolycyclique libre.

**15.** Procédé selon l'une des revendications 13 ou 14, pour l'obtention des composés ci-après : 2,9-[N,N',N,N'-bis(3,6-dioxaoctaméthylène)bis(aminométhyl)]-1,10-phénanthroline ; 2,9-[N,N',N,N'-bis-(3,6-dioxaoctaméthylène)bis(aminocarbonyl)]-1,10-phénanthroline, 9,10-[N,N',N,N'-bis(3,6-dioxaoctaméthylène)bis(aminoéthyl)]-anthracène ; 9,10-[N,N',N,N'-bis(3,6-dioxaoctaméthylène)bis(carbamoylméthyl)-]anthracène ; 1,1'-[N,N',N,N'-bis(3,6-dioxaoctaméthylène)bis(aminométhyl)]-3,3'-biisoquinoléine ; 6,6'-[N,N',N,N'-bis(3,6-dioxaoctaméthylène)bis(aminométhyl)]-4,4'-diphényl-2,2'-bipyridine.

**16.** Procédé selon la revendication 13, pour l'obtention des composés ci-après : 6,6',6'',6''',6'''',6'''''-bis[nitrilotri(méthylène)]tris-2,2'-bipyridine ; 2,9-[N,N',N,N'-bis(2,2'-bipyridine-6,6'-diméthyl)bis(aminométhyl)]-1,10-phénanthroline ; 1,1'-[N,N',N,N'-bis(2,2'-bipyridine-6,6'-diméthyl)bis-(aminométhyl)]-3,3'-biisoquinoléine ; 2,2',-2'',9,9',9''-bis[nitrilotri(méthylène)]tris(1,10-phénanthroline) ; 6,6'-[N,N',N,N'-bis(2,2'-bipyridine-6,6'-diméthyl)bis(aminométhyl)]-4,4'-diphényl-2,2'-bipyridine.

**17.** Procédé de préparation d'un complexe biologique, caractérisé en ce que l'on associe une molécule biologiquement active par couplage avec, ou adsorption sur, un complexe macropolycyclique de terre rare obtenu par un procédé tel que défini dans l'une des revendications 2 à 12.

**18.** Procédé selon la revendication 17, caractérisé en ce que la molécule biologiquement active est choisie parmi les antigènes, les anticorps, les anticorps monoclonaux, les fragments et combinaisons Ac-fragments, les drogues, les récepteurs, les hormones, les récepteurs hormonaux, les bactéries, les stéroïdes, les aminoacides, les peptides, les virus, les vitamines, les nucléotides ou polynucléotides, les enzymes, leurs substrats, les lectines, les acides nucléiques, l'ADN et l'ARN.

**Claims**

**Claims for the Contracting States : BE, CH, DE, FR, GB, IT, LI, LU, NL, SE**

**1.** Application, as labels of biological products in the methods of immunological detection and determination by fluorescence, of macropolycyclic complexes of rare earths consisting of at least one rare earth salt complexed by a macropolycyclic compound of general formula:

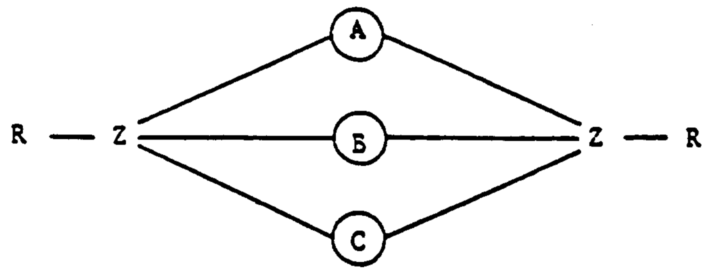

I

in which Z is an atom chosen from nitrogen, carbon or phosphorus, R does not exist or represents hydrogen, the hydroxyl group, an amino group or a hydrocarbon radical, the bivalent radicals Ⓐ , Ⓑ and Ⓒ are, independently from each other, hydrocarbon chains containing at least two atoms of carbon and optionally one or more heteroatoms chosen from oxygen or sulphur and being optionally interrupted by a heteromacrocycle, at least one of the radicals Ⓐ , Ⓑ and Ⓒ moreover comprising at least one molecular unit or essentially consisting of a molecular unit, the said molecular unit having a triplet energy greater than that of the emissive level of the complexed rare earth ion.

**2.** Application according to Claim 1, characterised in that the said rare earth complexes are substituted by a radical chosen from the alkylamino, arylamino, isothiocyano, cyano, isocyano, thiocyano, carboxyl, hydroxyl, mercapto, phenol, imidazole, aldehyde, epoxide radicals; from thionyl halides, sulphonyl halides, nitrobenzoyl halides, carbonyl halides; from the triazo, succinimido, anhydride, haloacetate, hydrazino, dihalotriazinyl radicals.

**3.** As new products, the macropolycyclic rare earth complexes consisting of at least one rare earth salt complexed by a macropolycyclic compound of formula:

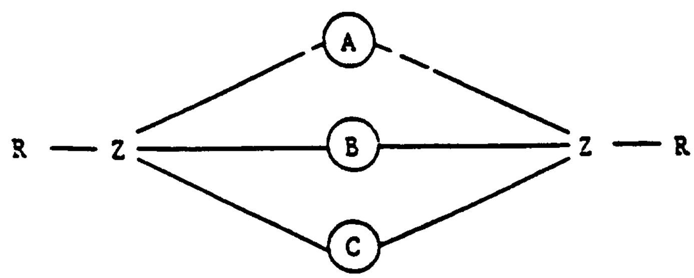

I

in which Z is an atom chosen from nitrogen, carbon or phosphorus, R does not exist or represents hydrogen, the hydroxyl group, an amino group or a hydrocarbon radical, the bivalent radicals Ⓐ , Ⓑ and Ⓒ are, independently of each other, hydrocarbon chains containing at least two atoms of carbon and optionally one or more heteroatoms chosen from oxygen and sulphur and optionally interrupted by a heteromacrocycle, at least one of the radicals Ⓐ , Ⓑ and Ⓒ moreover comprising at least one molecular unit or essentially consisting of a molecular unit, the said molecular unit having a triplet energy greater than that of the emissive level of the complexed rare earth ion, with the proviso that, when the rare earth salt is a europium or terbium salt, Z is nitrogen, Ⓐ is $-(CH_2)_2-O-C_6H_3R_1-O-(CH_2)_2-$ with $R_1$ = H or $NH_2$, Ⓑ and Ⓒ are not simultaneously one the $-(CH_2)_2-O-(CH_2)_2-O-(CH_2)_2-$ radical and the other the $-(CH_2)_2-O-(CH_2)_2-$ radical, and that, when the rare earth salt is a lanthanum or praseodymium salt, Z is nitrogen, Ⓐ is $-(CH_2)_2-O-C_6H_3NH_2-O-(CH_2)_2-$, Ⓑ and Ⓒ are not simultaneously $-(CH_2)_2-O-(CH_2)_2-$ and $-(CH_2)_2-O-(CH_2)_2-O-(CH_2)_2$.

**4.** Complexes according to Claim 3, characterised in that the hydrocarbon chains are oxyethylenated or polyoxyethylenated chains.

**5.** Macropolycyclic complexes according to one of Claims 3 and 4, characterised in that the rare earth ion is chosen from europium, terbium, samarium and dysprosium.

6. Macropolycyclic complexes according to any one of Claims 3 to 5, characterised in that the energy-donor unit has a phosphorescence wavelength of less than 580 nm.

7. Macropolycyclic complexes according to any one of Claims 3 to 6, characterised in that the molecular unit is chosen from phenanthroline, anthracene, benzene, naphthalene, biphenyl and terphenyl, azobenzene, azopyridine, pyridine, the bipyridines, the biquinolines, in particular the bisisoquinolines and the compounds having the formulae below:
$-C_2H_4-X_1-C_6H_4-X_2-C_2H_4-$
$-C_2H_4-X_1-CH_2-C_6H_4-CH_2-X_2-C_2H_4-$
in which $X_1$ and $X_2$, which can be identical or different, represent oxygen or sulphur;

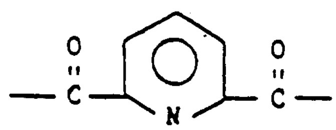

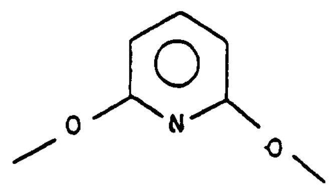

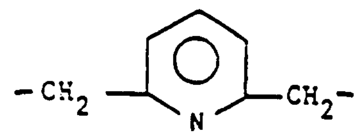

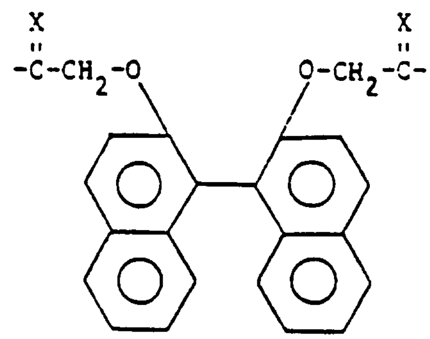

X being oxygen or hydrogen.

8. Macropolycyclic complex according to any one of Claims 3 to 7, characterised in that it consists of the terbium or europium ion complexed by one of the macrocyclic compounds below: 2,9-[N,N',N,N'-bis(3,6-dioxaoctamethylene)bis(aminomethyl)]-1,10-phenanthroline; 2,9-[N,N',N,N'-bis(3,6-dioxaoctamethylene)bis(aminocarbonyl)]-1,10-phenanthroline; 9,10-[N,N',N,N'-bis(3,6-dioxaoctamethylene)bis(aminoethyl)]anthracene; 9,10-[N,N',N,N',-bis(3,6-dioxaoctamethylene)bis-(carbamoylmethyl)]anthracene; 1,1'-[N,N',N,N'-bis(3,6-dioxaoctamethylene)bis(aminomethyl)]-3,3'-bisisoquinoline; 6,6'-[N,N',N,N'-bis(3,6-dioxaoctamethylene)bis(aminomethyl)]-4,4'-diphenyl-2,2'-bipyridine; 6,6'-[N,N',N,N'-bis(3,6-dioxaoctamethylene)bis(aminomethyl)]-2,2'-bipyridine; 6,6'-[N,N',N,N'-bis(3,6-dioxaoctamethylene)bis(aminocarbonyl)]-2,2'-bipyridine; 6,6',6'',6''',6'''',6'''''-bis[nitrilotri-(methylene)] tris-2,2'-bipyridine; 2,2',2'',9,9',9''-bis[nitrilotri(methylene)] tris(1,10-phenanthroline); 2,9-[N,N',N,N'-bis(2,2'-bipyridine-6,6'-dimethyl)bis(aminomethyl)]-1,10-phenanthroline; 1,1'-[N,N',N,N'-bis-(2,2'-bipyridine-6,6'-dimethyl)bis(aminomethyl)]-3,3'-biisoquinoline; 6,6'-[N,N',N,N'-bis(2,2'-bipyridine-6,6'-dimethyl)bis(aminomethyl)]-4,4'-diphenyl-2,2'-bipyridine.

9. Macropolycyclic complexes according to Claim 3, characterised in that they correspond to the formula (I) in which Z = N.

10. Macropolycyclic rare earth complexes consisting of at least one rare earth salt complexed by a macropolycyclic compound, characterised in that the said macropolycyclic compound is chosen from the compounds below:

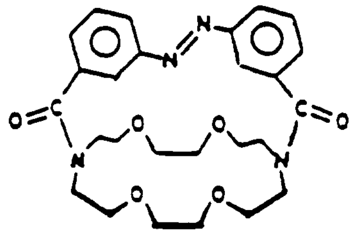

- (22) pyridine amide

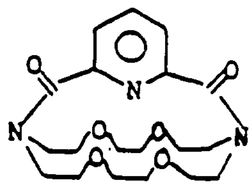

- (22) pyridine amine

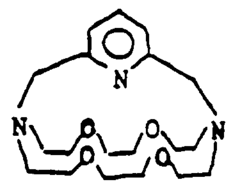

- (22) bipyridine

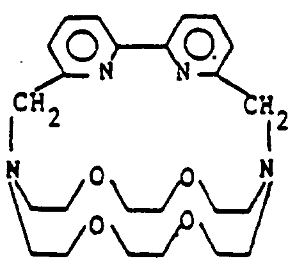

- (22) bipyridine amide

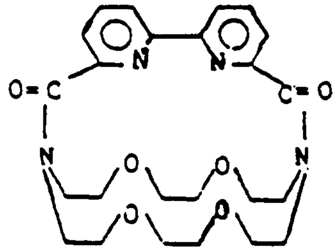

- (22) azopyridine

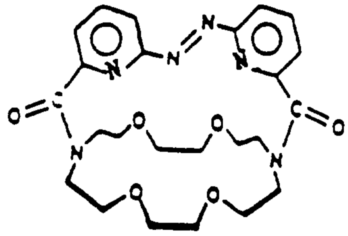

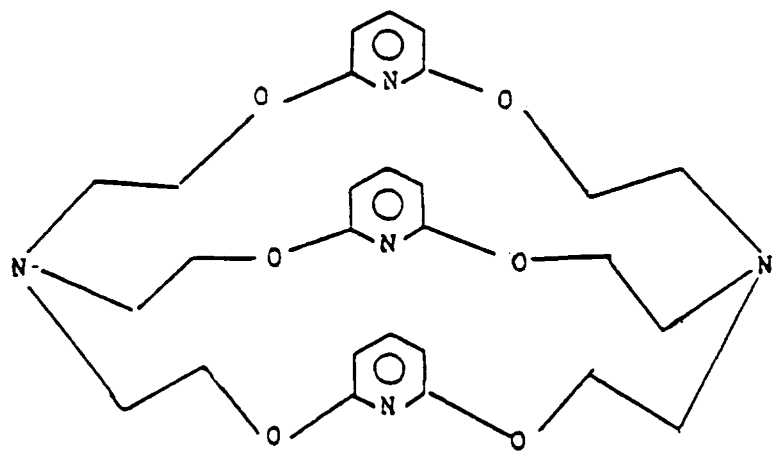

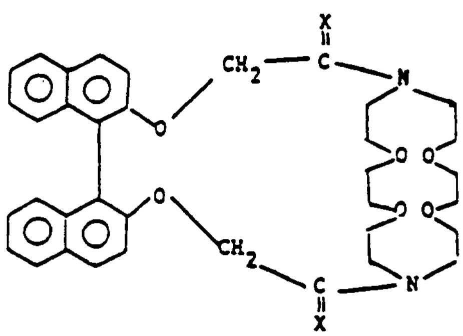

X = O, H

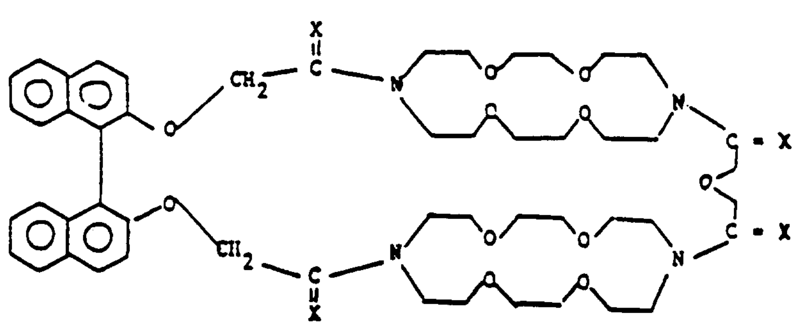

X = O, H

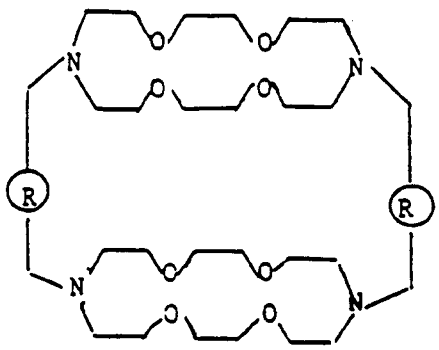

R =

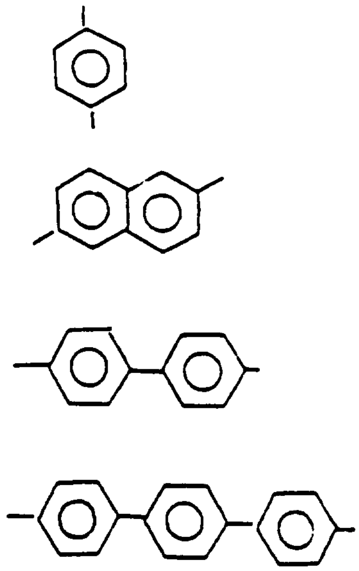

11. Macropolycyclic rare earth complexes consisting of at least one rare earth salt complexed by a macropolycyclic compound of formula I according to Claim 3, characterised in that Z = N, Ⓐ and Ⓑ are two chains containing one or more oxygen atoms and in that Ⓒ corresponds to one of the formulae below:

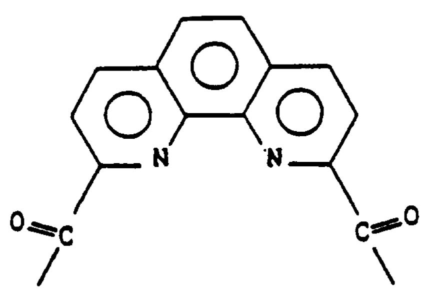

;

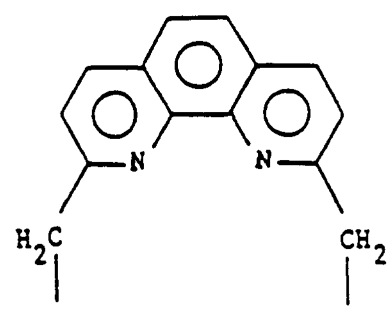

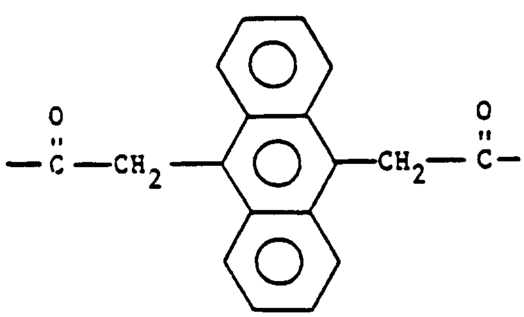

;

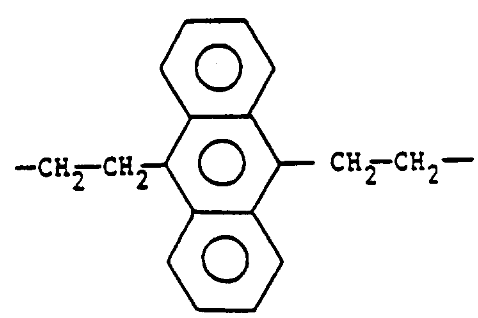

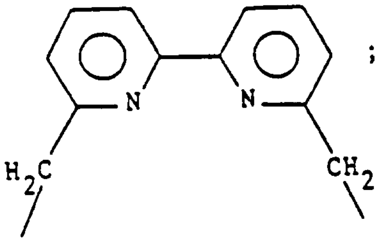 ; 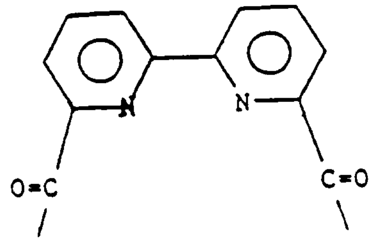 ;

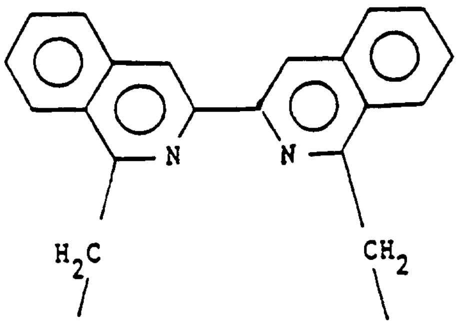 or 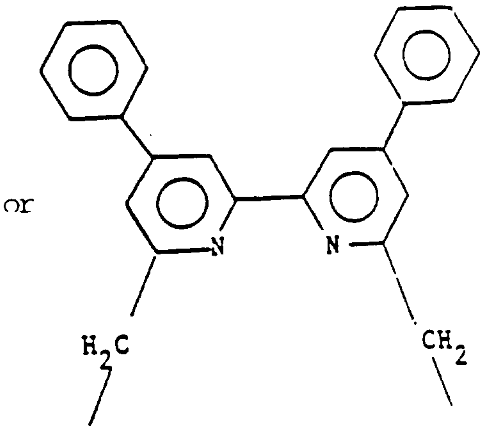

12. Macropolycyclic rare earth complexes consisting of at least one rare earth salt complexed by a macropolycyclic compound of formula I according to Claim 3, characterised in that Ⓐ and Ⓑ each represent the group

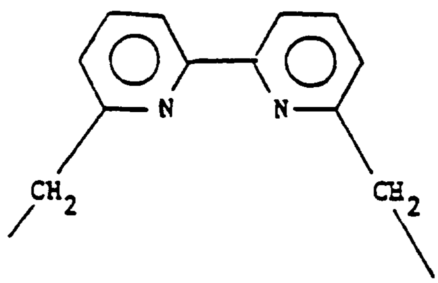

and Ⓒ is one of the following groups:

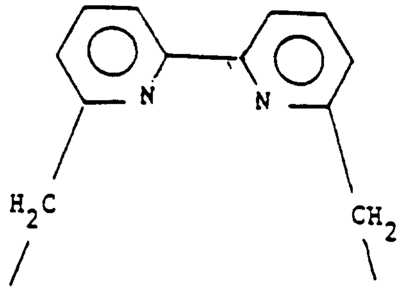 ; 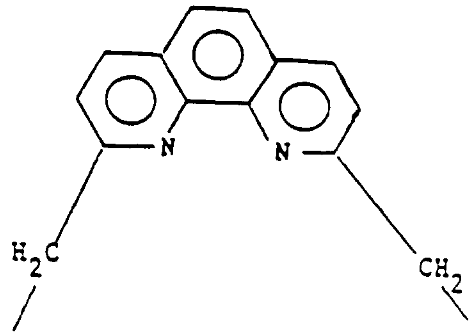 ;

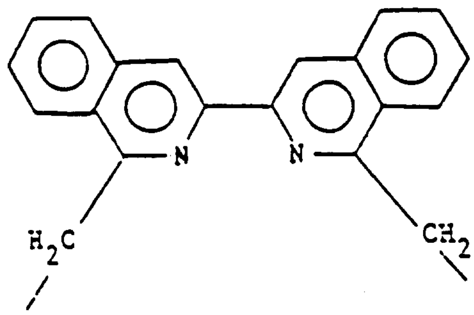 or 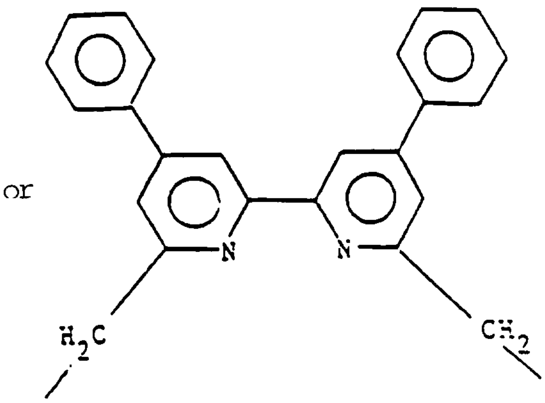

**13.** Macropolycyclic rare earth complexes consisting of at least one rare earth salt complexed by a macropolycyclic compound of formula I according to Claim 3, characterised in that Z = N; Ⓐ, Ⓑ and Ⓒ are identical and represent one of the heterocycles below:

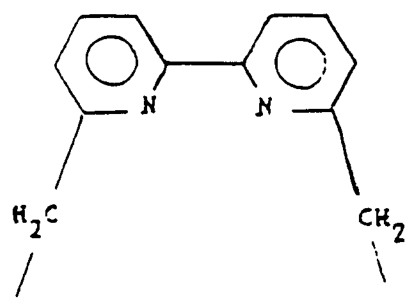 ; 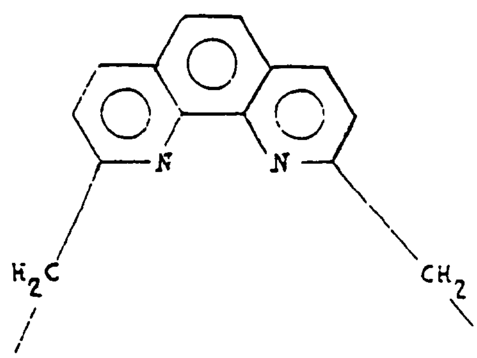 :

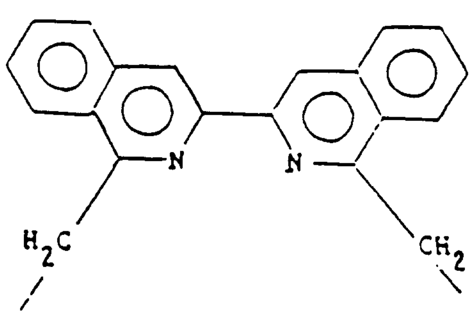 or 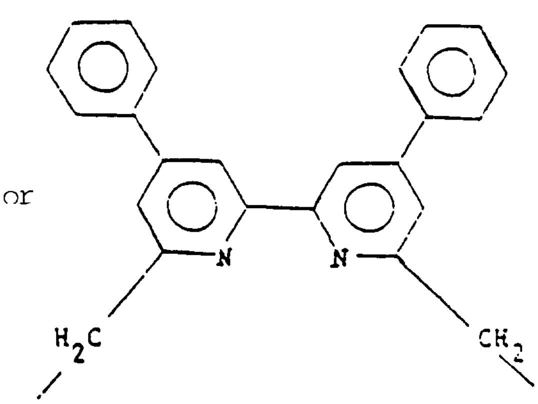

**14.** Macropolycyclic compound corresponding to the formula:

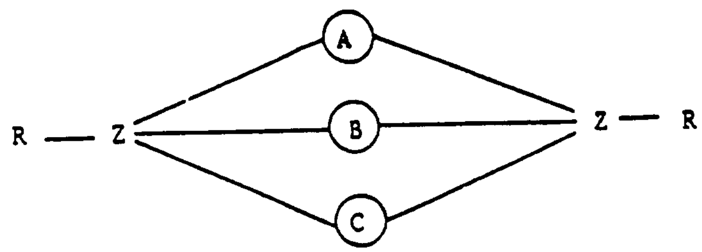

I

in which Z is an atom chosen from nitrogen, carbon or phosphorus, R does not exist or represents hydrogen, the hydroxyl group, an amino group or a hydrocarbon radical, the bivalent radicals Ⓐ, Ⓑ and Ⓒ are, independently of each other, hydrocarbon chains containing at least two atoms of carbon and optionally one or more heteroatoms chosen from oxygen or sulphur and optionally interrupted by a heteromacrocycle, at least one of the radicals Ⓐ, Ⓑ and Ⓒ comprising moreover at least one molecular unit or essentially consisting of a molecular unit, the said molecular unit having a triplet energy greater than that of the emissive level of the complexed rare earth ion and being chosen from phenanthroline, anthracene, the bipyridines and the biquinolines, in particular the biisoquinolines, with the proviso that, when Z is nitrogen and Ⓐ corresponds to one of the formulae below:

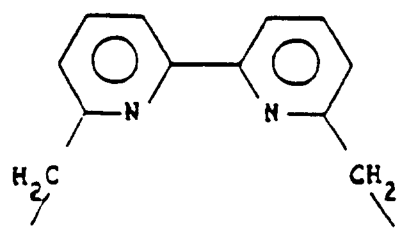

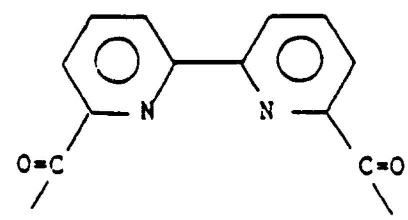

Ⓑ and Ⓒ are not simultaneously $-(CH_2)_2-O-(CH_2)_2-O-(CH_2)_2-$.

**15.** Compound according to Claim 14, characterised in that Z is nitrogen and Ⓐ and Ⓑ are polyoxyethylenated chains.

**16.** Compound according to one of Claims 14 and 15, characterised in that it is one of the compounds below:
2,9-[N,N',N,N'-bis(3,6-dioxaoctamethylene)bis(aminomethyl)]-1,10-phenanthroline
2,9-[N,N',N,N'-bis(3,6-dioxaoctamethylene)bis(aminocarbonyl)]-1,10-phenanthroline
9,10-[N,N',N,N'-bis(3,6-dioxaoctamethylene)bis(aminoethyl)]anthracene
9,10-[N,N',N,N'-bis(3,6-dioxaoctamethylene)bis(carbamoylmethyl)]anthracene
1,1'-[N,N',N,N'-bis(3,6-dioxaoctamethylene)bis(aminomethyl)]-3,3'-bisisoquinoline
6,6'-[N,N',N,N'-bis(3,6-dioxaoctamethylene)bis(aminomethyl)]-4,4'-diphenyl-2,2'-bipyridine.

**17.** Compound according to Claim 14, characterised in that the said compound is such as defined in any one of Claims 9 to 13.

**18.** Compound according to Claim 14, characterised in that it is 6,6',6'',6''',6'''',6'''''-bis[nitrilotri(methylene)-]tris-2,2'-bipyridine; 2,9-[N,N',N,N'-bis(2,2'-bipyridine-6,6'-dimethyl)bis(aminomethyl)]-1,10-phenanthroline; 1,1'-[N,N',N,N'-bis(2,2'-bipyridine-6,6'-dimethyl)bis(aminomethyl)]-3,3'-bisisoquinoline; 2,2',2'',9,9',9''-[nitrilotri(methylene)]tris(1,10-phenanthroline); 6,6'-[N,N',N,N'-bis(2,2'-bipyridine-6,6'-dimethyl)bis(aminomethyl)]-4,4'-diphenyl-2,2'-bipyridine.

**19.** Biological complex, characterised in that it consists of a biologically active molecule associated by coupling with, or adsorption on, a macropolycyclic rare earth complex such as defined in Claims 1, 3 to 13.

**20.** Biological complex according to Claim 19, characterised in that the biologically active molecule is chosen from antigens, antibodies, monoclonal antibodies, fragments and Ab-fragment combinations, drugs, receptors, hormones, hormonal receptors, bacteria, steroids, amino-acids, peptides, viruses, vitamins, nucleotides or polynucleotides, enzymes, their substrates, lectins, nucleic acids, DNA and RNA.

**21.** Method of exaltation of the fluorescence of rare earth ions, characterised in that it consists in complexing at least one rare earth ion with a macropolycyclic compound such as defined in Claims 1, 3 to 13, and in exciting the molecular unit of the said compound.

**Claims for the following Contracting State : AT**

**1.** Method of exaltation of the fluorescence of a rare earth ion, characterised in that it consists in complexing a rare earth ion with a macropolycyclic compound of general formula I below or one of its salts

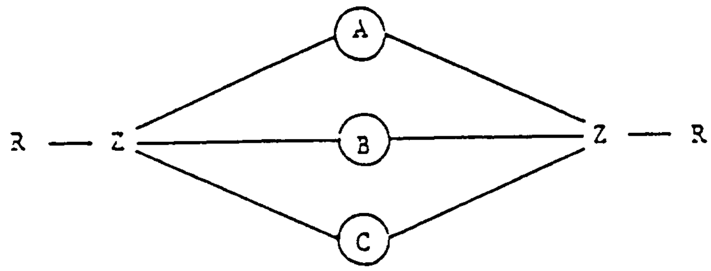

I

in which Z is an atom chosen from nitrogen, carbon or phosphorus, R does not exist or represents hydrogen, the hydroxyl group, an amino group or a hydrocarbon radical, the bivalent radicals Ⓐ, Ⓑ and Ⓒ are, independently from each other, hydrocarbon chains containing at least two atoms of carbon and optionally one or more heteroatoms chosen from oxygen or sulphur and being optionally interrupted by a heteromacrocycle, at least one of the radicals Ⓐ, Ⓑ and Ⓒ moreover comprising at least one molecular unit or essentially consisting of a molecular unit, the said molecular unit having a triplet energy greater than that of the emissive level of the complexed rare earth ion, and in exciting the molecular unit of the said macropolycyclic compound.

**2.** Method for obtaining macropolycyclic compounds of rare earths, characterised in that it consists in reacting at least one rare earth salt with a macropolycyclic compound of formula:

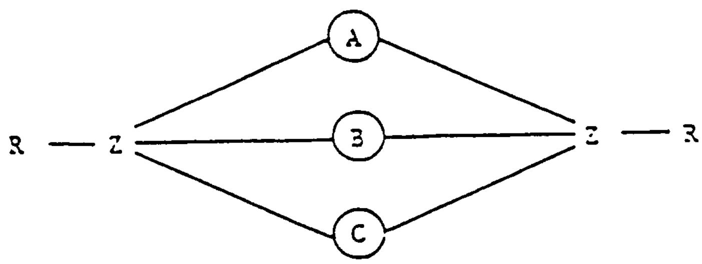

I

in which Z is an atom chosen from nitrogen, carbon or phosphorus, R does not exist or represents hydrogen, the hydroxyl group, an amino group or a hydrocarbon radical, the bivalent radicals Ⓐ, Ⓑ and Ⓒ are, independently of each other, hydrocarbon chains containing at least two atoms of carbon and optionally one or more heteroatoms chosen from oxygen and sulphur and optionally interrupted by a heteromacrocycle, at least one of the radicals Ⓐ, Ⓑ and Ⓒ moreover comprising at least one molecular unit or essentially consisting of a molecular unit, the said molecular unit having a triplet energy greater than that of the emissive level of the complexed rare earth ion, with the proviso that, when the rare earth salt is a europium or terbium salt, Z is nitrogen, Ⓐ is $-(CH_2)_2-O-C_6H_3R_1-O-(CH_2)_2-$ with $R_1$ = H or $NH_2$, Ⓑ and Ⓑ are not simultaneously one the $-(CH_2)_2-O-(CH_2)_2-O-(CH_2)_2-$ radical and the other the $-(CH_2)_2-O-(CH_2)_2-$ radical, and that, when the rare earth salt is a lanthanum or

praseodymium salt, Z is nitrogen, Ⓐ is $-(CH_2)_2-O-C_6H_3NH_2-O-(CH_2)_2-$, Ⓑ and Ⓒ are not simultaneously $-(CH_2)_2-O-(CH_2)_2-$ and $-(CH_2)_2-O-(CH_2)_2-O-(CH_2)_2$.

3. Method according to Claim 2, characterised in that the hydrocarbon chains are oxyethylenated or polyoxyethylenated chains.

4. Method according to one of Claims 2 and 3, characterised in that the rare earth ion is chosen from europium, terbium, samarium and dysprosium.

5. Method according to any one of Claims 2 to 4, characterised in that the energy-donor unit has a phosphorescence wavelength of less than 580 nm.

6. Method according to any one of Claims 2 to 5, characterised in that the molecular unit is chosen from phenanthroline, anthracene, benzene, naphthalene, biphenyl and terphenyl, azobenzene, azopyridine, pyridine, the bipyridines, the biquinolines, in particular the bisisoquinolines and the compounds having the formulae below:
$-C_2H_4-X_1-C_6H_4-X_2-C_2H_4-$
$-C_2H_4-X_1-CH_2-C_6H_4-CH_2-X_2-C_2H_4-$
in which $X_1$ and $X_2$, which can be identical or different, represent oxygen or sulphur;

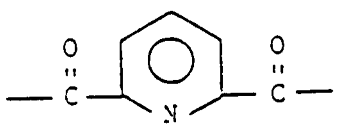

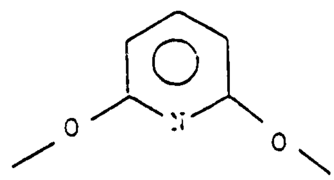

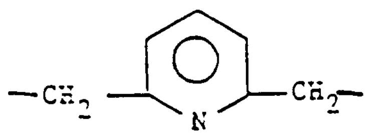

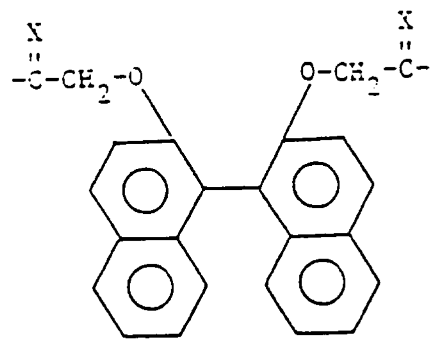

X being oxygen or hydrogen.

7. Method according to any one of Claims 2 to 6, characterised in that the macropolycyclic complex consists of the terbium or europium ion complexed by one of the macrocyclic compounds below: 2,9-[N,N',N,N'-bis(3,6-dioxaoctamethylene)bis(aminomethyl)]-1,10-phenanthroline; 2,9-[N,N',N,N'-bis(3,6-dioxaoctamethylene)bis(aminocarbonyl)]-1,10-phenanthroline; 9,10-[N,N',N,N'-bis(3,6-dioxaoctamethylene)bis(aminoethyl)]anthracene; 9,10-[N,N',N,N',-bis(3,6-dioxaoctamethylene)bis-(carbamoylmethyl)]anthracene; 1,1'-[N,N',N,N'-bis(3,6-dioxaoctamethylene)bis(aminomethyl)]-3,3'-bisisoquinoline; 6,6'-[N,N',N,N'-bis(3,6-dioxaoctamethylene)bis(aminomethyl)]-4,4'-diphenyl-2,2'-bipyridine; 6,6'-[N,N',N,N'-bis(3,6-dioxaoctamethylene)bis(aminomethyl)]-2,2'-bipyridine; 6,6'-[N,N',N,N'-bis(3,6-dioxaoctamethylene)bis(aminocarbonyl)]-2,2'-bipyridine; 6,6',6'',6''',6'''',6'''''-bis[nitrilotri-(methylene)] tris-2,2'-bipyridine; 2,2',2'',9,9',9''-bis[nitrilotri(methylene)] tris(1,10-phenanthroline); 2,9-[N,N',N,N'-bis(2,2'-bipyridine-6,6'-dimethyl)bis(aminomethyl)]-1,10-phenanthroline; 1,1'-[N,N',N,N'-bis-(2,2'-bipyridine-6,6'-dimethyl)bis(aminomethyl)]-3,3'-bisisoquinoline; 6,6'-[N,N',N,N'-bis(2,2'-bipyridine-6,6'-dimethyl)bis(aminomethyl)]-4,4'-diphenyl-2,2'-bipyridine.

8. Method according to Claim 2, characterised in that Z is nitrogen.

9. Method according to Claim 2, characterised in that the said macropolycyclic compound is chosen from the compounds below:

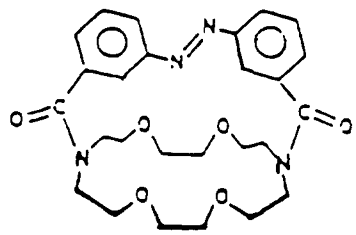

- (22) pyridine amide

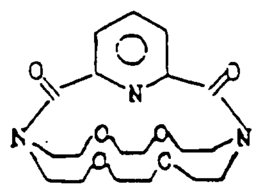

- (22) pyridine amine

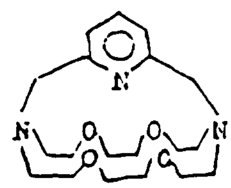

- (22) bipyridine

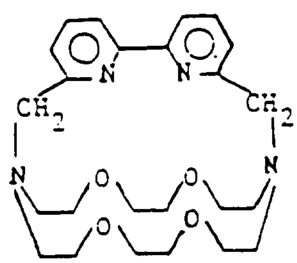

- (22) bipyridine amide

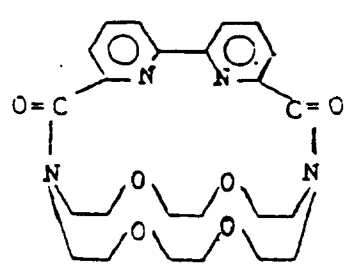

- (22) azopyridine

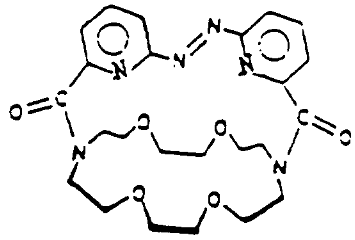

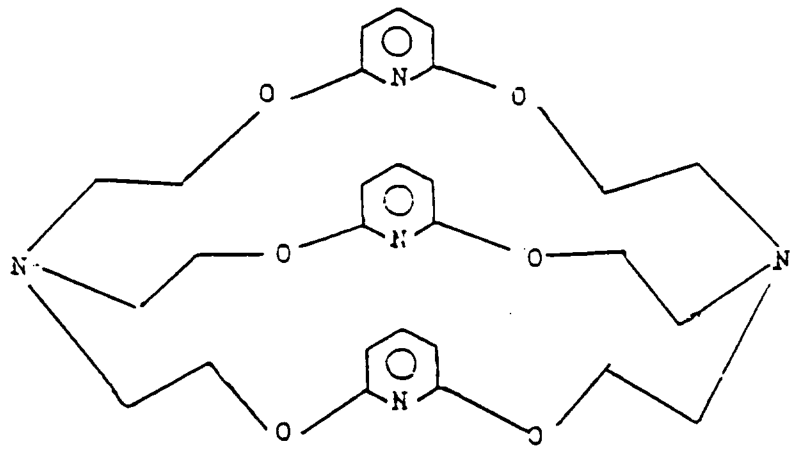

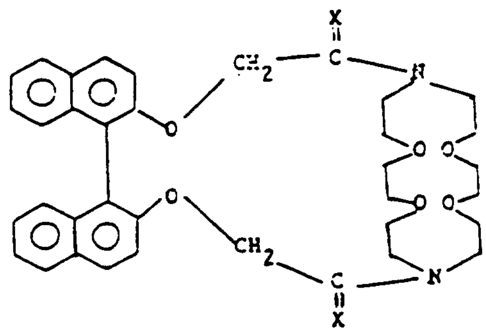

X = O, H

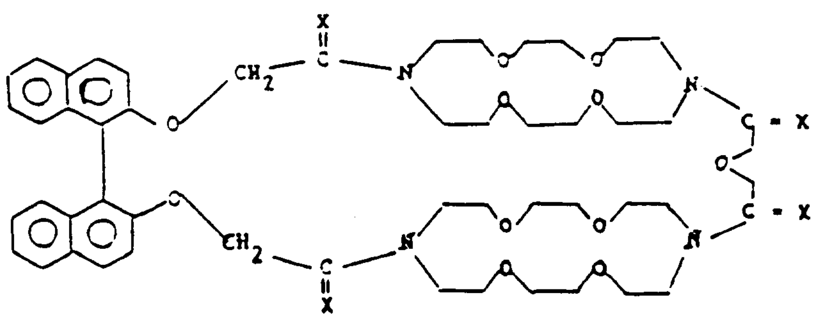

X = O, H

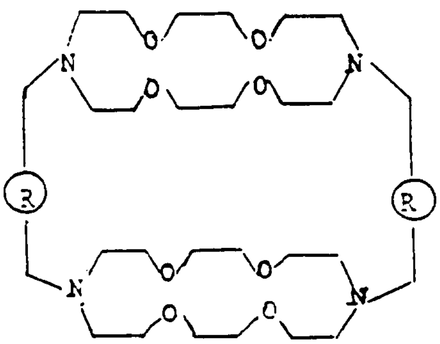

R =

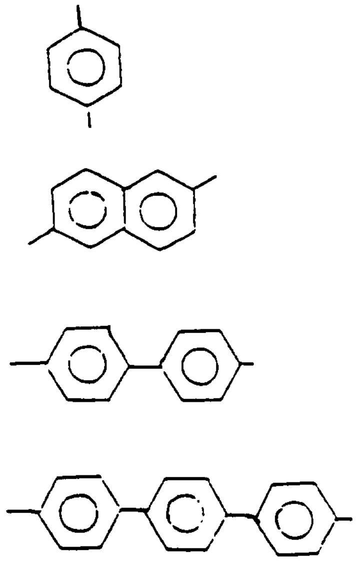

10. Method according to Claim 2, characterised in that Z = N, Ⓐ and Ⓑ are two chains containing one or more oxygen atoms and in that Ⓒ corresponds to one of the formulae below:

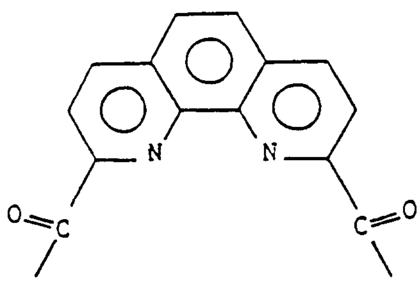

;

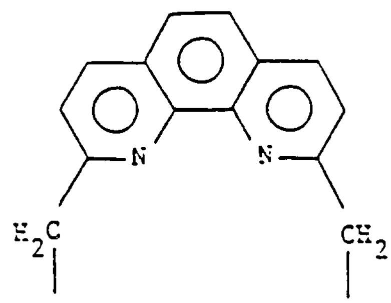

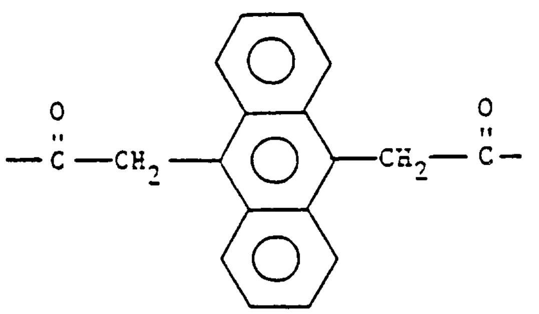

;

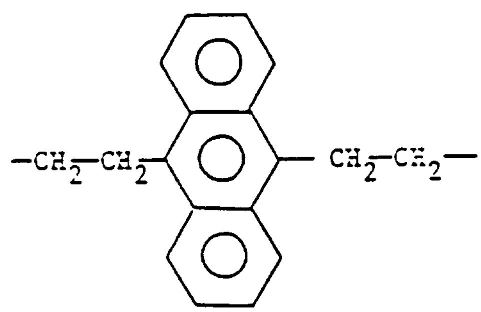

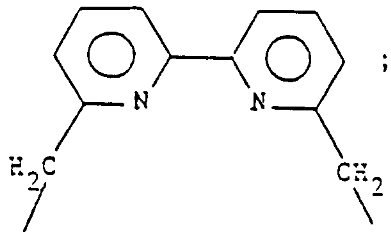 ; 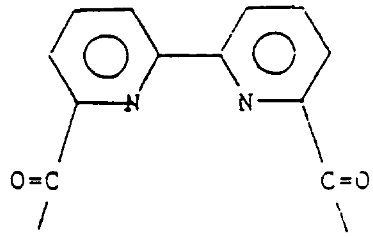 ;

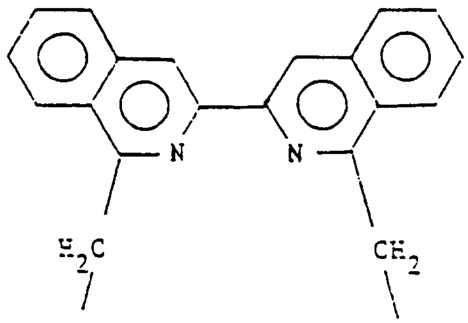 or 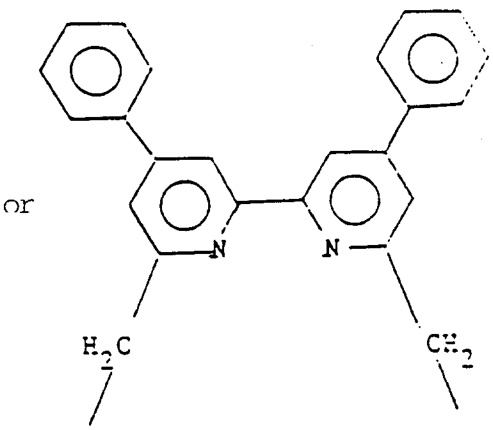

**11.** Method according to Claim 2, characterised in that Ⓐ and Ⓑ each represent the group

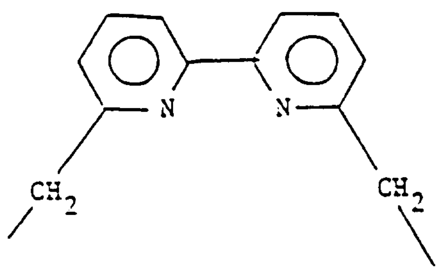

and Ⓒ is one of the following groups:

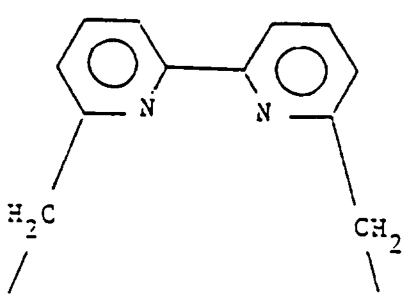 ; 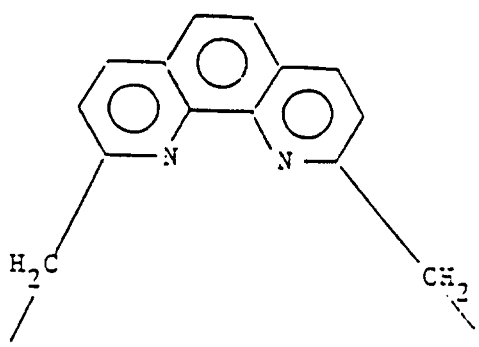 ;

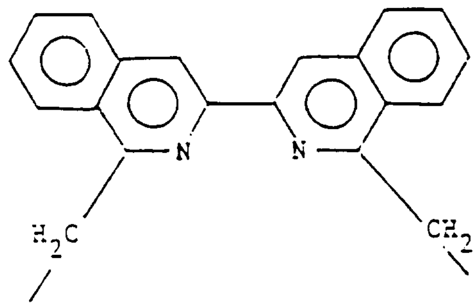 or 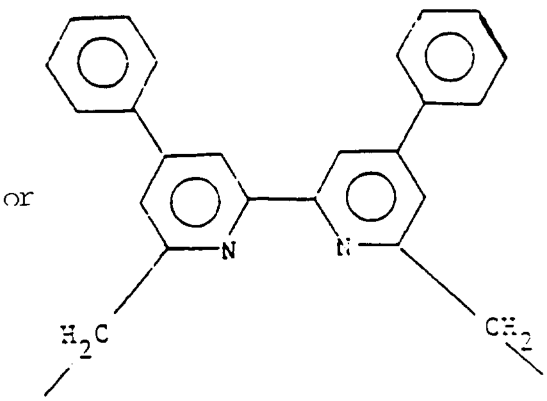

**12.** Method according to Claim 2, characterised in that Z = N; Ⓐ, Ⓑ and Ⓒ are identical and represent one of the heterocycles below:

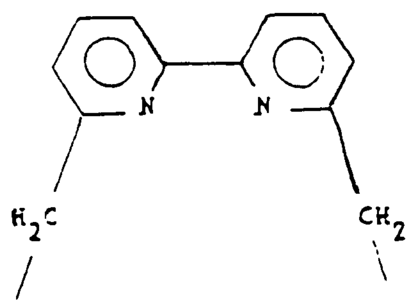 ; 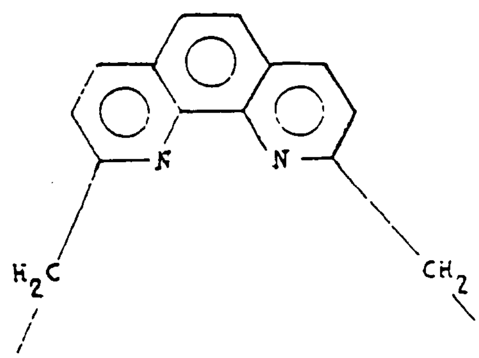 ;

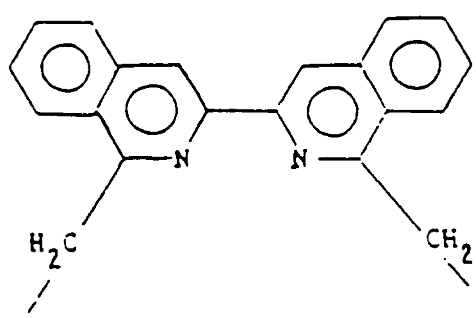 or 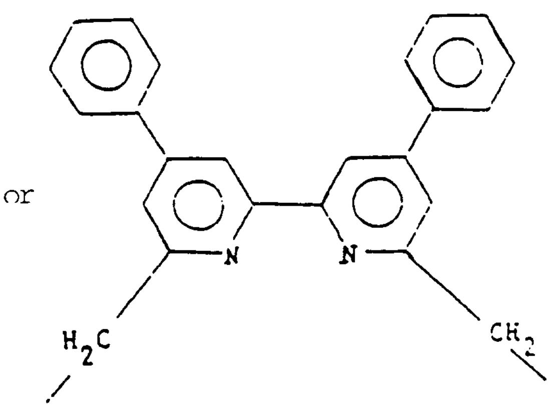

**13.** Method for obtaining macropolycyclic compounds corresponding to the formula:

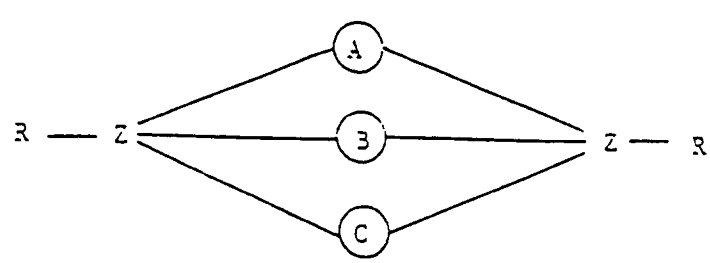

I

in which Z is an atom chosen from nitrogen, carbon or phosphorus, R does not exist or represents hydrogen, the hydroxyl group, an amino group or a hydrocarbon radical, the bivalent radicals Ⓐ, Ⓑ and Ⓒ are, independently of each other, hydrocarbon chains containing at least two atoms of carbon and optionally one or more heteroatoms chosen from oxygen or sulphur and optionally interrupted by a heteromacrocycle, at least one of the radicals Ⓐ, Ⓑ and Ⓒ comprising moreover at least one molecular unit or essentially consisting of a molecular unit, the said molecular unit having a triplet energy greater than that of the emissive level of the complexed rare earth ion and being chosen from phenanthroline, anthracene, the bipyridines and the biquinolines, in particular the bisisoquinolines, with the proviso that, when Z is nitrogen and Ⓐ corresponds to one of the formulae below:

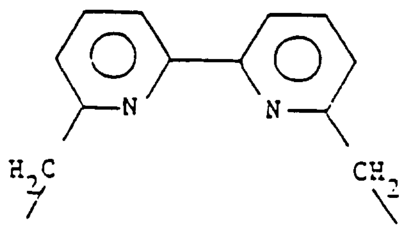

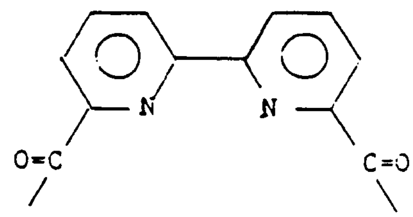

Ⓑ and Ⓒ are not simultaneously $-(CH_2)_2-O-(CH_2)_2-O-(CH_2)_2-$, characterised in that it consists in condensing chemical compounds each comprising one of the radicals Ⓐ, Ⓑ, and Ⓒ and terminal groups capable of being substituted or comprising radicals which can easily be eliminated.

**14.** Method according to Claim 13, characterised in that Z is nitrogen and Ⓐ and Ⓑ are polyoxyethylenated chains, characterised in that it consists:
1) in reacting:
- the nitrogen-containing macrocycle consisting of the two polyoxyethylenated chains with
- a hydrocarbon chain comprising the said molecular unit, the said chain having cleavable terminal groups such as, for example, halo groups, in an anhydrous solvent and optionally in the presence of a reducing agent such as sodium hydride or sodium carbonate, and

2) optionally in converting the salt obtained into a free macropolycyclic compound.

**15.** Method according to one of Claims 13 and 14, for obtaining the compounds below:
2,9-[N,N',N,N'-bis(3,6-dioxaoctamethylene)bis(aminomethyl)]-1,10-phenanthroline;
2,9-[N,N',N,N'-bis(3,6-dioxaoctamethylene)bis(aminocarbonyl)]-1,10-phenanthroline,
9,10-[N,N',N,N'-bis(3,6-dioxaoctamethylene)bis(aminoethyl)]anthracene;
9,10-[N,N',N,N'-bis(3,6-dioxaoctamethylene)bis(carbamoylmethyl)]anthracene;
1,1'-[N,N',N,N'-bis(3,6-dioxaoctamethylene)bis(aminomethyl)]-3,3'-bisisoquinoline;
6,6'-[N,N',N,N'-bis(3,6-dioxaoctamethylene)bis(aminomethyl)]-4,4'-diphenyl-2,2'-bipyridine.

**16.** Method according to Claim 13, for obtaining the compounds below:
6,6',6'',6''',6'''',6'''''-bis[nitrilotri(methylene)]tris-2,2'-bipyridine; 2,9-[N,N',N,N'-bis(2,2'-bipyridine-6,6'-dimethyl)bis(aminomethyl)]-1,10-phenan throline; 1,1'-[N,N',N,N'-bis(2,2'-bipyridine-6,6'-dimethyl)bis(aminomethyl)]-3,3'-bisisoquinoline; 2,2',2'',9,9',9''-[nitrilotri(methylene)]tris(1,10-phenanthroline); 6,6'-[N,N',N,N'-bis(2,2'-bipyridine-6,6'-dimethyl)bis(aminomethyl)]-4,4'-diphenyl-2,2'-bipyridine.

**17.** Method for preparing a biological complex, characterised in that a biologically active molecule is associated by coupling with, or adsorption on, a macropolycyclic rare earth complex obtained by a method such as defined in one of Claims 2 to 12.

**18.** Method according to Claim 17, characterised in that the biologically active molecule is chosen from antigens, antibodies, monoclonal antibodies, fragments and Ab-fragment combinations, drugs, receptors, hormones, hormonal receptors, bacteria, steroids, aminoacids, peptides, viruses, vitamins, nucleotides or polynucleotides, enzymes, their substrates, lectins, nucleic acids, DNA and RNA.

## Patentansprüche

## Patentansprüche für folgende Vertragsstaaten : BE, CH, DE, FR, GB, IT, LI, LU, NL, SE

1. Verwendung von makropolyzyklischen Komplexen von seltenen Erden, die mindestens aus einem Salz von seltenen Erden bestehen, das durch eine makropolyzyklische Verbindung der allgemeinen Formel:

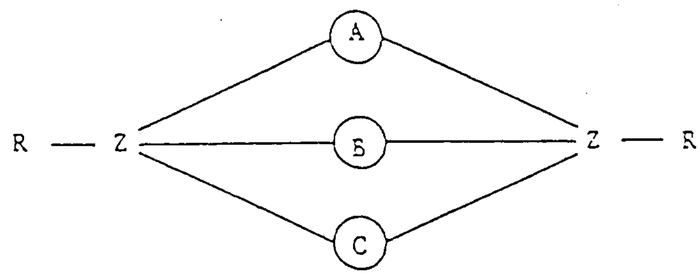

I

komplexiert ist, in der Z ein Atom, ausgewählt aus Stickstoff Kohlenstoff oder Phosphor ist, R nichts ist oder Wasserstoff, die Hydroxygruppe, eine Aminogruppe oder ein Kohlenwasserstoffradikal darstellt, die bivalenten Radikale Ⓐ, Ⓑ und Ⓒ unabhängig voneinander Kohlenwasserstoffketten sind, die mindestens zwei Kohlenstoffatome umfassen und gegebenenfalls ein oder mehrere Heteroatome, die aus Sauerstoff oder Schwefel ausgewählt und gegebenenfalls durch einen Hetero-Makrozyklus unterbrochen sind, mindestens eines der Radikale Ⓐ , Ⓑ oder Ⓒ zusätzlich mindestens ein molekulares Motiv aufweist oder im wesentlichen durch ein molekulares Motiv gebildet ist, das molekulare Motiv eine Triplett-Energie besitzt, die höher ist als die des Emissionsniveaus des komplexierten seltenen Erdions - als Markierungen von biologischen Produkten in den immunologischen Detektions- und Bestimmungsverfahren durch Fluoreszenz.

2. Verwendung gemäß Anspruch 1, dadurch gekennzeichnet, daß die besagten Komplexe von seltenen Erden durch ein Radikal substituiert sind, das ausgewählt ist aus den Radikalen Alkylamino, Arylamino, Isothiocyano, Cyano, Isocyano, Thiocyano, Carboxyl, Hydroxyl, Mercapto, Phenol, Imidazol, Aldehyd, Epoxid, Halogen : von Thionyl, von Sulfonyl, von Nitrobenzoyl, von Carbonyl; Triazo, Succinimido, Anhydrid, Halogenacetat, Hydrazino, Dihalogentriazinyl.

3. Makropolyzyklische Komplexe von seltenen Erden, die mindestens aus einem seltenen Erdsalz bestehen, das durch eine makropolyzyklische Verbindung der Formel:

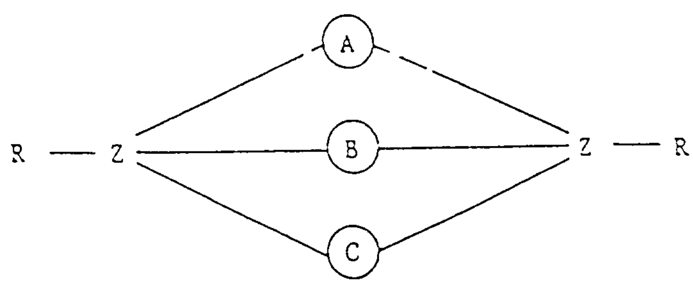

I

komplexiert ist, in der Z ein Atom, ausgewählt aus Stickstoff Kohlenstoff oder Phosphor ist, R nichts ist oder Wasserstoff, die Hydroxygruppe, eine Aminogruppe oder ein Kohlenwasserstoffradikal darstellt, die bivalenten Radikale Ⓐ , Ⓑ und Ⓒ unabhängig von einander Kohlenwasserstoffketten sind, die mindestens zwei Kohlenstoffatome umfassen und gegebenenfalls ein oder mehrere Heteroatome, die aus Sauerstoff und Schwefel ausgewählt und gegebenenfalls durch einen Hetero-Makrozyklus unterbrochen sind, mindestens eines der Radikale Ⓐ , Ⓑ oder Ⓒ zusätzlich mindestens ein molekulares Motiv aufweist oder im wesentlichen durch ein molekulares Motiv gebildet ist, das molekulare Motiv eine Triplett-Energie besitzt, die höher ist als die des Emissionsniveaus des komplexierten seltenen Erdions, vorausgesetzt, daß,

wenn das seltene Erdsalz ein Europium- oder Terbiumsalz ist, Z Stickstoff ist, Ⓐ $-(CH_2)_2-O-C_6H_3R_1-O-$

$(CH_2)_2$- mit $R_1$ = H oder $NH_2$ ist, Ⓑ und Ⓒ nicht gleichzeitig das eine das Radikal $-(CH_2)_2-O-(CH_2)_2-O-(CH_2)_2-$ und das andere das Radikal $-(CH_2)_2-O-(CH_2)_2-$ ist und daß,
wenn das seltene Erdsalz ein Lanthan- oder ein Praseodymsalz ist, Z Stickstoff ist, Ⓐ $-(CH_2)_2-O-C_6H_3NH_2-O-(CH_2)_2-$ ist, Ⓑ und Ⓒ nicht gleichzeitig $-(CH_2)_2-O-(CH_2)_2-$ und $-(CH_2)_2-O-(CH_2)_2-O-(CH_2)_2-$ sind, als neue Produkte.

4. Komplexe gemäß Anspruch 3, dadurch gekennzeichnet, daß die Kohlenwasserstoffketten Oxyethylen- oder Polyoxyethylenketten sind.

5. Makropolyzyklische Komplexe gemäß einem der Ansprüche 3 oder 4, dadurch gekennzeichnet, daß das seltene Erdion ausgewählt ist aus Europium, Terbium, Samarium und Dysprosium.

6. Makropolyzyklische Komplexe gemäß irgendeinem der Ansprüche 3 bis 5, dadurch gekennzeichnet, daß der Motiv-Energiedonor eine Phosphoreszenzwellenlänge unterhalb von 580 nm besitzt.

7. Makropolyzyklische Komplexe gemäß irgendeinem der Ansprüche 3 bis 6, dadurch gekennzeichnet, daß das molekulare Motiv ausgewählt ist aus Phenanthrolin, Anthracen, Benzol, Naphthalin, den Bi- und Terphenylen, Azobenzol, Azopyridin, Pyridin, den Bipyridinen, den Bichinolinen, insbesondere den Biisochinolinen und den Verbindungen der nachfolgenden Formeln:
$-C_2H_4-X_1-C_6H_4-X_2-C_2H_4-$
$-C_2H_4-X_1-CH_2-C_6H_4-CH_2-X_2-C_2H_4-$
in denen $X_1$ und $X_2$ gleich oder verschieden sein können, Sauerstoff oder Schwefel bezeichnen;

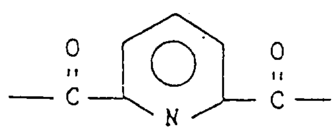

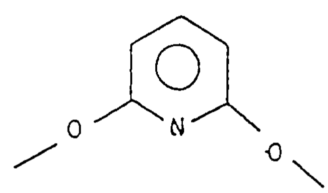

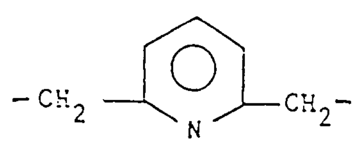

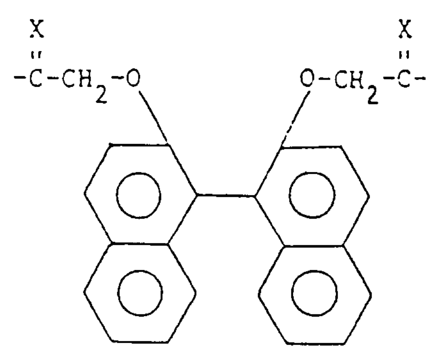

X Sauerstoff oder Wasserstoff ist.

8. Makropolyzyklischer Komplex gemäß irgendeinem der Ansprüche 3 bis 7, dadurch gekennzeichnet, daß er aus dem Terbium- oder Europiumion, das durch eine der nachfolgenden makropolyzyklischen Verbindungen komplexiert ist, besteht:
2,9-[N,N',N,N'-bis(3,6-dioxaoctamethylen)bis(aminomethyl)]-1,10-phenanthrolin, 2,9-[N,N',N,N'-bis(3,6-dioxaoctamethylen)bis(aminocarbonyl)]-1,10-phenanthrolin, 9,10-[N,N',N,N'-bis(3,6-dioxaoctamethylen)-bis(aminoethyl)]anthracen, 9,10-[N,N',N,N'-bis(3,6-dioxaoctamethylen)bis(carbamoylmethyl)]anthracen, 1,1'-[N,N',N,N'-bis(3,6-dioxaoctamethylen)bis(aminomethyl)]-3,3'-biisochinolin, 6,6'-[N,N',N,N'-bis(3,6-dioxaoctamethylen)bis(aminomethyl)]-4,4'-diphenyl-2,2'-bipyridin, 6,6'-[N,N',N,N'-bis(3,6-dioxaoctamethylen)bis(aminomethyl)]-2,2'-bipyridin, 6,6'-[N,N',N,N'-bis(3,6-dioxaoctamethylen)bis(aminocarbonyl)]-2,2'-bipyridin, 6,6',6'',6''''',6''''''-bis[nitrilotri(methylen)]tris-2,2'-bipyridin, 2,2',2'',9,9',9''-bis[nitrilotri-(methylen)]tris(1,10-phenanthrolin), 2,9-[N,N',N,N'-bis(2,2'-bipyridin-6,6'-dimethyl)bis(aminomethyl)]-1,10-phenanthrolin, 1,1-[N,N',N,N'-bis(2,2'-bipyridin-6,6'-dimethyl)bis(aminomethyl)]-3,3'-biisochinolin, 6,6'-[N,N',N,N'-bis(2,2'-bipyridin-6,6'-dimethyl)bis(aminomethyl)]-4,4'-diphenyl-2,2'-bipyridin.

**9.** Makropolyzyklische Komplexe gemäß Anspruch 3, dadurch gekennzeichnet, daß sie Formel (I) entsprechen, in der Z = N ist.

**10.** Makropolyzyklische Komplexe von seltenen Erden, die aus mindestens einem seltenen Erdsalz bestehen, das durch eine makropolyzyklische Verbindung komplexiert ist, dadurch gekennzeichnet, daß die makropolyzyklische Verbindung aus den nachfolgenden Verbindungen ausgewählt ist:

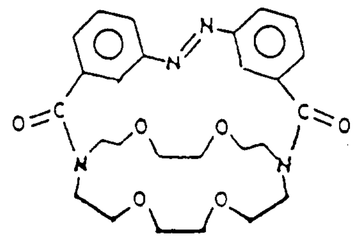

- (22) Pyridinamid

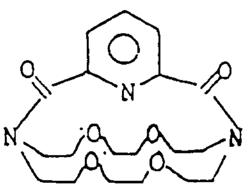

- (22) Pyridinamin

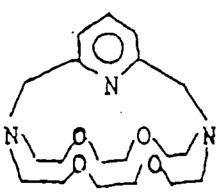

- (22) Bipyridin

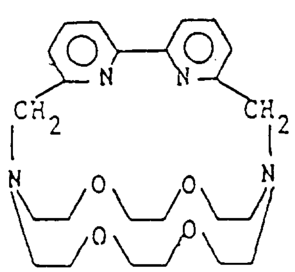

- (22) Bipyridinamid

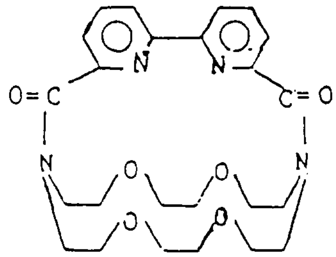

- (22) Azopyridin

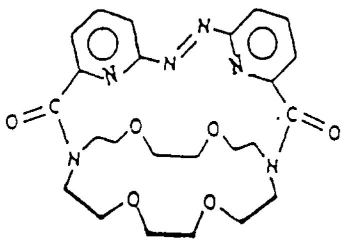

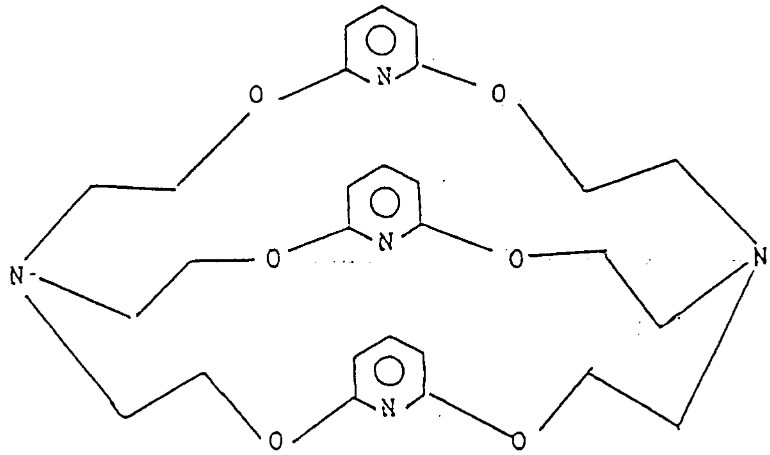

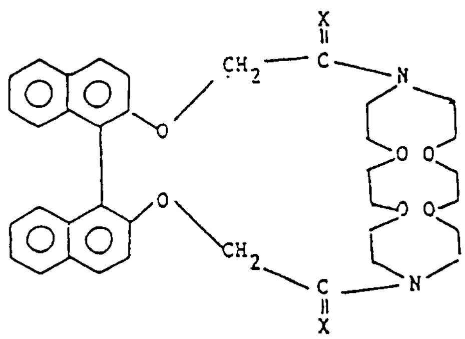

X = O, H

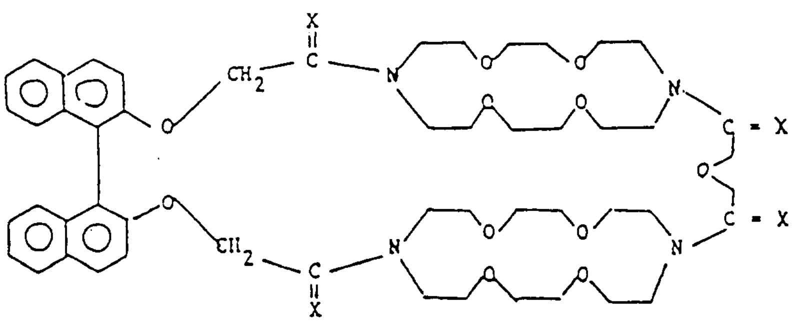

X = O, H

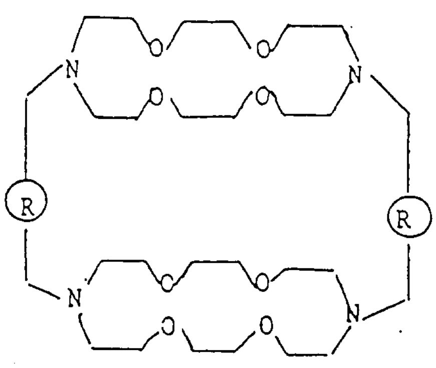

R =

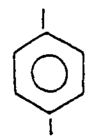

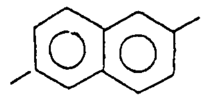

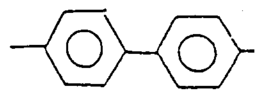

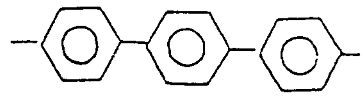

**11.** Makropolyzyklische Komplexe von seltenen Erden, die mindestens aus einem seltenen Erdsalz bestehen, das durch eine makropolyzyklische Verbindung nach Formel (I) gemäß Anspruch 3 komplexiert ist, dadurch gekennzeichnet, daß Z = N, Ⓐ und Ⓑ zwei mono- oder polyoxygenierte Ketten sind, und daß Ⓒ einer der nachfolgenden Formeln entspricht:

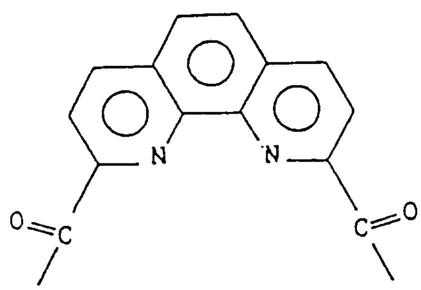

;

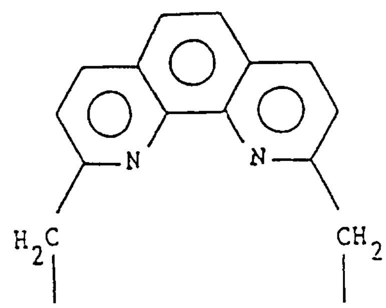

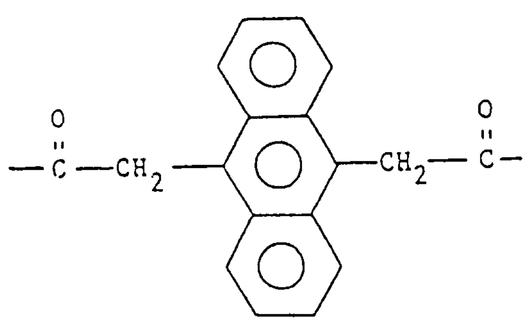

;

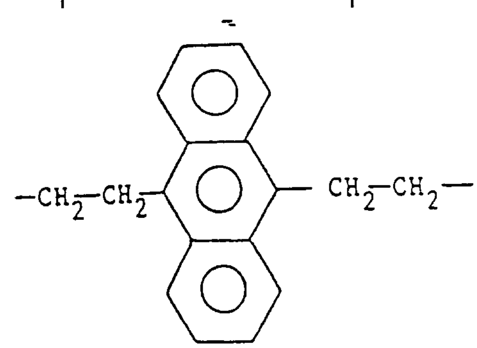

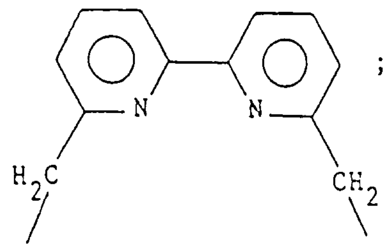 ; 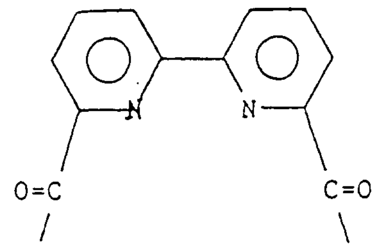 ;

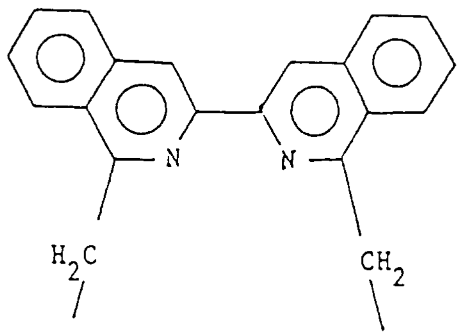 oder 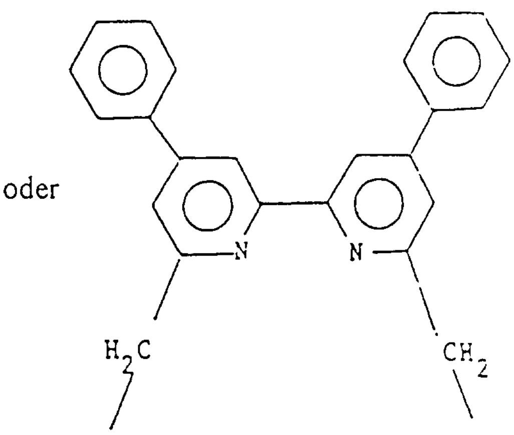

**12.** Makropolyzyklische Komplexe von seltenen Erden, die mindestens aus einem seltenen Erdsalz bestehen, das durch eine makropolyzyklische Verbindung der Formel (I) gemäß Anspruch 3 komplexiert ist, dadurch gekennzeichnet, daß Ⓐ und Ⓑ jeweils die Gruppe

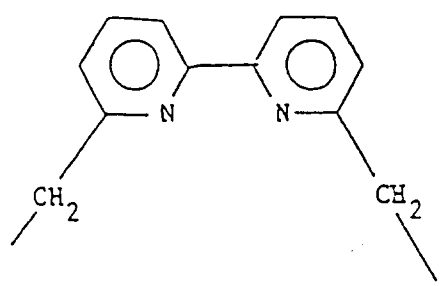

darstellen, und daß Ⓒ eine der nachfolgenden Gruppen ist:

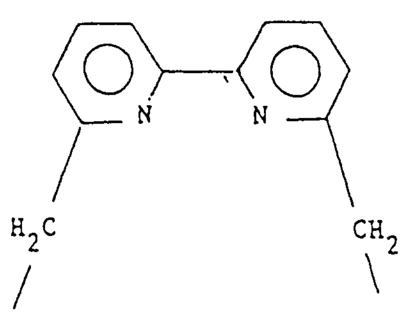 ; 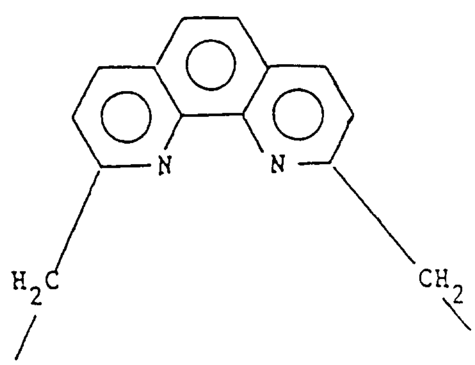 ;

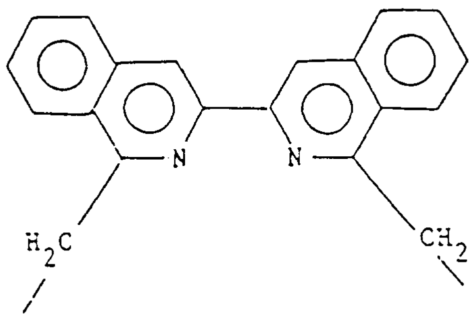

oder

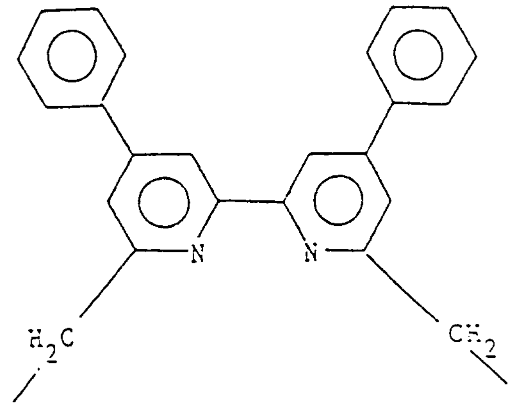

13. Makropolyzyklische Komplexe von seltenen Erden, die mindestens aus einem seltenen Erdsalz, das durch eine makropolyzyklische Verbindung der Formel (I) gemäß Anspruch 3 komplexiert ist, bestehen, dadurch gekennzeichnet, daß Z = N ist, Ⓐ, Ⓑ und Ⓒ identisch sind und einen der nachfolgenden Heterozyklen darstellen:

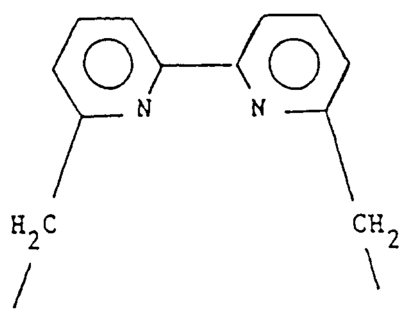

;

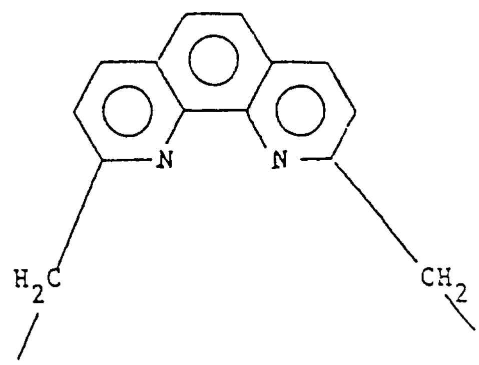

;

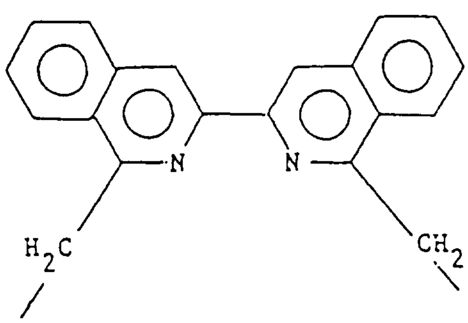

oder

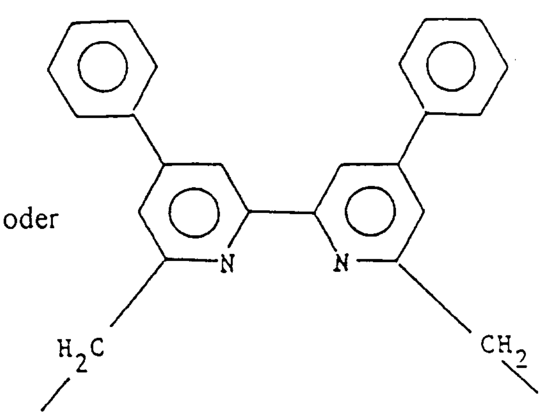

**14.** Makropolyzyklische Verbindung entsprechend der Formel:

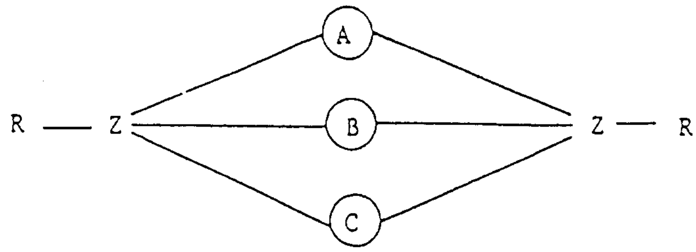

I

in der Z ein Atom, ausgewählt aus Stickstoff, Kohlenstoff oder Phosphor ist, R nichts ist oder Wasserstoff, die Hydroxygruppe, eine Aminogruppe oder ein Kohlenwasserstoffradikal darstellt, die bivalenten Radikale Ⓐ, Ⓑ und Ⓒ unabhängig voneinander Kohlenwasserstoffketten sind, die mindestens zwei Kohlenstoffatome umfassen und gegebenenfalls ein oder mehrere Heteroatome, die aus Sauerstoff oder Schwefel ausgewählt und gegebenenfalls durch einen Hetero-Makrozyklus unterbrochen sind, mindestens eines der Radikale Ⓐ, Ⓑ oder Ⓒ zusätzlich mindestens ein molekulares Motiv aufweist oder im wesentlichen durch ein molekulares Motiv gebildet ist, das molekulare Motiv eine Triplett-Energie besitzt, die höher ist als die des Emissionsniveaus des komplexierten seltenen Erdions und ausgewählt wird aus Phenanthrolin, Anthracen, Bipyridin und den Bichinolinen, insbesondere den Biisochinolinen, vorausgesetzt, daß, wenn Z Stickstoff ist und Ⓐ einer der nachfolgenden Formeln:

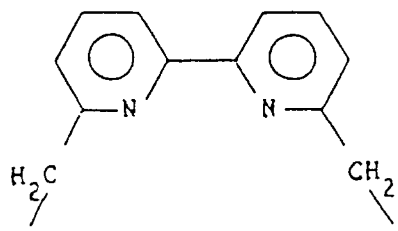

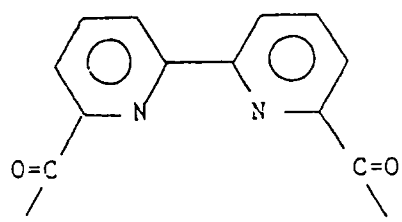

entspricht, Ⓑ und Ⓒ nicht gleichzeitig $-(CH_2)_2-O-(CH_2)_2-O-(CH_2)_2-$ sind.

**15.** Verbindung gemäß Anspruch 14, dadurch gekennzeichnet, daß Z Stickstoff ist und Ⓐ und Ⓑ Polyoxyethylenketten sind.

**16.** Verbindung gemäß einem der Ansprüche 14 oder 15, dadurch gekennzeichnet, daß sie eine der nachfolgenden Verbindungen ist: 2,9-[N,N',N,N'-bis(3,6-dioxaoctamethylen)bis(aminomethyl)]-1,10-phenanthrolin, 2,9-[N,N',N,N'-bis(3,6-dioxaoctamethylen)bis(aminocarbonyl)]-1,10-phenanthrolin, 9,10-[N,N',N,N'-bis(3,6-dioxaoctamethylen)-bis(aminoethyl)]anthracen, 9,10-[N,N',N,N'-bis(3,6-dioxaoctamethylen)bis(carbamoylmethyl)]anthracen, 1,1'-[N,N',N,N'-bis(3,6-dioxaoctamethylen)bis(aminomethyl)]-3,3'-biisochinolin, 6,6'-[N,N',N,N'-bis(3,6-dioxaoctamethylen)bis(aminomethyl)]-4,4'-diphenyl-2,2'-bipyridin.

**17.** Verbindung gemäß Anspruch 14, dadurch gekennzeichnet, daß die Verbindung so ist, wie in irgendeinem der Ansprüche 9 bis 13 definiert.

**18.** Verbindung gemäß Anspruch 14, dadurch gekennzeichnet, daß sie 6,6',6'',6''',6'''',6'''''-bis[nitrilotri-(methylen)]tris-2,2'-bipyridin, 2,9-[N,N',N,N'-bis(2,2'-bipyridin-6,6'-dimethyl)bis(aminomethyl)]-1,10-phenanthrolin, 1,1'-[N,N',N,N'-bis(2,2'-bipyridin-6,6'-dimethyl)bis(aminomethyl)]-3,3'biisochinolin, 2,2',2'',9,9',9''-bis[nitrilotri(methylen)]tris(1,10-phenanthrolin), 6,6'-[N,N',N,N'-bis(2,2'bipyridin-6,6'-dimethyl)bis(aminomethyl)]-4,4'-diphenyl-2,2'-bipyridin.

**19.** Biologischer Komplex, dadurch gekennzeichnet, daß er aus einem biologisch aktiven Molekül besteht, das durch Adsorption auf oder Kupplung mit einem makropolyzyklischen Komplex von seltenen Erden,

wie in den Ansprüchen 1, 3 bis 13 definiert, assoziiert ist.

**20.** Biologischer Komplex gemäß Anspruch 19, dadurch gekennzeichnet, daß das biologisch aktive Molekül ausgewählt wird aus den Antigenen, den Antikörpern, den monoklonalen Antikörpern, den Fragmenten und Kombinationen von Antikörper-Fragmenten, den Arzneimitteln, den Rezeptoren, den Hormonen, den Hormonrezeptoren, den Bakterien, den Steroiden, den Aminosäuren, den Peptiden, den Viren, den Vitaminen, den Nucleotiden oder Polynucleotiden, den Enzymen, ihren Substraten, den Lektinen, den Nucleinsäuren, DNS und RNS.

**21.** Exaltationsverfahren der Fluoreszenz von seltenen Erdionen, dadurch gekennzeichnet, daß es darauf beruht, mindestens ein selten Erdion mit einer makropolyzyklischen Verbindung, wie in den Ansprüchen 1, 3 bis 13 definiert, zu komplexieren und das molekulare Motiv der Verbindung anzuregen.

**Patentansprüche für folgenden Vertragsstaat : AT**

**1.** Exaltationsverfahren der Fluoreszenz eines seltenen Erdions, dadurch gekennzeichnet, daß es darin besteht, ein seltenes Erdion mit einer makropolyzyklischen Verbindung der nachfolgenden allgemeinen Formel I oder einem ihrer Salze zu komplexieren:

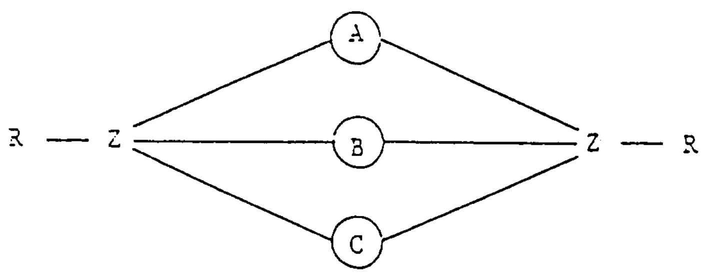

I

in der Z ein Atom, ausgewählt aus Stickstoff, Kohlenstoff oder Phosphor ist, R nichts ist oder Wasserstoff, die Hydroxygruppe, eine Aminogruppe oder ein Kohlenwasserstoffradikal darstellt, die bivalenten Radikale Ⓐ, Ⓑ und Ⓒ unabhängig voneinander Kohlenwasserstoffketten sind, die mindestens zwei Kohlenstoffatome umfassen und gegebenenfalls ein oder mehrere Heteroatome, die aus Sauerstoff oder Schwefel ausgewählt und gegebenenfalls durch einen Hetero-Makrozyklus unterbrochen sind, mindestens eines der Radikale Ⓐ, Ⓑ oder Ⓒ zusätzlich mindestens ein molekulares Motiv aufweist oder im wesentlichen durch ein molekulares Motiv gebildet ist, das molekulare Motiv eine Triplett-Energie besitzt, die höher ist als die des Emissionsniveaus des komplexierten seltenen Erdions - und das molekulare Motiv der makropolyzyklischen Verbindung anzuregen.

**2.** Herstellungsvenfahren von makropolyzyklischen Komplexen von seltenen Erden, dadurch gekennzeichnet, daß es darin besteht, wenigstens ein seltenes Erdsalz mit einer makropolyzyklischen Verbindung der Formel:

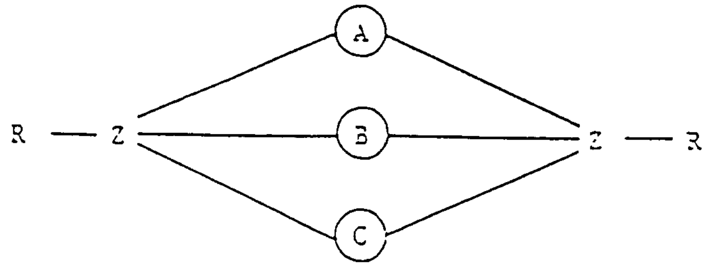

I

umzusetzen, in der Z ein Atom, ausgewählt aus Stickstoff, Kohlenstoff oder Phosphor ist, R nichts ist oder Wasserstoff, die Hydroxygruppe, eine Aminogruppe oder ein Kohlenwasserstoffradikal darstellt, die bivalenten Radikale Ⓐ, Ⓑ und Ⓒ unabhängig von einander Kohlenwasserstoffketten sind, die mindestens zwei Kohlenstoffatome umfassen und gegebenenfalls ein oder mehrere Heteroatome, die aus Sauerstoff und Schwefel ausgewählt und gegebenenfalls durch einen Hetero-Makrozyklus unterbrochen sind, mindestens eines der Radikale Ⓐ, Ⓑ oder Ⓒ zusätzlich mindestens ein molekulares Motiv aufweist oder im wesentlichen durch ein molekulares Motiv gebildet ist, das molekulare Motiv eine Triplett-Energie besitzt, die höher ist als die des Emissionsniveaus des komplexierten seltenen Erdions, vorausgesetzt, daß,
wenn das seltene Erdsalz ein Europium- oder Terbiumsalz ist, Z Stickstoff ist, Ⓐ $-(CH_2)_2-O-C_6H_3R_1-O-(CH_2)_2-$ mit $R_1$ = H oder $NH_2$ ist, Ⓑ und Ⓒ nicht gleichzeitig das eine das Radikal $-(CH_2)_2-O-(CH_2)_2-O-(CH_2)_2-$ und das andere das Radikal $-(CH_2)_2-O-(CH_2)_2-$ ist und daß,
wenn das seltene Erdsalz ein Lanthan- oder ein Praseodymsalz ist, Z Stickstoff ist, Ⓐ $-(CH_2)_2-O-C_6H_3NH_2-O-(CH_2)_2-$ ist, Ⓑ und Ⓒ nicht gleichzeitig $-(CH_2)_2-O-(CH_2)_2-$ und $-(CH_2)_2-O-(CH_2)_2-O-(CH_2)_2-$ sind.

**3.** Verfahren gemäß Anspruch 2, dadurch gekennzeichnet, daß die Kohlenwasserstoffketten Oxyethylen- oder Polyoxyethylenketten sind.

**4.** Verfahren gemäß einem der Ansprüche 2 oder 3, dadurch gekennzeichnet, daß das seltene Erdion ausgewählt ist aus Europium, Terbium, Samarium und Dysprosium.

**5.** Verfahren gemäß irgendeinem der Ansprüche 2 bis 4, dadurch gekennzeichnet, daß der Motiv-Energiedonor eine Phosphoreszenzwellenlänge unterhalb von 580 nm besitzt.

**6.** Verfahren gemäß irgendeinem der Ansprüche 2 bis 5, dadurch gekennzeichnet, daß das molekulare Motiv ausgewählt ist aus Phenanthrolin, Anthracen, Benzol, Naphthalin, den Bi- und Terphenylen, Azobenzol, Azopyridin, Pyridin, den Bipyridinen, den Bichinolinen, insbesondere den Biisochinolinen und den Verbindungen der nachfolgenden Formeln:
$-C_2H_4-X_1-C_6H_4-X_2-C_2H_4-$
$-C_2H_4-X_1-CH_2-C_6H_4-CH_2-X_2-C_2H_4-$
in denen $X_1$ und $X_2$ gleich oder verschieden sein können, Sauerstoff oder Schwefel bezeichnen;

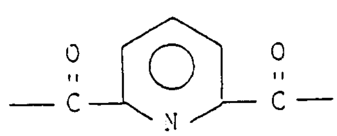

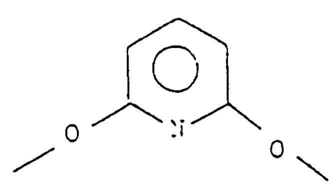

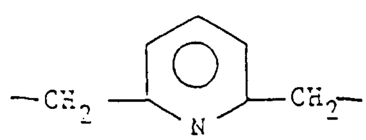

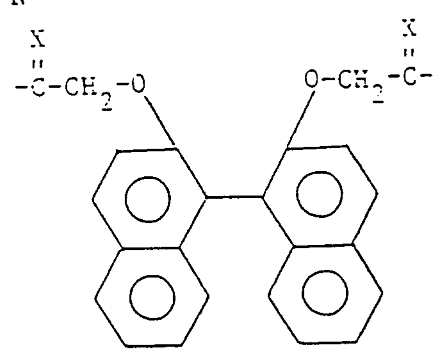

X Sauerstoff oder Wasserstoff ist.

**7.** Verfahren gemäß irgendeinem der Ansprüche 2 bis 6, dadurch gekennzeichnet, daß der makropolyzyklische Komplex aus dem Terbium- oder Europiumion, das durch eine der nachfolgenden makropolyzyklischen Verbindungen komplexiert ist, besteht:
2,9-[N,N',N,N'-bis(3,6-dioxaoctamethylen)bis(aminomethyl)]-1,10-phenanthrolin, 2,9-[N,N',N,N'-bis(3,6-dioxaoctamethylen)bis(aminocarbonyl)]-1,10-phenanthrolin, 9,10-[N,N',N,N'-bis(3,6-dioxaoctamethylen)-bis(aminoethyl)]anthracen, 9,10-[N,N',N,N'-bis(3,6-dioxaoctamethylen)bis(carbamoylmethyl)]anthracen,

1,1'-[N,N',N,N'-bis(3,6-dioxaoctamethylen)bis(aminomethyl)]-3,3'-biisochinolin, 6,6'-[N,N',N,N'-bis(3,6-dioxaoctamethylen)bis(aminomethyl)]-4,4'-diphenyl-2,2'-bipyridin, 6,6'-[N,N',N,N'-bis(3,6-dioxaoctamethylen)bis(aminomethyl)]-2,2'-bipyridin, 6,6'-[N,N',N,N'-bis(3,6-dioxaoctamethylen)bis(aminocarbonyl)]-2,2'-bipyridin, 6,6',6'',6''',6'''',6'''''-bis[nitrilotri(methylen)]tris-2,2'-bipyridin, 2,2',2'',9,9',9''-bis[nitrilotri-(methylen)]tris(1,10-phenanthrolin), 2,9-[N,N',N,N'-bis(2,2'-bipyridin-6,6'-dimethyl)bis(aminomethyl)]-1,10-phenanthrolin, 1,1'-[N,N',N,N'-bis(2,2'-bipyridin-6,6'-dimethyl)bis(aminomethyl)]-3,3'-biisochinolin, 6,6'-[N,N',N,N'-bis(2,2'-bipyridin-6,6'-dimethyl)bis(aminomethyl)]-4,4'-diphenyl-2,2'-bipyridin.

**8.** Verfahren gemäß Anspruch 2, dadurch gekennzeichnet, daß Z Stickstoff ist.

**9.** Verfahren gemäß Anspruch 2, dadurch gekennzeichnet, daß die makropolyzyklische Verbindung aus den nachfolgenden Verbindungen ausgewählt ist:

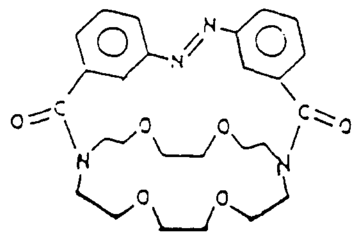

- (22) Pyridinamid

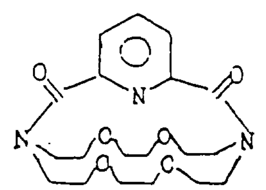

- (22) Pyridinamin

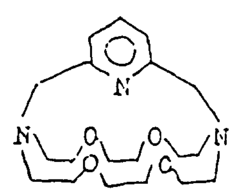

- (22) Bipyridin

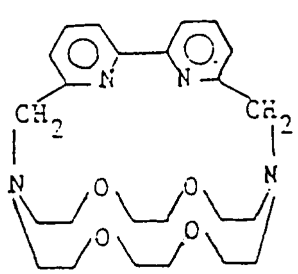

- (22) Bipyridinamid

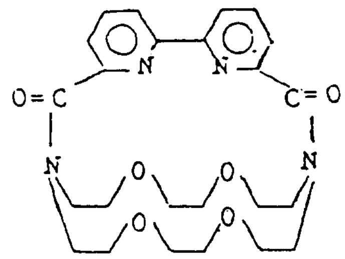

- (22) Azopyridin

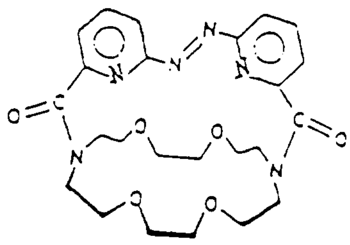

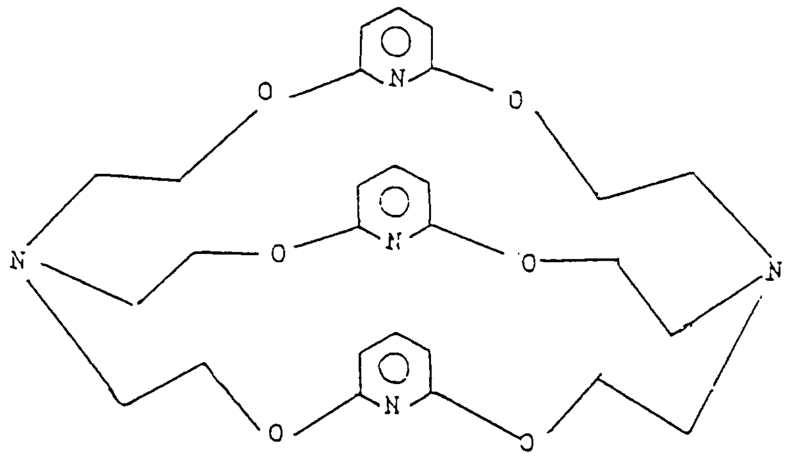

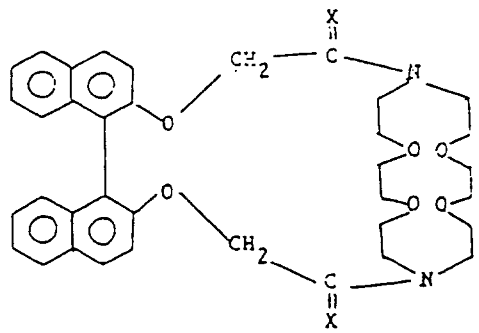

X = O, H

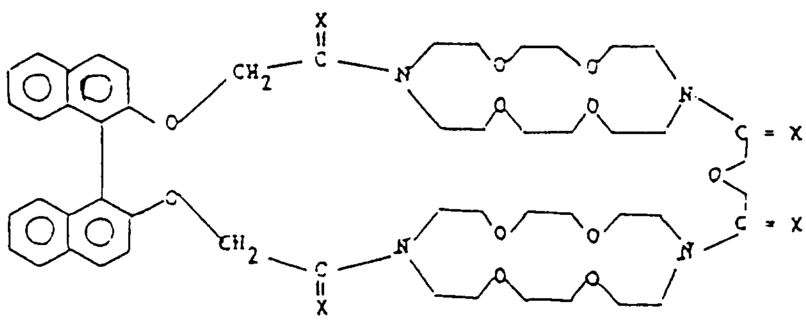

X = O, H

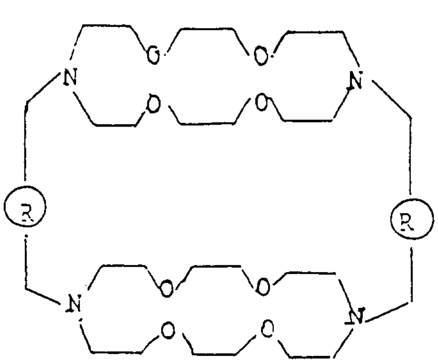

R =

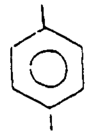

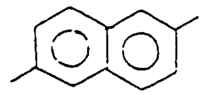

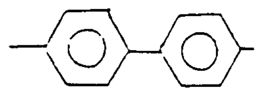

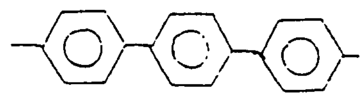

**10.** Verfahren gemäß Anspruch 2, dadurch gekennzeichnet, daß Z = N, Ⓐ und Ⓑ zwei mono- oder polyoxygenierte Ketten sind, und daß Ⓒ einer der nachfolgenden Formeln entspricht:

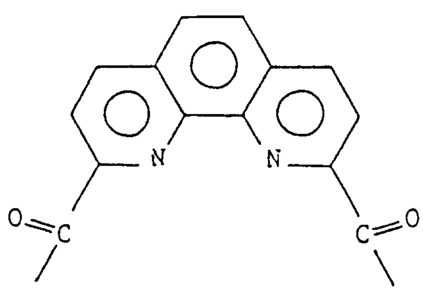

;

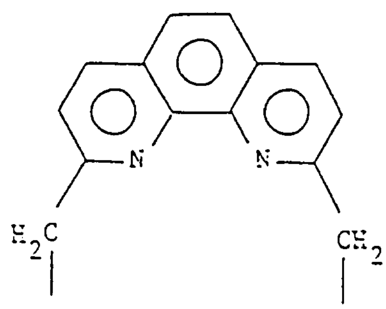

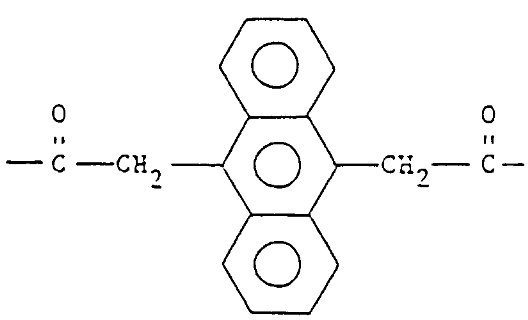

;

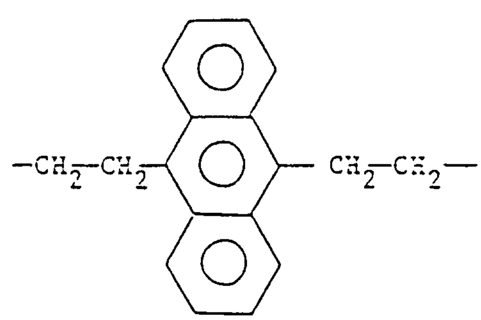

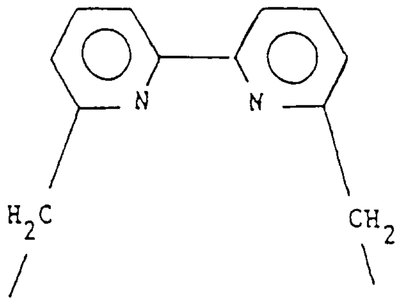

;

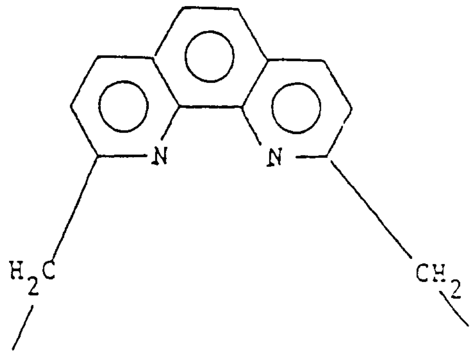

;

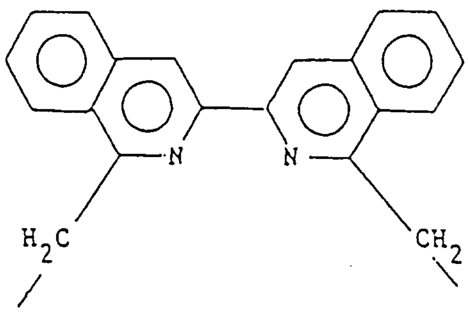

oder

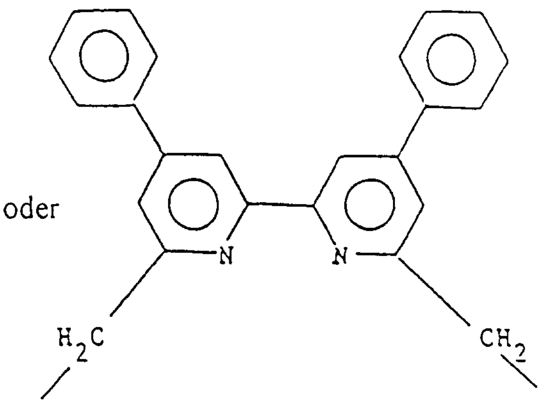

**11.** Verfahren gemäß Anspruch 2, dadurch gekennzeichnet, daß Ⓐ und Ⓑ jeweils die Gruppe

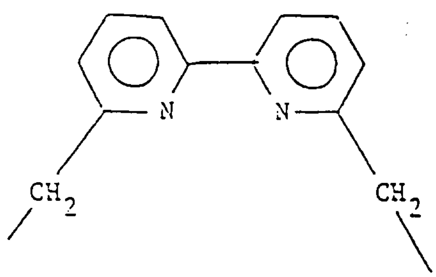

darstellen, und daß Ⓒ eine der nachfolgenden Gruppen ist:

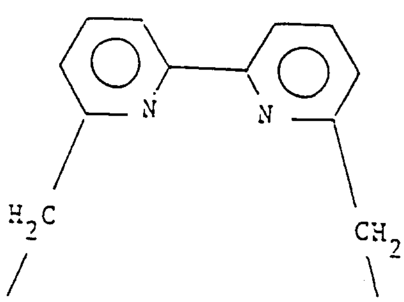

;

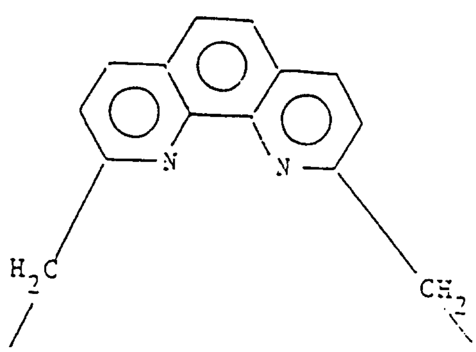

;

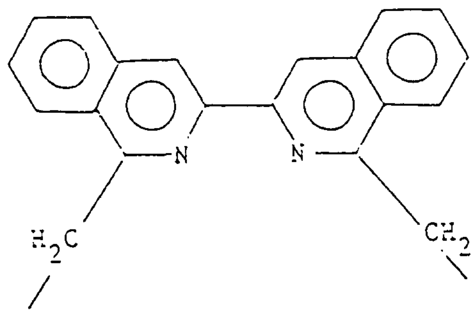

oder

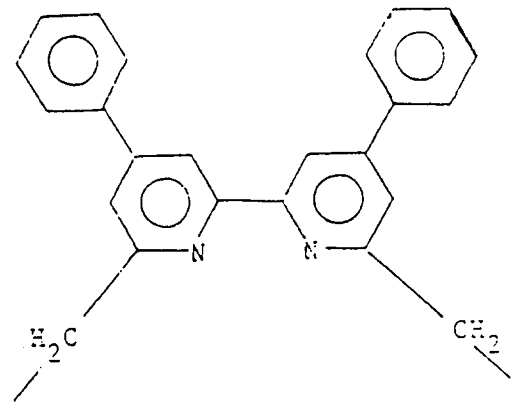

**12.** Verfahren gemäß Anspruch 2, dadurch gekennzeichnet, daß Z = N ist, Ⓐ, Ⓑ und Ⓒ identisch sind und einen der nachfolgenden Heterozyklen darstellen:

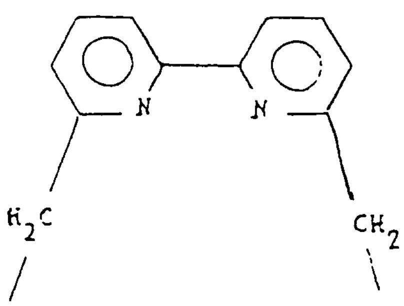

;

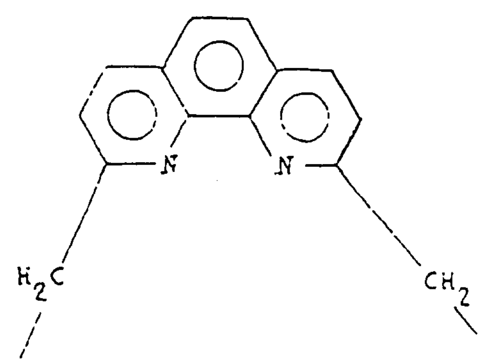

;

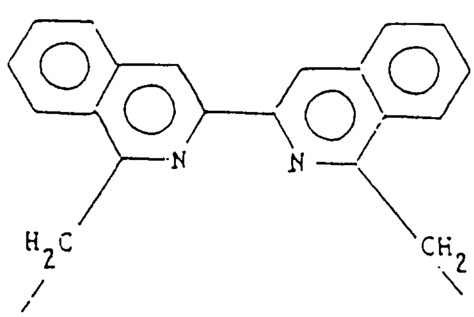

oder

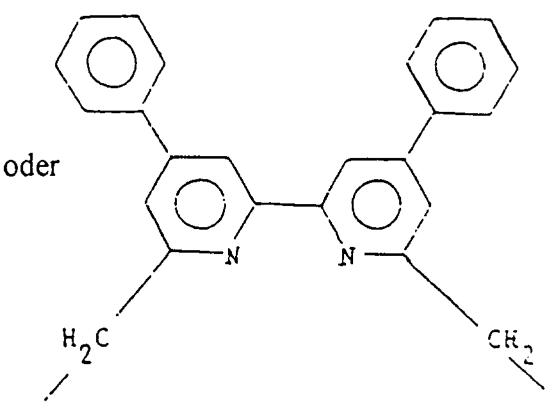

**13.** Verfahren zur Herstellung von makropolyzyklischen Verbindungen der Formel:

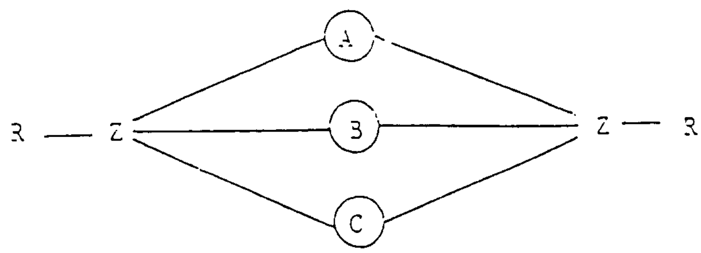

I

in der Z ein Atom, ausgewählt aus Stickstoff, Kohlenstoff oder Phosphor ist, R nichts ist oder Wasserstoff, die Hydroxygruppe, eine Aminogruppe oder ein Kohlenwasserstoffradikal darstellt, die bivalenten Radikale Ⓐ, Ⓑ und Ⓒ unabhängig voneinander Kohlenwasserstoffketten sind, die mindestens zwei Kohlenstoffatome umfassen und gegebenenfalls ein oder mehrere Heteroatome, die aus Sauerstoff oder Schwefel ausgewählt und gegebenenfalls durch einen Hetero-Makrozyklus unterbrochen sind, mindestens eines der Radikale Ⓐ, Ⓑ oder Ⓒ zusätzlich mindestens ein molekulares Motiv aufweist oder im wesentlichen durch ein molekulares Motiv gebildet ist, das molekulare Motiv eine Triplett-Energie besitzt, die höher ist als die des Emissionsniveaus des komplexierten seltenen Erdions und ausgewählt wird aus Phenanthrolin, Anthracen, den Bipyridinen und den Bichinolinen, insbesondere den Biisochinolinen, vorausgesetzt, daß, wenn Z Stickstoff ist und Ⓐ einer der nachfolgenden Formeln entspricht:

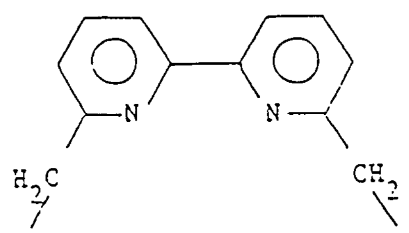

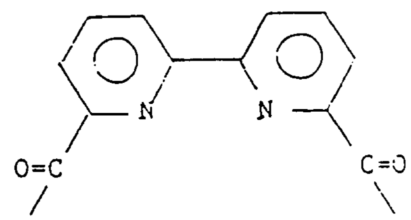

Ⓑ und Ⓒ nicht gleichzeitig $-(CH_2)_2-O-(CH_2)_2-O-(CH_2)_2-$ sind, dadurch gekennzeichnet, daß es darin besteht, chemische Verbindungen, deren jede eines der Radikale Ⓐ, Ⓑ oder Ⓒ und Endgruppen aufweist, die geeignet sind, substituiert zu werden oder die leicht eliminierbare Radikale aufweisen.

**14.** Verfahren gemäß Anspruch 13, dadurch gekennzeichnet, daß Z Stickstoff ist und Ⓐ und Ⓑ Polyoxyethylenketten sind,
dadurch gekennzeichnet, daß es darin besteht:
1)
- den stickstoffhaltigen Makrozyklus, der durch die beiden Polyoxyethylenketten gebildet ist, mit
- einer Kohlenwasserstoffkette, die das molekulare Motiv aufweist, wobei die Kette abspaltbare Endgruppen, wie beispielsweise Halogengruppen, hat

in einem wasserfreien Lösungsmittel und gegebenenfalls in Gegenwart eines Reduktionsmittels, wie Natriumhydrid oder Natriumcarbonat
umzusetzen, und
2) gegebenenfalls das erhaltene Salz in die freie makropolyzyklische Verbindung umzuwandeln.

**15.** Verfahren gemäß einem der Ansprüche 13 oder 14 zur Herstellung der nachfolgenden Verbindungen: 2,9-[N,N',N,N'-bis(3,6-dioxaoctamethylen)bis(aminomethyl)]-1,10-phenanthrolin, 2,9-[N,N',N,N'-bis(3,6-dioxaoctamethylen)bis(aminocarbonyl)]-1,10-phenanthrolin, 9,10-[N,N',N,N'-bis(3,6-dioxaoctamethylen)-bis(aminoethyl)]anthracen, 9,10-[N,N',N,N'-bis(3,6-dioxaoctamethylen)bis(carbamoylmethyl)]anthracen, 1,1'-[N,N',N,N'-bis(3,6-dioxaoctamethylen)bis(aminomethyl)]-3,3'-biisochinolin, 6,6'-[N,N',N,N'-bis(3,6-dioxaoctamethylen)bis(aminomethyl)]-4,4'-diphenyl-2,2'-bipyridin.

16. Verfahren gemäß Anspruch 13 zur Herstellung der nachfolgenden Verbindungen: 6,6',6'',6''',6'''',6'''''-bis[nitrilotri(methylen)tris-2,2'-bipyridin, 2,9-[N,N',N,N'-bis(2,2'-bipyridin-6,6'-dimethyl)bis(aminomethyl)]-1,10-phenanthrolin, 1,1'-[N,N',N,N'-bis(2,2'-bipyridin-6,6'-dimethyl)bis-(aminomethyl)]-3,3'-biisochinolin, 2,2',2''9,9',9''-bis[nitrilotri(methylen)]tris(1,10-phenanthrolin), 6,6'-[N,N',N,N'-bis(2,2'-bipyridin-6,6'-dimethyl)bis(aminomethyl)]-4,4'-diphenyl-2,2'-bipyridin.

17. Herstellungsverfahren eines biologischen Komplexes, dadurch gekennzeichnet, daß man ein biologisch aktives Molekül durch Adsorption auf oder Kupplung mit einem makropolyzyklischen Komplex von seltenen Erden assoziiert, der durch ein Verfahren erhalten wurde, wie in einem der Ansprüche 2 bis 12 definiert.

18. Verfahren gemäß Anspruch 17, dadurch gekennzeichnet, daß das biologisch aktive Molekül ausgewählt wird aus den Antigenen, den Antikörpern, den monoklonalen Antikörpern, den Fragmenten und Kombinationen von Antikörper-Fragmenten, den Arzneimitteln, den Rezeptoren, den Hormonen, den Hormonrezeptoren, den Bakterien, den Steroiden, den Aminosäuren, den Peptiden, den Viren, den Vitaminen, den Nucleotiden oder Polynucleotiden, den Enzymen, ihren Substraten, den Lektinen, den Nucleinsäuren, DNS und RNS.